# EUROPEAN PATENT SPECIFICATION

(11) **EP 2 841 947 B1**
(45) Date of publication and mention of the grant of the patent: **09.10.2019**
(21) Application number: 13720130.7
(22) Date of filing: 26.04.2013
(51) Int. Cl.: G01N 33/574

(54) **BIOMARKERS**
BIOMARKER
BIOMARQUEURS

(30) Priority: 26.04.2012 NL 2008707; 08.02.2013 NL 2010276
(43) Date of publication of application: 04.03.2015
(73) Proprietor: Stichting VUmc, 1081 HV Amsterdam (NL)
(72) Inventor: BOSCH, Linda Janna Willemien, 1081 HV Amsterdam (NL); DE WIT, Meike, 1081 HV Amsterdam (NL); PINTO MORAIS DE CARVALHO, Beatriz, 1081 HV Amsterdam (NL); FIJNEMAN, Remondus Johannes Adriaan, 1081 HV Amsterdam (NL); MEIJER, Gerrit Albert, 1081 HV Amsterdam (NL); JIMENEZ, Cornelia Ramona, 1081 HV Amsterdam (NL); PIERSMA, Sander Rogier, 1081 HV Amsterdam (NL); PHAM, Viet Thang, 1081 HV Amsterdam (NL); OUDGENOEG, Gideon, 1081 HV Amsterdam (NL)
(74) Representative: V.O.
(86) International application number: PCT/NL2013/050316
(87) International publication number: WO 2013/162368

(56) References cited:
- WO-A1-2009/099651
- WO-A2-2010/009368
- US-A- 5 552 292
- US-A- 5 552 292
- US-A1- 2009 035 801
- US-A1- 2011 104 256
- CHING-SENG ANG ET AL: "Targeted In-Gel MRM: A Hypothesis Driven Approach for Colorectal Cancer Biomarker Discovery in Human Feces", JOURNAL OF PROTEOME RESEARCH, vol. 9, no. 9, 2010, pages 4346-4355, XP055050298,
- L. DI MARZIO: "Detection of Alkaline Sphingomyelinase Activity in Human Stool: Proposed Role as a New Diagnostic and Prognostic Marker of Colorectal Cancer", CANCER EPIDEMIOLOGY, BIOMARKERS & PREVENTION, vol. 14, no. 4, 2005, pages 856-862, XP055050295,
- MARTIN W. VOLMER ET AL: "Differential proteome analysis of conditioned media to detect Smad4 regulated secreted biomarkers in colon cancer", PROTEOMICS, vol. 5, no. 10, 2005, pages 2587-2601, XP055068469,
- ZIMMERMANN-NIELSEN E ET AL: "Complement activation mediated by mannan-binding lectin in plasma from healthy individuals and from patients with SLE, Crohn's disease and colorectal cancer. Suppressed activation by SLE plasma", SCANDINAVIAN JOURNAL OF IMMUNOLOGY, vol. 55, 2002, pages 105-110, XP002261993,
- DEUG-CHAN LEE ET AL: "Protein profiling underscores immunological functions of uterine cervical mucus plug in human pregnancy", JOURNAL OF PROTEOMICS, vol. 74, no. 6, 2011, pages 817-828, XP028481130,
- XIAOYANG LUO ET AL: "A high-quality secretome of A549 cells aided the discovery of C4b-binding protein as a novel serum biomarker for non-small cell lung cancer", JOURNAL OF PROTEOMICS, vol. 74, no. 4, 2011, pages 528-538, XP028369097,
- DATABASE MEDLINE [Online] US NATIONAL LIBRARY OF MEDICINE (NLM), BETHESDA, MD, US; August 1990 (1990-08), YUAN M: "[Value of fecal detection of cancer-associated antigens using monoclonal antibodies in the diagnosis of colorectal cancer].", XP002690733, Database accession no. NLM2086118 & ZHONGHUA WAI KE ZA ZHI [CHINESE JOURNAL OF SURGERY] AUG 1990, vol. 28, no. 8, August 1990 (1990-08), pages 497-500 , 511, ISSN: 0529-5815
- CHING-SENG ANG ET AL: "Targeted In-Gel MRM: A Hypothesis Driven Approach for Colorectal Cancer Biomarker Discovery in Human Feces", JOURNAL OF PROTEOME RESEARCH, vol. 9, no. 9, 2010, pages 4346-4355, XP055050298,
- L. Roncucci ET AL: "Myeloperoxidase-Positive Cell Infiltration in Colorectal Carcinogenesis as Indicator of Colorectal Cancer Risk", CANCER EPIDEMIOLOGY, BIOMARKERS AND PREVENTION., vol. 17, no. 9, 1 September 2008 (2008-09-01), pages 2291-2297, XP055490020, US ISSN: 1055-9965, DOI: 10.1158/1055-9965.EPI-08-0224

## Description

### Field of the Invention

The present invention relates to the identification of biomarkers of colorectal cancer precursors, i.e. high risk adenomas, and their use in screening methods, as well as arrays for use in advanced colorectal adenoma screening methods.

### Background of the Invention

Colorectal cancer (CRC) is a significant health problem. It is the 3rd most common cancer worldwide, with over 1.200.000 new cases each year, with a fatal outcome for almost half the patients (Karsa LV, et al. Best Pract Res Clin Gastroenterol 2010; 24:381-96.). Because CRC develops over many years, there is an excellent window of opportunity to detect the disease in an early, curable, or even premalignant stage. This can be achieved by screening of asymptomatic individuals. However, the currently available screening tests are either cumbersome or carry risk of complications and over treatment like colonoscopy.

The immunochemical fecal occult blood test (iFOBT), further referred to as fecal immunochemical test (FIT), is a widely used test that detects small traces of blood in stool, derived from lesions such as tumors that bleed in the colon. The FIT is a non-invasive method that makes use of an antibody directed against hemoglobin. However, not all colon tumors bleed and therefore the FIT has a sensitivity that leaves room for improvement (Duffy MJ, et al. Int J Cancer 2011; 128:3-11; van Veen W, Mali WP. [Colorectal cancer screening: advice from the Health Council of the Netherlands]. Ned Tijdschr Geneeskd 2009; 153:A1441.). Additional markers that detect other tumor characteristics besides bleeding in the stool could increase this sensitivity and the chance of identifying a colon tumor. Molecular changes resulting from the neoplastic process include changes in protein expression. The proteins for which expression is increased have the potential to serve as informative biomarkers with high diagnostic performance. US 5,552,292 discloses screening for colorectal cancer by using proteins such as myeloperoxidase as biomarker in feces.

Several studies have been performed to identify proteins in stool and blood that can be used for the early detection of CRC and high risk colorectal adenomas, but most of these proteins have not been validated in a screening setting or failed to improve current tests for early detection, such as Carcinoembryonic antigen (CEA) or Calprotectin (Bosch LJ, et al. Molecular tests for colorectal cancer screening. Clin Colorectal Cancer 2011; 10:8-23.). There therefore remains a need for further tumor-specific protein markers to improve the current available non-invasive screening possibilities for CRC and high risk adenomas.

Recent technological advances in mass spectrometry can boost the discovery of novel protein markers (de Wit M, et al. Gut 2011**;** Jimenez CR, et al. J Proteomics 2010; 73: 1873-95). Tandem mass spectrometry-based approaches now have the power to analyze complex protein samples and to detect proteins in low concentrations (Cox J, Mann M. Annu Rev Biochem 2011; 80: 273-99). Although most biomarker discovery studies have been performed using tissue and/or cell line material followed by validation in stool or blood, the chemical composition of the biological sample used for screening may significantly affect the nature of biomarkers that can be identified. This holds especially for stool samples, in which the low pH, the protease- and glycosidase activities of bacteria, enzymes and other substances can disturb the specific detection of these markers (Young GP, Bosch LJW. Curr Colorectal Cancer Rep 2011; 7: 62-70). Measuring molecules directly in the biological sample that may be used for screening, i.e. stool, could therefore be a valuable approach to provide us with reliable biomarkers that are stable in the fecal environment. A recent study by Ang et al. has shown the feasibility of protein biomarker discovery in human stool samples using mass spectrometry (Ang CS, Nice EC. J Proteome Res 2010; 9: 4346-55).

The present invention aims to overcome some or all of the problems associated with the prior art.

### Summary of the Invention

According to a first aspect of the invention there is provided a method for screening for the presence of advanced colorectal adenoma, the method comprising:screening a stool sample obtained from an individual for two or more biomarkers comprising myeloperoxidase (MPO) and one or more biomarkers selected from the group consisting of: azurocidin 1 (AZU1), cathepsin G (CTSG), defensin, alpha 1 (DEFA1), elastase, neutrophil expressed (ELANE), integrin, alpha M (complement component 3 receptor 3 subunit) (ITGAM), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), annexin A2 (ANXA2), serpin peptidase inhibitor, clade B (ovalbumin), member 5 (SERPINB5), wherein the presence of or increased expression of MPO and the one or more biomarkers, relative to a control sample, is indicative that the individual is at risk of suffering from or is suffering from advanced colorectal adenoma. In a second embodiment, the first aspect of the invention comprises screening for two or more of the further biomarkers.

In a third embodiment, the first aspect of the invention comprises screening for four or more of the further biomarkers.

In a fourth embodiment, the first aspect of the invention comprises screening for all sixteen biomarkers.

In a fifth embodiment, the first aspect of the invention the stool sample is screened for the two or more biomarkers using targeted mass spectrometry.

In a sixth embodiment, the first aspect of the invention the stool sample is screened for the two or more biomarkers using binding agents capable of binding to the two or more biomarkers.

In a seventh embodiment, the first aspect of the invention the binding agents are antibodies or fragments thereof.

In the eighth embodiment, said antibodies or fragments thereof are selected from the group consisting of scFv, Fab, and a binding domain of an immunoglobulin molecule.

In a ninth embodiment, the first aspect of the invention the screening is performed using an array, optionally wherein the array is a bead-based array or a surface-based array.

In a tenth embodiment, the first aspect of the invention the control sample comprises a biological sample from an individual known to be free from advanced colorectal adenoma.

In the eleventh embodiment, the first aspect of the invention the biological sample is also analysed by the fecal immunochemical test.

In a second aspect the invention concerns the use of two or more biomarkers comprising myeloperoxidase (MPO), and one or more biomarkers selected from the group consisting of: azurocidin 1 (AZU1), cathepsin G (CTSG), defensin, alpha 1 (DEFA1), elastase, neutrophil expressed (ELANE), integrin, alpha M (complement component 3 receptor 3 subunit) (ITGAM), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), annexin A2 (ANXA2), serpin peptidase inhibitor, clade B (ovalbumin), member 5 (SERPINB5) as a biomarker in stool samples for diagnosing or predicting the presence of advanced colorectal adenoma in an individual.

According to a third aspect the invention concerns the use of an array for determining whether an individual is at risk of suffering from or is suffering from advanced colorectal adenoma according to the method of the first aspect of the invention, the array comprising one or more binding agents as defined in any one of embodiments 6 to 8 mentioned above.

In a fourth aspect the invention concerns the use of myeloperoxidase (MPO) as a biomarker in stool samples for screening for the presence of advanced colorectal adenoma.

Any one or more features described for any aspect of the present invention or preferred embodiments or examples thereof, described herein, may be used in conjunction with any one or more other features described for any other aspect of the present invention or preferred embodiments or examples thereof described herein. The fact that a feature may only be described in relation to one aspect or embodiment or example does not limit its relevance to only that aspect or embodiment or example if it is technically relevant to one or more other aspect or embodiment or example.

### Detailed Description of the Invention

### Colorectal cancer

The most common colon cancer cell type is adenocarcinoma which accounts for 95% of cases. Other, rarer types include lymphoma and squamous cell carcinoma. Colorectal adenocarcinoma arises from precursor lesions called adenomas, of which only a minority progress to cancer. Adenomas that progress to cancer are referred to as high risk adenomas.

Protein markers have great potential to be applied for stool-based CRC screening, because they can be measured in small sample volumes with simple and relatively cheap assays, of which the widely used FIT is an excellent example (Bosch LJ, et al. Molecular tests for colorectal cancer screening. Clin Colorectal Cancer 2011; 10; 8-23; Young GP, Bosch LJW. Curr Colorectal Cancer Rep 2011; 7: 62-70; Oort FA, et al. Aliment Pharmacol Ther 2010; 31: 432-9).

The present study has identified novel protein biomarkers by applying in-depth proteomics to stool samples. From a total of 830 detected human proteins, 134 were significantly enriched in stool samples from CRC patients compared to control stool samples, of which several showed higher discriminative power than hemoglobin and/or complementarity to hemoglobin.

The approach of measuring molecules directly in stool is of significance to reveal biomarkers that are stable in the fecal environment and detectable in the background of bacterial- and food-related molecules.

The present disclosure is advantageously used for screening for colorectal cancer, that is adenocarcinoma found in the colon. However, the methods of the disclosure should not be considered as being limited solely to the detection of colonic adenocarcinomas. Rather, the methods of the invention are also useful in the detection of advanced or high-risk colonic adenomas, thus enabling the identification of an individual at risk of developing colorectal cancer due to the presence of an advanced or a high-risk adenoma.

References herein to screening for colorectal cancer thus may include screening for advanced colonic adenomas and high-risk adenomas as well as colonic adenocarcinoma.

It is also expected that the biomarkers identified by the present disclosure may also find application for the diagnosis of adenocarcinomas present higher up the gastrointestinal tract.

Thus, the present disclosure may also provide a method for screening for gastrointestinal disease or gastrointestinal cancer, the method comprising: screening a biological sample, for example a stool sample, from an individual for one or more biomarkers selected from the group defined in Table 1, wherein the presence of or increased expression of the one or more biomarkers relative to a control sample is indicative that the patient is at risk of suffering from or is suffering from gastrointestinal disease or gastrointestinal cancer.

### Sample for screening

The sample for screening may include cell lines, biopsies, whole blood, blood serum, sputum, stool, urine, synovial fluid, wound fluid, cerebral-spinal fluid, tissue from eyes, intestine, kidney, brain, skin, heart, prostate, lung, breast, liver, muscle or connective tissue, the said tissue being optionally embedded in paraffin, histologic object slides, and all possible combinations thereof.

According to the invention the biological sample is stool. The sample may be prepared by any conventional method for extracting proteins from a biological sample. One exemplary method can be found in Ang CS, Nice EC. J Proteome Res. 2010; 9:4346-55.

### Biomarkers

The present disclosure provides a set of biomarkers which may be detected directly from a stool sample and which have been shown to be reliable indicators for the presence of advanced colonic adenomas or adenocarcinomas in an individual.

The biomarkers identified are listed in Table 1 and/or Table 6. In one embodiment, the methods of the invention screen for more than one biomarker from the group defined in Table 1, for example two, three, four, five, six, seven, eight, nine, ten of the biomarkers of the group defined in Table 1 and/or Table 6. In an alternative embodiment, the methods of the invention screen for more than ten of the biomarkers of the group defined in Table 1, for example, eleven, twelve, thirteen, fourteen, fifteen, sixteen, seventeen, eighteen, nineteen, twenty, thirty, forty, fifty, sixty, seventy, eighty, ninety, one hundred of the biomarkers of the group defined in Table 1 and/or Table 6.

Thus, the methods of the disclosure screen for the presence of or increased expression of one or more of: complement component C4B (Chido blood group) 2 (C4A/C4B); glutamic-oxaloacetic transaminase 2, mitochondrial (aspartate aminotransferase 2) (GOT2); glucose-6-phosphate isomerase (GPI); transketolase (TKT); N-acylaminoacyl-peptide hydrolase (APEH); histone cluster 1, H4c (HIST4H4 (includes others)); Fatty acid-binding protein 5 (psoriasis-associated) (FABP5); hexosaminidase B (beta polypeptide) (HEXB); epithelial cell adhesion molecule (EPCAM); NME1-NME2 walkthrough (NME1-NME2); Superoxide dismutase 2, mitochondrial (SOD2); Tu translation elongation factor, mitochondrial (TUFM); Glutathione synthetase (GSS); annexin A2 (ANXA2); ATP synthase, H+ transporting, mitochondrial F1 complex, beta polypeptide (ATP5B); 10 kDa heat shock protein (chaperonin 10) (HSPE1); glyoxalase I (GLO1); histone cluster 2, H2be (HIST2H2BE (includes others)); S100 calcium binding protein A4 (S100A4); S100 calcium binding protein A11 (S100A11); latexin (LXN); dehydrogenase/reductase (SDR family) member 11 (DHRS11); N-acetylglucosaminidase, alpha (NAGLU); Translin (TSN); Proteasome (prosome, macropain) subunit alpha type-4 (PSMA4); Proteasome (prosome, macropain) subunit alpha type-6 (PSMA6); ras-related C3 botulinum toxin substrate 1 (rho family, small GTP binding protein Rac1) (RAC1); Adenosylhomocysteinase (AHCY); fucosidase, alpha-L- 1, tissue (FUCA1); S100 calcium binding protein P (S100P); Proteasome (prosome, macropain) subunit beta type-2 (PSMB2); X-prolyl aminopeptidase (aminopeptidase P) 1 (XPNPEP1); Keratin 18 (KRT18); Nuclear cap-binding protein subunit 1 80 kDa (NCBP1); mannosidase, alpha, class 2B, member 1 (MAN2B1); S100 calcium binding protein A6 (S100A6); valosin containing protein (VCP); quinolinate phosphoribosyltransferase (QPRT); major histocompatibility complex, class I, B (HLA-B); phosphoglycerate mutase 1 (brain) (PGAM1); ectonucleotide pyrophosphatase/phosphodiesterase 3 (ENPP3); serpin peptidase inhibitor, clade B (ovalbumin), member 10 (SERPINB10); myeloperoxidase (MPO); creatine kinase, mitochondrial 1B (CKMT1A/CKMT1B); proteinase 3 (PRTN3); elastase, neutrophil expressed (ELANE); MORC family CW-type zinc finger 1 (MORC1); ubiquitin B (UBB); phospholipase A2, group IIA (platelets, synovial fluid) (PLA2G2A); carbonic anhydrase IV (CA4); G elongation factor, mitochondrial 2 (GFM2); S100 calcium binding protein A7 (S100A7); Bactericidal permeability-increasing protein (BPI); collagen, type VI, alpha 5 (COL6A5); LIM homeobox 8 (LHX8); cysteine-rich secretory protein 3 (CRISP3); Azurocidin (AZU1); hemicentin 1 (HMCN1); Transglutaminase 3 (E polypeptide, protein-glutamine gamma-glutamyltransferase) (TGM3); CDC42 binding protein kinase alpha (DMPK-like) (CDC42BPA); Cathepsin G (CTSG); Resistin (RETN); methylmalonyl CoA mutase (MUT); armadillo repeat containing, X-linked 4 (ARMCX4); Integrin alpha-M (complement component 3 receptor 3 subunit) (ITGAM); Calcium channel, voltage dependent, R-type alpha-1E subunit (CACNA1E); T-cell lymphoma invasion and metastasis 2 (TIAM2); HIR histone cell cycle regulation defective homolog A (S. cerevisiae) (HIRA); dopey family member 2 (DOPEY2); integrin beta 1 binding protein 3 (ITGB1BP3); Sodium channel, voltage-gated, type VII, alpha (SCN7A); Rab3C, member RAS oncogene family (RAB3C); chromosome 9 open reading frame 79 (C9orf79); nuclear factor of activated T-cells, cytoplasmic, calcineurin-dependent 4 (NFATC4); UDP-glucose glycoprotein glucosyltransferase 2 (UGGT2); Cornulin (CRNN); kielin/chordin-like protein (KCP); CD1E molecule (CD1E); coiled-coil domain-containing 18 (CCDC18); leukotriene A-4 hydrolase (LTA4H); albumin (ALB); alpha-2-macroglobulin (A2M); complement component 3 (C3); hemoglobin, beta (HBB); transferrin (TF); hemoglobin, alpha 1 (HBA1/HBA2); lactotransferrin (LTF); ceruloplasmin (ferroxidase) (CP); catalase (CAT); group-specific component (vitamin D-binding protein) (GC); serpin peptidase inhibitor, clade C (antithrombin), member 1 (SERPINC1); fibrinogen gamma chain (FGG); S100 calcium binding protein A8 (S100A8); ferritin, light polypeptide (FTL); actin, beta (ACTB); fibronectin 1 (FN1); defensin, alpha 1 (DEFA1 (includes others)); serpin peptidase inhibitor, clade G (C1 inhibitor), member 1 (SERPING1); retinol binding protein 4, plasma (RBP4); peroxiredoxin 2 (PRDX2); fibrinogen alpha chain (FGA); serpin peptidase inhibitor, clade F (alpha-2 antiplasmin, pigment epithelium derived factor), member 2 (SERPINF2); carbonic anhydrase II (CA2); orosomucoid 1 (ORM1/ORM2); lactate dehydrogenase A (LDHA); vitronectin (VTN); kininogen-1 (KNG1); actin, alpha, cardiac muscle 1 (ACTC1); leucine-rich alpha-2-glycoprotein 1 (LRG1); gamma-glutamyl hydrolase (conjugase, folylpolygammaglutamyl hydrolase) (GGH); enolase 1, (alpha) (ENO1); profilin 1 (PFN1); serpin peptidase inhibitor, clade A (alpha-1 antiproteinase, antitrypsin), member 7 (SERPINA7); alpha-1-microglobulin/bikunin precursor (AMBP); lamin A/C (LMNA); apolipoprotein D (APOD); thyroid hormone receptor interactor 11 (TRIP11); complement component 4 binding protein, alpha (C4BPA); tropomyosin 4 (TPM4); filamin A, alpha (FLNA); haptoglobin (HP); hemopexin (HPX); hemoglobin, delta (HBD); fibrinogen beta chain (FGB); S100 calcium binding protein A9 (S100A9); complement component 5 (C5); solute carrier family 26, member 3 (SLC26A3); complement component 9 (C9); amyloid P component, serum (APCS); alpha-1-B glycoprotein (A1BG); complement C3-like (LOC100133511); inter-alpha (globulin) inhibitor H4 (plasma Kallikrein-sensitive glycoprotein) (ITIH4); complement component C8, alpha polypeptide (C8A); inter-alpha (globulin) inhibitor H1 (ITIH1); acyl-CoA dehydrogenase, very long chain (ACADVL); cDNA FLJ60317, highly similar to Aminoacylase-1 (ACY1); Ankyrin repeat domain-containing protein 35 (ANKRD35); baculoviral IAP repeat-containing 6 (BIRC6); Bleomycin hydrolase (BLMH); bone marrow stromal cell antigen 12 (BST1); hypothetical protein LOC643677 (C13orf40); Cytidine deaminase (CDA); chitinase 1 (chitotriosidase) (CHIT1); cathepsin C (CTSC); Cathepsin S (CTSS); Isoform 2 of Dedicator of cytokinesis protein 4 (DOCK4); Glutathione reductase (GSR); hect (homologous to the E6-AP (UBE3A) carboxyl terminus) domain and RCC1 (CHC1)-like domain (RLD) 1 (HERC1); hect domain and RLD 2 (HERC2); major histocompatibility complex, class II, DR beta 5 (HLA-DRB5); isocitrate dehydrogenase 1 (NADP+), soluble (IDH1); inter-alpha (globulin) inhibitor H2 (ITIH2); Uncharacterized protein KIAA1797 (KIAA1797); Lysozyme C (LYZ); Nebulin (NEB); NIMA (never in mitosis gene a)- related kinase 10 (NEK10); peptidase D (PEPD); quiescin Q6 sulfhydryl oxidase 1 (QSOX1); ribonuclease T2 (RNASET2); serpin peptidase inhibitor, clade A (alpha-1 antiproteinase, antitrypsin), member 1 (SERPINA1); serpin peptidase inhibitor, clade A (alpha-1 antiproteinase, antitrypsin), member 3 (SERPINA3); serpin peptidase inhibitor, clade B (ovalbumin), member 3 (SERPINB3); SET domain containing 2 (SETD2); Shugoshin-like 2 (SGOL2); sialic acid acetylesterase (SIAE); spectrin repeat containing, nuclear envelope 1 (SYNE1); Transaldolase 1 (TALDO1); Taste receptor type 2 member 42 (TAS2R42); triosephosphate isomerase 1 (TPI1); Vinculin (VCL); Zymogen granule membrane protein 16 (ZG16); hypothetical protein LOC79887 (PLBD1); Isoform 1 of Serine/threonine-protein phosphatase 6 regulatory ankyrin repeat subunit A (ANKRD28); Cystatin-C (CST3); D-dopachrome decarboxylase (DDT); Synapse-associated protein 1 (SYAP1); Proteasome subunit alpha type-2 (PSMA2); SUB1 homolog (S. cerevisiae) (SUB1); Microfibril-associated glycoprotein 3 (MFAP3); Cathepsin D (CTSD); proteasome (prosome, macropain) subunit, beta type, 1 (PSMB1); proteasome (prosome, macropain) subunit, beta type, 5 (PSMB5); cDNA FLJ61112, highly similar to BTB/POZ domain-containing protein KCTD15 (KCTD15); prolyl 4-hydroxylase, beta polypeptide (P4HB); glutathione peroxidase 1 (GPX1); serpin peptidase inhibitor, clade B (ovalbumin), member 5 (SERPINB5); Isoform 1 of collagen, type IV, alpha 3 (Goodpasture antigen) binding protein (COL4A3BP); proteasome (prosome, macropain) subunit, beta type, 6 (PSMB6); Keratin 20 (KRT20); Calpain small subunit 1 (CAPNS1); peroxiredoxin 3 (PRDX3); NACC family member 2, BEN and BTB (POZ) domain containing (NACC2); Rho GDP-dissociation inhibitor 2 (ARHGDIB); Macrophage migration inhibitory factor (MIF); Ran-binding protein 6 (RANBP6); spinster homolog 3 (Drosophila) (SPNS3); minichromosome maintenance complex component 2 (MCM2); Fumarylacetoacetase (FAH); heat shock 70kDa protein 8 (HSPA8); brain abundant, membrane attached signal protein 1 (BASP1); Branched-chain-amino-acid aminotransferase (BCAT2); Moesin (MSN); serpin peptidase inhibitor, clade B (ovalbumin), member 8 (SERPINB8); glucose-6-phosphate dehydrogenase (G6PD); Isoform 1 of UPF0557 protein C10orf119 (C10orf119); Prosaposin (PSAP); eukaryotic translation elongation factor 1 gamma (EEF1G); four and a half LIM domains 1 (FHL1); carboxypeptidase, vitellogenic-like (CPVL); tubulin tyrosine ligase-like family, member 3 (TTLL3); IPI:IPI00942608.1|ENSEMBL:ENSP0000 (unmapped; 26kDa protein); proline-rich protein BstNI subfamily 2 (PRB1/PRB2); Protocadherin-8 (PCDH8); Alpha-2-macroglobulin-like protein 1 (A2ML1); Guanine deaminase (GDA); Lipocalin-1 (LCN1); Histone H1.4 (HIST1H1E); IPI:IPI00937064.1|REFSEQ:XP_002342720 (ZAN); heterogeneous nuclear ribonucleoprotein A2/B1 (HNRNPA2B1);); Endoplasmic reticulum aminopeptidase 2 (ERAP2); 14-3-3 protein zeta/delta (YWHAZ); G-protein coupled receptor 39 (GPR39); similar to KIAA1783 protein (KIAA1783 protein); apolipoprotein A-I binding protein (APOA1BP); pleckstrin and Sec7 domain containing 2 (PSD2); prolylcarboxypeptidase (angiotensinase C) (PRCP); Tubulin alpha-1C chain (TUBA1C); Calmodulin-like protein 5 (CALML5); ARP3 actin-related protein 3 homolog (yeast) (ACTR3); myosin, light chain 6, alkali, smooth muscle and non-muscle (MYL6); Vasodilator-stimulated phosphoprotein (VASP); ARP2 actin-related protein 2 homolog (yeast) (ACTR2); Rheumatoid factor (RF-IP18); Phosphoglycerate kinase 1 (PGK1); Solute carrier family 35 member F1 (SLC35F1); Solute carrier family 35 member F1 (SLC35F1); alkaline phosphatase, liver/bone/kidney (ALPL); I tropomyosin 3 (TPM3); Hexokinase-3 (HK3); Vimentin (VIM); Annexin A1 (ANXA1); IPI:IPI00930073.1|TREMBL:B2R853 (KRT6C); Keratin, type II cytoskeletal 6C (KRT6C); myosin, heavy chain 13, skeletal muscle (MYH13); cell cycle progression 1 (CCPG1); Hypothetical protein (H-INV); calcium channel, voltage-dependent, L type, alpha 1D subunit (CACNA1D); LY6/PLAUR domain containing 5 (LYPD5); aarF domain containing kinase 2 (ADCK2); Myosin-Ic (MYO1C); amyloid beta precursor protein (cytoplasmic tail) binding protein 2 (APPBP2); integrin, alpha 2b (platelet glycoprotein IIb of IIb/IIIa complex, antigen CD41) (ITGA2B); tubulin, beta 6 (TUBB6); synaptotagmin-like 4 (SYTL4); aquaporin 4 (AQP4); cell division cycle 42 (GTP binding protein, 25kDa) (CDC42); myosin, light chain 12B, regulatory (MYL12B); protein L-Myc-2-like (LOC100293553); RAP1B, member of RAS oncogene family (RAP1B); glycoprotein IX (platelet) (GP9); Destrin (DSTN); complement component 1, q subcomponent, C chain (C1QC); epidermal growth factor receptor pathway substrate 8 (EPS8); dual specificity phosphatase 3 (DUSP3); ras homolog gene family, member A (RHOA); myosin, light chain 9, regulatory (MYL9); peptidylprolyl isomerase A (cyclophilin A) (PPIA); Cofilin-1 (CFL1); and/or lactotransferrin (LTF), collagen, type XII, alpha 1 (COL12A1), agrin (AGRN), +MYB binding protein (P160) 1a (MYBBP1A), transformation/transcription domain-associated protein (TRRAP), annexin A6 (ANXA6), cytoskeleton associated protein 5 (CKAP5), minichromosome maintenance complex component 5 (MCM5), importin 4 (IPO4), neurobeachin-like 2 (NBEAL2), minichromosome maintenance complex component 4 (MCM4), 2'-5'-oligoadenylate synthetase 3, 100kDa (OAS3), minichromosome maintenance complex component 3 (MCM3), NEDD8 activating enzyme E1 subunit 1 (NAE1), tripartite motif containing 28 (TRIM28), fused in sarcoma (FUS), phenylalanyl-tRNA synthetase, alpha subunit (FARSA), myeloid cell nuclear differentiation antigen (MNDA), suppressor of Ty 16 homolog (S. cerevisiae) (SUPT16H), DEAD (Asp-Glu-Ala-Asp) box polypeptide 5 (DDX5), tenascin C (TNC), nuclear import 7 homolog (S. cerevisiae) (NIP7), chromodomain helicase DNA binding protein 4 (CHD4), regulator of chromosome condensation 2 (RCC2), DNA (cytosine-5-)-methyltransferase 1 (DNMT1), exportin 4 (XPO4), chaperonin containing TCP1, subunit 5 (epsilon) (CCT5), serine/arginine-rich splicing factor 9 (SRSF9), spectrin, beta, non-erythrocytic 2 (SPTBN2), TIMP metallopeptidase inhibitor 1 (TIMP1), nidogen 1 (NID1), ribonucleotide reductase M1 (RRM1), eukaryotic translation initiation factor 4 gamma, 1 (EIF4G1), component of oligomeric golgi complex 4 (COG4), polymerase (DNA directed), delta 1, catalytic subunit 125kDa (POLD1), splicing factor 3b, subunit 2, 145kDa (SF3B2), exosome component 2 (EXOSC2), minichromosome maintenance complex component 6 (MCM6), plastin 3 (PLS3), aldolase B, fructose-bisphosphate (ALDOB), SMG1 homolog, phosphatidylinositol 3-kinase-related kinase (C. elegans) (SMG1), G1 to S phase transition 1 (GSPT1), KH-type splicing regulatory protein (KHSRP), DEAD (Asp-Glu-Ala-Asp) box polypeptide 21 (DDX21), phosphatidylinositol transfer protein, beta (PITPNB), aquarius homolog (mouse) (AQR), heterogeneous nuclear ribonucleoprotein D-like (HNRPDL), annexin A3 (ANXA3), processing of precursor 1, ribonuclease P/MRP subunit (S. cerevisiae) (POP1), structural maintenance of chromosomes 2 (SMC2), dynein, cytoplasmic 1, light intermediate chain 2 (DYNC1LI2), peptidylprolyl isomerase D (PPID), vacuolar protein sorting 37 homolog B (S. cerevisiae) (VPS37B), adrenergic, beta, receptor kinase 1 (ADRBK1), DIS3 mitotic control homolog (S. cerevisiae) (DIS3), polymerase (RNA) I polypeptide A, 194kDa (POLR1A), t-complex 1 (TCP1), plakophilin 3 (PKP3), La ribonucleoprotein domain family, member 1B (LARP1B), poly (ADP-ribose) polymerase 1 (PARP1), CD46 molecule, complement regulatory protein (CD46),
p21 protein (Cdc42/Rac)-activated kinase 2 (PAK2), ATP-binding cassette, sub-family E (OABP), member 1 (ABCE1), ubiquitin specific peptidase 14 (tRNA-guanine transglycosylase) (USP14), chaperonin containing TCP1, subunit 3 (gamma) (CCT3), Ran GTPase activating protein 1 (RANGAP1), deoxythymidylate kinase (thymidylate kinase) (DTYMK), N-myristoyltransferase 1 (NMT1), dynamin 1-like (DNM1L), interferon induced transmembrane protein 2 (1-8D) (IFITM2), fermitin family member 1 (FERMT1), tubulin folding cofactor D (TBCD), serine/arginine-rich splicing factor 10 (LOC100505793/SRSF10), STE20-like kinase (SLK), mucin 5AC, oligomeric mucus/gel-forming (MUC5AC/MUC5B), methionyl-tRNA synthetase (MARS), SMEK homolog 1, suppressor of mek1 (Dictyostelium) (SMEK1), high mobility group box 2 (HMGB2), non-POU domain containing, octamer-binding (NONO), transforming growth factor, beta-induced, 68kDa (TGFBI), fibulin 2 (FBLN2), high density lipoprotein binding protein (HDLBP), collagen, type IV, alpha 2 (COL4A2), copine I (CPNE1), N(alpha)-acetyltransferase 50, NatE catalytic subunit (NAA50), LSM7 homolog, U6 small nuclear RNA associated (S. cerevisiae) (LSM7), structure specific recognition protein 1 (SSRP1), importin 8 (IPO8), yippee-like 5 (Drosophila) (YPEL5), phosphoglucomutase 3 (PGM3), ring finger protein 40 (RNF40), structural maintenance of chromosomes 3 (SMC3), regenerating islet-derived family, member 4 (REG4), splicing factor 3a, subunit 3, 60kDa (SF3A3), thrombospondin 1 (THBS1), chaperonin containing TCP1, subunit 6A (zeta 1) (CCT6A), PRP8 pre-mRNA processing factor 8 homolog (S. cerevisiae) (PRPF8), symplekin (SYMPK), far upstream element (FUSE) binding protein 1 (FUBP1), U2 small nuclear RNA auxiliary factor 1 (U2AF1), huntingtin (HTT), eukaryotic translation initiation factor 5B (EIF5B), nuclear autoantigenic sperm protein (histone-binding) (NASP), heterogeneous nuclear ribonucleoprotein K (HNRNPK), Y box binding protein 1 (YBX1), annexin A11 (ANXA11), RecQ protein-like (DNA helicase Q1-like) (RECQL), cortactin (CTTN), tubulin, beta 3 (TUBB3), pyrroline-5-carboxylate reductase-like (PYCRL), periplakin (PPL), phosphoglucomutase 2-like 1 (PGM2L1), chromosome 17 open reading frame 49 (C17orf49), mRNA turnover 4 homolog (S. cerevisiae) (MRTO4), methyltransferase like 1 (METTL1), squamous cell carcinoma antigen recognized by T cells 3 (SART3), S100 calcium binding protein A13 (S100A13), aminopeptidase-like 1 (NPEPL1), cyclin-dependent kinase 1 (CDK1), ubiquitin protein ligase E3 component n-recognin 1 (UBR1), Rho GTPase activating protein 18 (ARHGAP18), signal recognition particle 14kDa (homologous Alu RNA binding protein) (SRP14), cathelicidin antimicrobial peptide (CAMP), splicing factor proline/glutamine-rich (SFPQ), RAS p21 protein activator (GTPase activating protein) 1 (RASA1), Ral GTPase activating protein, beta subunit (non-catalytic) (RALGAPB), laminin, beta 1 (LAMB1), RAB3 GTPase activating protein subunit 2 (non-catalytic) (RAB3GAP2), chaperonin containing TCP1, subunit 8 (theta) (CCT8), heterogeneous nuclear ribonucleoprotein L-like (HNRPLL), RAN binding protein 1 (RANBP1), kinetochore associated 1 (KNTC1), dyskeratosis congenita 1, dyskerin (DKC1), casein kinase 2, alpha 1 polypeptide (CSNK2A1), CAP-GLY domain containing linker protein 1 (CLIP1), chaperonin containing TCP1, subunit 2 (beta) (CCT2), tubulin tyrosine ligase-like family, member 12 (TTLL12), ataxia telangiectasia mutated (ATM), splicing factor 3a, subunit 1, 120kDa (SF3A1), ribosomal protein S20 (RPS20), ubiquitin-conjugating enzyme E2O (UBE2O), translocated promoter region (to activated MET oncogene) (TPR), BRCA2 and CDKN1A interacting protein (BCCIP), gem (nuclear organelle) associated protein 5 (GEMIN5), ribonuclease P/MRP 30kDa subunit (RPP30), loss of heterozygosity, 12, chromosomal region 1 (LOH12CR1), syntaxin binding protein 2 (STXBP2), ubiquitin-conjugating enzyme E2H (UBE2H), DIP2 disco-interacting protein 2 homolog B (Drosophila) (DIP2B), RAP1, GTP-GDP dissociation stimulator 1 (RAP1GDS1), heterogeneous nuclear ribonucleoprotein M (HNRNPM), LIM domain 7 (LMO7), RNA binding motif protein 25 (RBM25), aldehyde dehydrogenase 7 family, member A1 (ALDH7A1), cleavage and polyadenylation specific factor 1, 160kDa (CPSF1), calponin 2 (CNN2), chaperonin containing TCP1, subunit 7 (eta) (CCT7), lysyl-tRNA synthetase (KARS), UDP-N-acteylglucosamine pyrophosphorylase 1 (UAP1), heat shock 70kDa protein 4-like (HSPA4L), 138 kDa protein (138 kDa protein), thimet oligopeptidase 1 (THOP1), glutaredoxin 3 (GLRX3), phosphoglycerate dehydrogenase (PHGDH), CDV3 homolog (mouse) (CDV3), structural maintenance of chromosomes 4 (SMC4), RNA binding motif (RNP1, RRM) protein 3 (RBM3), hepatoma-derived growth factor (HDGF), heterogeneous nuclear ribonucleoprotein U (scaffold attachment factor A) (HNRNPU), nuclear receptor binding protein 1 (NRBP1), polymerase (RNA) I polypeptide B, 128kDa (POLR1B), protein phosphatase 5, catalytic subunit (PPP5C), glucose-6-phosphate dehydrogenase (G6PD), arginase, liver (ARG1), 3-hydroxy-3-methylglutaryl-CoA synthase 1 (soluble) (HMGCS1), ubiquitin-like modifier activating enzyme 2 (UBA2), KIAA1033 (KIAA1033), annexin A4 (ANXA4), DEAD (Asp-Glu-Ala-Asp) box polypeptide 17 (DDX17), acidic (leucine-rich) nuclear phosphoprotein 32 family, member E (ANP32E), glucosamine (UDP-N-acetyl)-2-epimerase/N-acetylmannosamine kinase (GNE), KIAA0368 (KIAA0368), vacuolar protein sorting 4 homolog B (S. cerevisiae) (VPS4B), replication protein A1, 70kDa (RPA1), eukaryotic translation initiation factor 2, subunit 1 alpha, 35kDa (EIF2S1), eukaryotic translation initiation factor 3, subunit J (EIF3J), suppressor of Ty 6 homolog (S. cerevisiae) (SUPT6H), heat shock 105kDa/110kDa protein 1 (HSPH1), exportin 5 (XPO5), transcription elongation factor A (SII), 1 (TCEA1), Sjogren syndrome antigen B (autoantigen La) (SSB), AE binding protein 1 (AEBP1), LIM and cysteine-rich domains 1 (LMCD1), interleukin enhancer binding factor 3, 90kDa (ILF3), WD repeat domain 61 (WDR61), N(alpha)-acetyltransferase 15, NatA auxiliary subunit (NAA15), serine/arginine-rich splicing factor 4 (SRSF4), ring finger protein 20 (RNF20), lactamase, beta 2 (LACTB2), NHP2 ribonucleoprotein homolog (yeast) (NHP2), chromosome 17 open reading frame 28 (C17orf28), CTP synthase II (CTPS2), fascin homolog 1, actin-bundling protein (Strongylocentrotus purpuratus) (FSCN1), tRNA nucleotidyl transferase, CCA-adding, 1 (TRNT1), splicing regulatory glutamine/lysinerich protein 1 (SREK1),stromal antigen 1 (STAG1), oxysterol binding protein (OSBP), deoxyuridine triphosphatase (DUT), coiled-coil domain containing 25 (CCDC25), DEK oncogene (DEK), coiled-coil domain containing 72 (CCDC72), polymerase (RNA) II (DNA directed) polypeptide E, 25kDa (POLR2E), phosphoserine phosphatase (PSPH), structural maintenance of chromosomes 1A (SMC1A), DEAD (Asp-Glu-Ala-Asp) box polypeptide 23 (DDX23), tRNA methyltransferase 11-2 homolog (S. cerevisiae) (TRMT112), COP9 constitutive photomorphogenic homolog subunit 2 (Arabidopsis) (COPS2), programmed cell death 5 (PDCD5), cyclin-dependent kinase 2 (CDK2), proteasome (prosome, macropain) 26S subunit, non-ATPase, 3 (PSMD3), RAN binding protein 2 (RANBP2), SERPINE1 mRNA binding protein 1 (SERBP1), O-linked N-acetylglucosamine (GlcNAc) transferase (UDP-N-acetylglucosamine:polypeptide-N-acetylglucosaminyl transferase) (OGT), non-SMC condensin I complex, subunit D2 (NCAPD2), SWI/SNF related, matrix associated, actin dependent regulator of chromatin, subfamily c, member 2 (SMARCC2), NOP10 ribonucleoprotein homolog (yeast) (NOP10), LPS-responsive vesicle trafficking, beach and anchor containing (LRBA), apoptosis inhibitor 5 (API5), signal recognition particle receptor (docking protein) (SRPR), transferrin receptor (p90, CD71) (TFRC), basic leucine zipper and W2 domains 2 (BZW2), ribonuclease, RNase A family, 3 (RNASE3), diazepam binding inhibitor (GABA receptor modulator, acyl-CoA binding protein) (DBI), FK506 binding protein 4, 59kDa (FKBP4), chromosome 6 open reading frame 130 (C6orf130), cofactor of BRCA1 (COBRA1), flap structure-specific endonuclease 1 (FEN1), glucan (1,4-alpha-), branching enzyme 1 (GBE1), small nuclear ribonucleoprotein polypeptide B (SNRPB2), NSFL1 (p97) cofactor (p47) (NSFL1C), acyl-CoA thioesterase 7 (ACOT7), NOP2/Sun domain family, member 2 (NSUN2), chaperonin containing TCP1, subunit 4 (delta) (CCT4), kallikrein-related peptidase 6 (KLK6), glutaminyl-peptide cyclotransferase (QPCT), BCL2-associated athanogene 6 (BAG6), eukaryotic translation initiation factor 3, subunit C (EIF3C/EIF3CL), ATPase, H+ transporting, lysosomal 56/58kDa, V1 subunit B2 (ATP6V1B2), matrix metallopeptidase 8 (neutrophil collagenase) (MMP8), proteasome (prosome, macropain) 26S subunit, ATPase, 5 (PSMC5), GTP cyclohydrolase I feedback regulator (GCHFR), poly(A) polymerase alpha (PAPOLA), hippocalcin-like 1 (HPCAL1), GTPase activating protein (SH3 domain) binding protein 1 (G3BP1), polymerase (RNA) III (DNA directed) polypeptide A, 155kDa (POLR3A), superkiller viralicidic activity 2-like 2 (S. cerevisiae) (SKIV2L2), polymerase (RNA) II (DNA directed) polypeptide A, 220kDa (POLR2A), collagen, type I, alpha 2 (COL1A2), fibrillarin (FBL), glutamyl-prolyl-tRNA synthetase (EPRS), ELAV (embryonic lethal, abnormal vision, Drosophila)-like 1 (Hu antigen R) (ELAVL1), nuclear cap binding protein subunit 2, 20kDa (NCBP2), GCN1 general control of amino-acid synthesis 1-like 1 (yeast) (GCN1L1), histone acetyltransferase 1 (HAT1), stromal antigen 2 (STAG2), sorbitol dehydrogenase (SORD), REX2, RNA exonuclease 2 homolog (S. cerevisiae) (REXO2), heterogeneous nuclear ribonucleoprotein F (HNRNPF), thymopoietin (TMPO), ubiquitin specific peptidase 24 (USP24), KIAA1967 (KIAA1967), complement component 1, r subcomponent (C1R), annexin A7 (ANXA7), RuvB-like 2 (E. coli) (RUVBL2), acireductone dioxygenase 1 (ADI1), eukaryotic translation initiation factor 4A3 (EIF4A3), heterogeneous nuclear ribonucleoprotein U-like 2 (HNRNPUL2), ubiquitin protein ligase E3 component n-recognin 4 (UBR4), SWI/SNF related, matrix associated, actin dependent regulator of chromatin, subfamily a, member 2 (SMARCA2), cytochrome b5 reductase 2 (CYB5R2), splicing factor 3b, subunit 3, 130kDa (SF3B3), G protein pathway suppressor 1 (GPS1), MAD2 mitotic arrest deficient-like 1 (yeast) (MAD2L1), phospholipase C, gamma 1 (PLCG1), eukaryotic translation initiation factor 4H (EIF4H), U2 small nuclear RNA auxiliary factor 2 (U2AF2), tankyrase 1 binding protein 1, 182kDa (TNKS1BP1), transglutaminase 2 (C polypeptide, protein-glutamine-gamma-glutamyltransferase) (TGM2), heterogeneous nuclear ribonucleoprotein L (HNRNPL), inositol polyphosphate-5-phosphatase, 145kDa (INPP5D), annexin A10 (ANXA10), BUD31 homolog (S. cerevisiae) (BUD31),
phosphatidylinositol transfer protein, alpha (PITPNA), leucyl-tRNA synthetase (LARS), nicotinamide N-methyltransferase (NNMT), proteasome (prosome, macropain) 26S subunit, non-ATPase, 12 (PSMD12), v-crk sarcoma virus CT10 oncogene homolog (avian) (CRK), proteoglycan 2, bone marrow (natural killer cell activator, eosinophil granule major basic protein) (PRG2), versican (VCAN),
exportin, tRNA (nuclear export receptor for tRNAs) (XPOT), EMG1 nucleolar protein homolog (S. cerevisiae) (EMG1), chromosome 11 open reading frame 73 (C11orf73), transportin 1 (TNPO1), latent transforming growth factor beta binding protein 2 (LTBP2), cold shock domain containing E1, RNA-binding (CSDE1),
sulfiredoxin 1 (SRXN1), paraspeckle component 1 (PSPC1), ribosomal protein S3A (RPS3A), ISG15 ubiquitin-like modifier (ISG15), polymerase (RNA) II (DNA directed) polypeptide B, 140kDa (POLR2B), general transcription factor IIi (GTF2I), NHP2 non-histone chromosome protein 2-like 1 (S. cerevisiae) (NHP2L1), proteasome (prosome, macropain) 26S subunit, non-ATPase, 10 (PSMD10), signal transducer and activator of transcription 1, 91kDa (STAT1), elongation factor Tu GTP binding domain containing 1 (EFTUD1), mediator complex subunit 23 (MED23), eukaryotic translation initiation factor 2C, 2 (EIF2C2), RNA binding motif protein 4B (RBM4B), KIAA0664 (KIAA0664), core-binding factor, beta subunit (CBFB),
poly(A) binding protein, cytoplasmic 1 (PABPC1), nicotinamide phosphoribosyltransferase (NAMPT), cellular retinoic acid binding protein 2 (CRABP2), thyroid hormone receptor interactor 12 (TRIP12), DnaJ (Hsp40) homolog, subfamily C, member 9 (DNAJC9), StAR-related lipid transfer (START) domain containing 10 (STARD10), ring finger protein 213 (RNF213), eukaryotic translation initiation factor 2B, subunit 5 epsilon, 82kDa (EIF2B5), bolA homolog 2 (E. coli) (BOLA2/BOLA2B), meningioma expressed antigen 5 (hyaluronidase) (MGEA5), Rab geranylgeranyltransferase, alpha subunit (RABGGTA), pyridoxal-dependent decarboxylase domain containing 1 (PDXDC1), exosome component 8 (EXOSC8), phosphoserine aminotransferase 1 (PSAT1), eukaryotic translation initiation factor 6 (EIF6), chromosome 16 open reading frame 13 (C16orf13), signal transducer and activator of transcription 3 (acute-phase response factor) (STAT3), EGF containing fibulin-like extracellular matrix protein 1 (EFEMP1), defensin, alpha 6, Paneth cell-specific (DEFA6), pyrophosphatase (inorganic) 1 (PPA1), glutathione peroxidase 2 (gastrointestinal) (GPX2), unc-13 homolog D (C. elegans) (UNC13D), protein tyrosine phosphatase, non-receptor type 6 (PTPN6), myosin XVIIIA (MYO18A), fibulin 1 (FBLN1), ribosomal protein L19 (RPL19), diaphanous homolog 1 (Drosophila) (DIAPH1), MMS19 nucleotide excision repair homolog (S. cerevisiae) (MMS19), nudix (nucleoside diphosphate linked moiety X)-type motif 21 (NUDT21), splicing factor 3b, subunit 5, 10kDa (SF3B5), SAP domain containing ribonucleoprotein (SARNP), ADP-ribosylation factor guanine nucleotide-exchange factor 2 (brefeldin A-inhibited) (ARFGEF2), guanylate binding protein 1, interferon-inducible (GBP1), HECT, UBA and WWE domain containing 1 (HUWE1), processing of precursor 7, ribonuclease P/MRP subunit (S. cerevisiae) (POP7), eukaryotic translation initiation factor 2B, subunit 3 gamma, 58kDa (EIF2B3), N(alpha)-acetyltransferase 10, NatA catalytic subunit (NAA10), DnaJ (Hsp40) homolog, subfamily B, member 1 (DNAJB1), eukaryotic translation initiation factor 2-alpha kinase 2 (EIF2AK2), elongation factor Tu GTP binding domain containing 2 (EFTUD2), grancalcin, EF-hand calcium binding protein (GCA), neuronal cell adhesion molecule (NRCAM), DEAH (Asp-Glu-Ala-His) box polypeptide 16 (DHX16), small glutamine-rich tetratricopeptide repeat (TPR)-containing, alpha (SGTA), serine/threonine kinase 10 (STK10), smu-1 suppressor of mec-8 and unc-52 homolog (C. elegans) (SMU1), PRP4 pre-mRNA processing factor 4 homolog (yeast) (PRPF4), heterogeneous nuclear ribonucleoprotein D (AU-rich element RNA binding protein 1, 37kDa) (HNRNPD), CTP synthase (CTPS), cleavage stimulation factor, 3' pre-RNA, subunit 3, 77kDa (CSTF3), cleavage stimulation factor, 3' pre-RNA, subunit 1, 50kDa (CSTF1), heterogeneous nuclear ribonucleoprotein U-like 1 (HNRNPUL1), ribosomal protein S5 (RPS5), protein tyrosine phosphatase, non-receptor type 11 (PTPN11), ladinin 1 (LAD1), component of oligomeric golgi complex 2 (COG2), cullin 2 (CUL2), ribosomal protein S17 (RPS17/RPS17L), proteasome (prosome, macropain) 26S subunit, non-ATPase, 4 (PSMD4), annexin A1 (ANXA1), annexin A2 (ANXA2), 5-aminoimidazole-4-carboxamide ribonucleotide formyltransferase/IMP cyclohydrolase (ATIC), azurocidin 1 (AZU1), baculoviral IAP repeat containing 6 (BIRC6), chitinase 3-like 1 (cartilage glycoprotein-39) (CHI3L1), carbamoyl-phosphate synthase 1, mitochondrial (CPS1), cathepsin G (CTSG), defensin, alpha 1 (DEFA1 (includes others)), deleted in malignant brain tumors 1 (DMBT1), elastase, neutrophil expressed (ELANE), integrin, alpha M (complement component 3 receptor 3 subunit) (ITGAM), lipocalin 2 (LCN2), lectin, galactoside-binding, soluble, 3 binding protein (LGALS3BP), minichromosome maintenance complex component 2 (MCM2), matrix metallopeptidase 9 (gelatinase B, 92kDa gelatinase, 92kDa type IV collagenase) (MMP9), myeloperoxidase (MPO), mucin 5AC, oligomeric mucus/gel-forming (MUC5AC/MUC5B), nuclear cap binding protein subunit 1, 80kDa (NCBP1), neurofibromin 1 (NF1), olfactomedin 4 (OLFM4), PDS5, regulator of cohesion maintenance, homolog A (S. cerevisiae) (PDS5A), peptidoglycan recognition protein 1 (PGLYRP1), proteinase 3 (PRTN3), quiescin Q6 sulfhydryl oxidase 1 (QSOX1), regenerating islet-derived 1 alpha (REG1A), S100 calcium binding protein A9 (S100A9), serpin peptidase inhibitor, clade B (ovalbumin), member 10 (SERPINB10), serpin peptidase inhibitor, clade B (ovalbumin), member 5 (SERPINB5), unc-45 homolog A (C. elegans) (UNC45A) .

In one embodiment, the methods of the disclosure screen for one or more biomarkers, the presence of which in a sample is indicative that the individual is at risk of suffering from or is suffering from colorectal cancer.

In an alternative embodiment, the methods of the disclosure screen for one or more biomarkers, the increased expression of which in a sample relative to a control sample is indicative that the individual is at risk of suffering from or is suffering from colorectal cancer.

In a preferred embodiment, the methods of the disclosure screen for one or more biomarkers from the group defined in Table 2. The biomarkers provided in Table 2 represent a preferred subset of those defined in Table 1, the differential expression for which relative to control samples is considered statistically significant. Therefore, this group of biomarkers represents a panel of biomarkers from which any number of biomarkers may be selected for screening.

Thus, in one embodiment, the methods of the disclosure may screen for more than one biomarker from the group defined in Table 2, for example two, three, four, five, six, seven, eight, nine, ten of the biomarkers of the group defined in Table 2. In an alternative embodiment, the methods of the disclsure screen for more than ten of the biomarkers of the group defined in Table 2, for example, eleven, twelve, thirteen, fourteen, fifteen, sixteen, seventeen, eighteen, nineteen, twenty, thirty, forty, fifty, sixty, seventy, eighty, ninety, one hundred of the biomarkers of the group defined in Table 2. Thus, the methods of the disclosure screen for the presence of or increased expression of one or more of: complement component C4B (Chido blood group) 2 (C4A/C4B); glutamic-oxaloacetic transaminase 2, mitochondrial (aspartate aminotransferase 2) (GOT2); glucose-6-phosphate isomerase (GPI); transketolase (TKT); N-acylaminoacyl-peptide hydrolase (APEH); histone cluster 1, H4c (HIST4H4 (includes others)); Fatty acid-binding protein 5 (psoriasis-associated) (FABP5); hexosaminidase B (beta polypeptide) (HEXB); epithelial cell adhesion molecule (EPCAM); Nucleoside diphosphate kinase (NME1-NME2); Superoxide dismutase 2, mitochondrial (SOD2); Tu translation elongation factor, mitochondrial (TUFM); Glutathione synthetase (GSS); annexin A2 (ANXA2); ATP synthase, H+ transporting, mitochondrial F1 complex, beta polypeptide (ATP5B); 10 kDa heat shock protein (chaperonin 10) (HSPE1); glyoxalase I (GLO1); histone cluster 2, H2be (HIST2H2BE (includes others)); S100 calcium binding protein A4 (S100A4); S100 calcium binding protein A11 (S100A11); latexin (LXN); dehydrogenase/reductase (SDR family) member 11 (DHRS11); N-acetylglucosaminidase, alpha (NAGLU); Translin (TSN); Proteasome (prosome, macropain) subunit alpha type-4 (PSMA4); Proteasome (prosome, macropain) subunit alpha type-6 (PSMA6); ras-related C3 botulinum toxin substrate 1 (rho family, small GTP binding protein Rac1) (RAC1); Adenosylhomocysteinase (AHCY); fucosidase, alpha-L- 1, tissue (FUCA1); S100 calcium binding protein P (S100P); Proteasome (prosome, macropain) subunit beta type-2 (PSMB2); X-prolyl aminopeptidase (aminopeptidase P) 1 (XPNPEP1); Keratin 18 (KRT18); Nuclear cap-binding protein subunit 1 80 kDa (NCBP1); mannosidase, alpha, class 2B, member 1 (MAN2B1); S100 calcium binding protein A6 (S100A6); valosin containing protein (VCP); quinolinate phosphoribosyltransferase (QPRT); major histocompatibility complex, class I, B (HLA-B); phosphoglycerate mutase 1 (brain) (PGAM1); ectonucleotide pyrophosphatase/phosphodiesterase 3 (ENPP3); serpin peptidase inhibitor, clade B (ovalbumin), member 10 (SERPINB10); myeloperoxidase (MPO); creatine kinase, mitochondrial 1B (CKMT1A/CKMT1B); proteinase 3 (PRTN3); elastase, neutrophil expressed (ELANE); MORC family CW-type zinc finger 1 (MORC1); ubiquitin B (UBB); phospholipase A2, group IIA (platelets, synovial fluid) (PLA2G2A); carbonic anhydrase IV (CA4); G elongation factor, mitochondrial 2 (GFM2); S100 calcium binding protein A7 (S100A7); Bactericidal permeability-increasing protein (BPI); collagen, type VI, alpha 5 (COL6A5); LIM homeobox 8 (LHX8); cysteine-rich secretory protein 3 (CRISP3); Azurocidin (AZU1); hemicentin 1 (HMCN1); Transglutaminase 3 (E polypeptide, protein-glutamine gamma-glutamyltransferase) (TGM3); CDC42 binding protein kinase alpha (DMPK-like) (CDC42BPA); Cathepsin G (CTSG); Resistin (RETN); methylmalonyl CoA mutase (MUT); armadillo repeat containing, X-linked 4 (ARMCX4); Integrin alpha-M (ITGAM); Calcium channel, voltage dependent, R-type alpha-1E subunit (CACNA1E); T-cell lymphoma invasion and metastasis 2 (TIAM2); HIR histone cell cycle regulation defective homolog A (S. cerevisiae) (HIRA); dopey family member 2 (DOPEY2); integrin beta 1 binding protein 3 (ITGB1BP3); Sodium channel, voltage-gated, type VII, alpha (SCN7A); Rab3C, member RAS oncogene family (RAB3C); chromosome 9 open reading frame 79 (C9orf79); nuclear factor of activated T-cells, calcineurin-dependent 4 (NFATC4); UDP-glucose glycoprotein glucosyltransferase 2 (UGGT2); Cornulin (CRNN); kielin/chordin-like protein (KCP); CD1E molecule (CD1E); coiled-coil domain-containing 18 (CCDC18); leukotriene A-4 hydrolase (LTA4H); albumin (ALB); alpha-2-macroglobulin (A2M); complement component 3 (C3); hemoglobin, beta (HBB); transferrin (TF); hemoglobin, alpha 1 (HBA1/HBA2); lactotransferrin (LTF); ceruloplasmin (ferroxidase) (CP); catalase (CAT); group-specific component (vitamin D-binding protein) (GC); serpin peptidase inhibitor, clade C (antithrombin), member 1 (SERPINC1); fibrinogen gamma chain (FGG); S100 calcium binding protein A8 (S100A8); ferritin, light polypeptide (FTL); actin, beta (ACTB); fibronectin 1 (FN1); defensin, alpha 1 (DEFA1 (includes others)); serpin peptidase inhibitor, clade G (C1 inhibitor), member 1 (SERPING1); retinol binding protein 4, plasma (RBP4); peroxiredoxin 2 (PRDX2); fibrinogen alpha chain (FGA); serpin peptidase inhibitor, clade F (alpha-2 antiplasmin, pigment epithelium derived factor), member 2 (SERPINF2); carbonic anhydrase II (CA2); orosomucoid 1 (ORM1/ORM2); lactate dehydrogenase A (LDHA); vitronectin (VTN); kininogen-1 (KNG1); actin, alpha, cardiac muscle 1 (ACTC1); leucine-rich alpha-2-glycoprotein 1 (LRG1); gamma-glutamyl hydrolase (conjugase, folylpolygammaglutamyl hydrolase) (GGH); enolase 1, (alpha) (ENO1); profilin 1 (PFN1); serpin peptidase inhibitor, clade A (alpha-1 antiproteinase, antitrypsin), member 7 (SERPINA7); alpha-1-microglobulin/bikunin precursor (AMBP); lamin A/C (LMNA); apolipoprotein D (APOD); thyroid hormone receptor interactor 11 (TRIP11); complement component 4 binding protein, alpha (C4BPA); tropomyosin 4 (TPM4); filamin A, alpha (FLNA); haptoglobin (HP); hemopexin (HPX); hemoglobin, delta (HBD); fibrinogen beta chain (FGB); S100 calcium binding protein A9 (S100A9); complement component 5 (C5); solute carrier family 26, member 3 (SLC26A3); complement component 9 (C9); amyloid P component, serum (APCS); alpha-1-B glycoprotein (A1BG); complement C3-like (LOC100133511); inter-alpha (globulin) inhibitor H4 (plasma Kallikrein-sensitive glycoprotein) (ITIH4); complement component C8, alpha polypeptide (C8A); inter-alpha (globulin) inhibitor H1 (ITIH1).

In one embodiment, the method of the disclosure screens for one or more biomarkers capable of diagnosing or predicting CRC in an individual who tested negative in the fecal immunochemical test.

Thus, the methods of the disclosure may screen for one or more biomarkers selected from the group defined in Table 3. The biomarkers provided in Table 3 represent a preferred subset of those defined in Table 1, which have been found to be present in significantly higher levels in CRC samples which came back negative from the fecal immunochemical test. Thus, the group of Table 3 represents a panel of biomarkers from which any number of biomarkers may be selected for screening.

Thus, in one embodiment, the methods of the disclosure may screen for more than one biomarker from the group defined in Table 3, for example two, three, four, five, six, seven, eight, nine, ten of the biomarkers of the group defined in Table 3. In an alternative embodiment, the methods of the disclosure screen for more than ten of the biomarkers of the group defined in Table 3, for example, eleven, twelve, thirteen, fourteen, fifteen, sixteen, seventeen, eighteen, nineteen, twenty, thirty, forty, fifty, sixty, seventy, eighty, ninety, one hundred of the biomarkers of the group defined in Table 3. Thus, the methods of the disclosure may screen for the presence of or increased expression of one or more of: complement component C4B (Chido blood group) 2 (C4A/C4B); glutamic-oxaloacetic transaminase 2, mitochondrial (aspartate aminotransferase 2) (GOT2); glucose-6-phosphate isomerase (GPI); transketolase (TKT); N-acylaminoacyl-peptide hydrolase (APEH); hexosaminidase B (beta polypeptide) (HEXB); epithelial cell adhesion molecule (EPCAM); NME1-NME2 readthrough (NME1-NME2); Tu translation elongation factor, mitochondrial (TUFM); Glutathione synthetase (GSS); glyoxalase I (GLO1); latexin (LXN); Proteasome (prosome, macropain) subunit alpha type-4 (PSMA4); fucosidase, alpha-L- 1, tissue (FUCA1); Keratin 18 (KRT18); Nuclear cap-binding protein subunit 1 80 kDa (NCBP1); mannosidase, alpha, class 2B, member 1 (MAN2B1); S100 calcium binding protein A6 (S100A6); major histocompatibility complex, class I, B (HLA-B); ectonucleotide pyrophosphatase/phosphodiesterase 3 (ENPP3); serpin peptidase inhibitor, clade B (ovalbumin), member 10 (SERPINB10); myeloperoxidase (MPO); proteinase 3 (PRTN3); phospholipase A2, group IIA (platelets, synovial fluid) (PLA2G2A); carbonic anhydrase IV (CA4); G elongation factor, mitochondrial 2 (GFM2); S100 calcium binding protein A7 (S100A7); Bactericidal permeability-increasing protein (BPI); collagen, type VI, alpha 5 (COL6A5); LIM homeobox 8 (LHX8); Azurocidin (AZU1); hemicentin 1 (HMCN1); methylmalonyl CoA mutase (MUT); armadillo repeat containing, X-linked 4 (ARMCX4); Integrin alpha-M (ITGAM); Calcium channel, voltage dependent, R-type alpha-1E subunit (CACNA1E); T-cell lymphoma invasion and metastasis 2 (TIAM2); HIR histone cell cycle regulation defective homolog A (S. cerevisiae) (HIRA); dopey family member 2 (DOPEY2); Sodium channel, voltage-gated, type VII, alpha (SCN7A); chromosome 9 open reading frame 79 (C9orf79); UDP-glucose glycoprotein glucosyltransferase 2 (UGGT2); Cornulin (CRNN); coiled-coil domain-containing 18 (CCDC18); alpha-2-macroglobulin (A2M); complement component 3 (C3); hemoglobin, beta (HBB); transferrin (TF); hemoglobin, alpha 1 (HBA1/HBA2); lactotransferrin (LTF); ceruloplasmin (ferroxidase) (CP); catalase (CAT); fibrinogen gamma chain (FGG); S100 calcium binding protein A8 (S100A8); ferritin, light polypeptide (FTL); fibronectin 1 (FN1); defensin, alpha 1 (DEFA1 (includes others)); serpin peptidase inhibitor, clade G (C1 inhibitor), member 1 (SERPING1); retinol binding protein 4, plasma (RBP4); peroxiredoxin 2 (PRDX2); serpin peptidase inhibitor, clade F (alpha-2 antiplasmin, pigment epithelium derived factor), member 2 (SERPINF2); lactate dehydrogenase (LDHA); vitronectin (VTN); kininogen-1 (KNG1); leucine-rich alpha-2-glycoprotein 1 (LRG1); gamma-glutamyl hydrolase (conjugase, folylpolygammaglutamyl hydrolase) (GGH); serpin peptidase inhibitor, clade A (alpha-1 antiproteinase, antitrypsin), member 7 (SERPINA7); alpha-1-microglobulin/bikunin precursor (AMBP); thyroid hormone receptor interactor 11 (TRIP11); complement component 4 binding protein, alpha (C4BPA); filamin A, alpha (FLNA); hemopexin (HPX); S100 calcium binding protein A9 (S100A9); complement component 5 (C5); solute carrier family 26, member 3 (SLC26A3); complement component 9 (C9); amyloid P component, serum (APCS); complement C3-like (LOC100133511); serpin peptidase inhibitor, clade A (alpha-1 antiproteinase, antitrypsin), member 3 (SERPINA3); Cathepsin S (CTSS); Cytidine deaminase (CDA); Shugoshin-like 2 (SGOL2); serpin peptidase inhibitor, clade B (ovalbumin), member 3 (SERPINB3); hect domain and RLD 2 (HERC2); triosephosphate isomerase 1 (TPI1); Isoform 1 of collagen, type IV, alpha 3 (Goodpasture antigen) binding protein (COL4A3BP); ribonuclease T2 (RNASET2); Glutathione reductase (GSR); spinster homolog 3 (Drosophila) (SPNS3); cDNA FLJ60317, highly similar to Aminoacylase-1 (ACY1); serpin peptidase inhibitor, clade B (ovalbumin), member 8 (SERPINB8); Branched-chain-amino-acid aminotransferase (BCAT2); sialic acid acetylesterase (SIAE); peptidase D (PEPD); major histocompatibility complex, class II, DR beta 5 (HLA-DRB5); SET domain containing 2 (SETD2); hect (homologous to the E6-AP (UBE3A) carboxyl terminus) domain and RCC1 (CHC1)-like domain (RLD) 1 (HERC1); isocitrate dehydrogenase 1 (NADP+), soluble (IDH1); cathepsin C (CTSC); serpin peptidase inhibitor, clade A (alpha-1 antiproteinase, antitrypsin), member 1 (SERPINA1); spectrin repeat containing, nuclear envelope 1 (SYNE1); Ankyrin repeat domain-containing protein 35 (ANKRD35); NIMA (never in mitosis gene a)- related kinase 10 (NEK10); inter-alpha (globulin) inhibitor H2 (ITIH2); acyl-CoA dehydrogenase, very long chain (ACADVL); Nebulin (NEB); Zymogen granule membrane protein 16 (ZG16); Vinculin (VCL); Isoform 2 of Dedicator of cytokinesis protein 4 (DOCK4); hypothetical protein LOC643677 (C13orf40); Uncharacterized protein KIAA1797 (KIAA1797); baculoviral IAP repeat-containing 6 (BIRC6); Transaldolase 1 (TALDO1); Taste receptor type 2 member 42 (TAS2R42); chitinase 1 (chitotriosidase) (CHIT1); quiescin Q6 sulfhydryl oxidase 1 (QSOX1); bone marrow stromal cell antigen 12 (BST1); Bleomycin hydrolase (BLMH); Lysozyme C (LYZ).

In one embodiment, the method of the disclosure screens for one or more biomarkers having a higher discriminatory power than haemoglobin. In other words, the one or more biomarkers have a higher sensitivity and higher specificity than haemoglobin in detecting the presence of advanced adenoma or adenocarcinoma in a sample.

Thus, in one embodiment, the method of the disclosure screens for one or more biomarker, for example two, three, four, five, six, seven, eight, nine, ten of the biomarkers selected from the group consisting of: S100 calcium binding protein A8 (S100A8), complement component C4B (Chido blood group) 2 (C4A/C4B), transferrin (TF), alpha-2-macroglobulin (A2M), S100 calcium binding protein A9 (S100A9), proteinase 3 (PRTN3), Azurocidin (AZU1), lactotransferrin (LTF), hemopexin (HPX) and defensin, alpha 1 (DEFA1).

Thus, in one embodiment, the method of the disclosure screens for one or more biomarker, for example two, three, four, five, six, seven, eight, nine, ten of the biomarkers selected from the group consisting of: annexin A2 (ANXA2), azurocidin 1 (AZU1), cathepsin G (CTSG), defensin, alpha 1 (DEFA1 includes others)), elastase, neutrophil expressed (ELANE), integrin, alpha M (complement component 3 receptor 3 subunit) (ITGAM), myeloperoxidase (MPO), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), serpin peptidase inhibitor, clade B (ovalbumin), member 5 (SERPINB5).

In one embodiment, the method of the disclosure screens for one or more biomarkers, the presence of which in a sample is indicative that the individual is at risk of suffering from or is suffering from colorectal cancer.

Thus, in one embodiment, the method of the disclosure screens for one or more biomarker, for example two, three, four, five, six, seven, eight, nine, ten of the biomarkers selected from the group defined in Table 4. The biomarkers provided in Table 4 represent a preferred subset of those defined in Table 1, which have been found to be expressed only in CRC samples, not in control samples. Thus, the group of Table 4 represents a panel of biomarkers from which any number of biomarkers may be selected for screening.

In one embodiment, the method of the disclosure screens for one or more biomarkers, the presence of which in a sample is indicative that the individual is at risk of suffering from or is suffering from colorectal cancer.

Thus, in one embodiment, the method of the disclosure screens for one or more biomarker, for example two, three, four, five, six, seven, eight, nine, ten of the biomarkers selected from the group defined in Table 5. The biomarkers provided in Table 5 represent a preferred subset of those defined in Table 1, which were identified in an independent verification set of stool samples.

Thus, in a further embodiment, the method of the disclosure screens for one or more biomarker, for example two, three, four, five, six, seven, eight, nine, ten of the biomarkers selected from the group consisting of: alpha-1-B glycoprotein (A1BG), alpha-2-macroglobulin (A2M), actin, beta (ACTB), actin, alpha, ardiac muscle 1 (ACTC1), albumin (ALB), amyloid P component, serum (APCS), N-cylaminoacyl-peptide hydrolase (APEH), apolipoprotein D (APOD), azurocidin 1 (AZU1), complement component 3 (C3), complement component 4B (Chido blood group) (C4A/C4B), complement component 5 (C5), carbonic anhydrase II (CA2), carbonic anhydrase IV (CA4), catalase (CAT), ceruloplasmin (ferroxidase) (CP), cathepsin G (CTSG), defensin, alpha 1 (DEFA1(includesothers)), elastase, neutrophil expressed (ELANE), enolase 1, (alpha) (ENO1), filamin A, alpha (FLNA), fibronectin 1 (FN1), ferritin, light polypeptide (FTL), fucosidase, alpha-L- 1, tissue (FUCA1), gamma-glutamyl hydrolase (conjugase, folylpolygammaglutamyl hydrolase) (GGH), glutamic-oxaloacetic transaminase 2, mitochondrial (aspartate aminotransferase 2) (GOT2), glucose-6-phosphate isomerase (GPI), glutathione synthetase (GSS), hemoglobin, alpha 1 (HBA1/HBA2), hemoglobin, beta (HBB), hemoglobin, delta (HBD), hexosaminidase B (beta polypeptide) (HEXB), histone cluster 1, H4c (HIST4H4(includesothers)), major histocompatibility complex, class I, B (HLA-B), haptoglobin (HP), hemopexin (HPX), heat shock 10kDa protein 1 (chaperonin 10) (HSPE1), lactate dehydrogenase A (LDHA), complement C3-like (LOC100133511), leucine-rich alpha-2-glycoprotein 1 (LRG1), lactotransferrin (LTF), latexin (LXN), mannosidase, alpha, class 2B, member 1 (MAN2B1), myeloperoxidase (MPO), N-acetylglucosaminidase, alpha (NAGLU), orosomucoid 1 (ORM1/ORM2), profilin 1 (PFN1), phospholipase A2, group IIA (platelets, synovial fluid) (PLA2G2A), proteinase 3 (PRTN3), proteasome (prosome, macropain) subunit, alpha type, 4 (PSMA4), proteasome (prosome, macropain) subunit, alpha type, 6 (PSMA6), proteasome (prosome, macropain) subunit, beta type, 2 (PSMB2), retinol binding protein 4, plasma (RBP4), resistin (RETN), S100 calcium binding protein A8 (S100A8), S100 calcium binding protein A9 (S100A9), serpin peptidase inhibitor, clade B (ovalbumin), member 10 (SERPINB10), serpin peptidase inhibitor, clade C (antithrombin), member 1 (SERPINC1), serpin peptidase inhibitor, clade F (alpha-2 antiplasmin, pigment epithelium derived factor), member 2 (SERPINF2), serpin peptidase inhibitor, clade G (C1 inhibitor), member 1 (SERPING1), superoxide dismutase 2, mitochondrial (SOD2), transferrin (TF), transketolase (TKT), ubiquitin B (UBB), Unmapped by Ingenuity (IPI00884078), Unmapped by Ingenuity (26kDaprotein, IPI00942608), alpha-2-macroglobulin-like 1 (A2ML1), acyl-CoA dehydrogenase, very long chain (ACADVL), Unmapped by Ingenuity (ACY1), alkaline phosphatase, liver/bone/kidney (ALPL), bleomycin hydrolase (BLMH), bone marrow stromal cell antigen 1 (BST1), calcium channel, voltage-dependent, L type, alpha 1D subunit (CACNA1D), creatine kinase, mitochondrial 1B (CKMT1A/CKMT1B), cathepsin S (CTSS), fumarylacetoacetate hydrolase (fumarylacetoacetase) (FAH), guanine deaminase (GDA), glutathione reductase (GSR), heterogeneous nuclear ribonucleoprotein A2/B1 (HNRNPA2B1), heat shock 70kDa protein (HSPA8), keratin 6C (KRT6C), keratin 6C (KRT6C), lipocalin 1 (tear prealbumin) (LCN1), lysozyme (LYZ), minichromosome maintenance complex component 2 (MCM2), peptidase D (PEPD), phospholipase B domain containing 1 (PLBD1), proteasome (prosome, macropain) subunit, alpha type, 2 (PSMA2), proteasome (prosome, macropain) subunit, beta type, 1 (PSMB1), proteasome (prosome, macropain) subunit, beta type, 6 (PSMB6), quiescin Q6 sulfhydryl oxidase 1 (QSOX1), ribonuclease T2 (RNASET2), serpin peptidase inhibitor, clade A (alpha-1 antiproteinase, antitrypsin), member 1 (SERPINA1), serpin peptidase inhibitor, clade A (alpha-1 antiproteinase, antitrypsin), member 3 (SERPINA3), serpin peptidase inhibitor, clade B (ovalbumin), member 3 (SERPINB3), sialic acid acetylesterase (SIAE), triosephosphate isomerase 1 (TPI1), zymogen granule protein 16 homolog (rat) (ZG16).

In one embodiment, the method of the disclosure screens for one or more biomarkers, the presence of which in a sample is indicative that the individual is at risk of suffering from or is suffering from colorectal cancer, which biomarkers have been selecte on the basis of correlation with secretomes of colon tumor tissue.

Thus, in one embodiment, the method of the disclosure screens for one or more biomarker, for example two, three, four, five, six, seven, eight, nine, ten of the biomarkers selected from the group defined in Table 7. The biomarkers provided in Table 7 represent a preferred subset of those defined in Table 6, which have been found to be expressed both in tumor issue and stool samples. Thus, the group of Table 7 represents a panel of biomarkers from which any number of biomarkers may be selected for screening. Thus, in a further embodiment, the method of the disclosure screens for one or more biomarker, for example two, three, four, five, six, seven, eight, nine, ten of the biomarkers selected from the group consisting of: annexin A2 (ANXA2), azurocidin 1 (AZU1), cathepsin G (CTSG), defensin, alpha 1 (DEFA1 (includes others)), elastase, neutrophil expressed (ELANE), integrin, alpha M (complement component 3 receptor 3 subunit) (ITGAM), myeloperoxidase (MPO), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), serpin peptidase inhibitor, clade B (ovalbumin), member 5 (SERPINB5).

Preferably, the method of the disclosure has an accuracy of at least 75%, for example 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% accuracy. Preferably, the method of the invention has a sensitivity of at least 75%, for example 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity.

Preferably, the method of the invention has a specificity of at least 75%, for example 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% specificity.

By "accuracy" it is meant the proportion of correct outcomes of a diagnosis, by "sensitivity" it is meant the proportion of all positive diagnoses that are correctly classified as positives, and by "specificity" it is meant the proportion of all negative diagnoses that are correctly classified as negatives. The methods of the invention may be verified by subsequent colonoscopy.

### Screening methods

In one embodiment, the biological sample, for example a stool sample, may be screened for the one or more biomarkers using (targeted) mass spectrometry.

Protein marker discovery and screening by LC-MS/MS and hypothesis-based protein marker detection by SRM-MS has been applied before on murine and human stool samples, respectively (Ang CS, Nice EC. J Proteome Res 2010; 9: 4346-55; Ang CS, et al. Electrophoresis 2011; 32: 1926-38; Ang CS, et al. J Chromatogr A 2010; 1217: 3330-40).

Despite the complexity of stool material, the present disclosure has successfully detected several of these previously reported human stool proteins. In addition, the number of included samples and corresponding identified proteins in the current study exceeds that of previous studies. Therefore the data presented here is the largest overview of the human stool proteome to date.

While sample screening by the mass spectrometric techniques described herein has been proven to be effective, a preferred screening method comprises an antibody based screening assay. The fecal immunochemical test (FIT) comprises an antibody based screening assay and so an antibody based screening assay for one or more of the biomarkers identified in the present application provides a complementary screen which can be readily incorporated into the existing FIT assay.

Thus, in one embodiment of the methods of the disclosure, the biological sample is screened for the one or more biomarkers using a binding agent capable of binding to the one or more biomarkers.

Binding agents (also referred to as binding molecules) can be selected from a library, based on their ability to bind a given motif, as discussed below.

In one embodiment, the first binding agent is an antibody or a fragment thereof. Thus, a fragment may contain one or more of the variable heavy (V_{H}) or variable light (V_{L}) domains. For example, the term antibody fragment includes Fab-like molecules (Better et al Science 1988; 240, 1041); Fv molecules (Skerra et al Science 1988; 240, 1038); single-chain Fv (ScFv) molecules where the V_{H} and V_{L} partner domains are linked via a flexible oligopeptide (Bird et al Science 1988; 242,423; Huston et al Proc. Natl. Acad. Sci. USA 1988; 85, 5879) and single domain antibodies (dAbs) comprising isolated V domains (Ward et al Nature 1989; 341,544).

The term "antibody variant" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecule capable of binding to an antigen in an immunoassay format that is known to those skilled in the art.

A general review of the techniques involved in the synthesis of antibody fragments which retain their specific binding sites is to be found in Winter & Milstein Nature 1991; 349, 293-299.

In one embodiment, the antibody or fragment thereof is a recombinant antibody or fragment thereof (such as an scFv). By "ScFv molecules" it is meant molecules wherein the V_{H} and V_{L} partner domains are linked via a flexible oligopeptide.

The advantages of using antibody fragments, rather than whole antibodies, are several-fold. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')₂ fragments are "bivalent". By "bivalent" it is meant that the said antibodies and F(ab')₂ fragments have two antigen combining sites. In contrast, Fab, Fv, ScFv and dAb fragments are monovalent, having only one antigen combining site.

The antibodies may be monoclonal or polyclonal. Suitable antibodies may be prepared by known techniques and need no further discussion.

Additionally or alternatively the binding agent may be an aptamer. Suitable aptamers may be prepared by known techniques and need no further discussion.

In one embodiment, the one or more biomarker(s) in the test sample is labelled with a detectable moiety. In one embodiment, the one or more biomarker(s) in the control sample is labelled with a detectable moiety (which may be the same or different from the detectable moiety used to label the test sample).

A "detectable moiety" is one which may be detected and the relative amount and/or location of the moiety (for example, the location on an array) determined.

Detectable moieties are well known in the art. A detectable moiety may be a fluorescent and/or luminescent and/or chemiluminescent moiety which, when exposed to specific conditions, may be detected. For example, a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

Alternatively, the detectable moiety may be a radioactive label, which may be incorporated by methods well known in the art.

### Arrays

In one embodiment, the methods of the present invention may be carried out on an array.

Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution.

The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, antibodies and other suitable molecules and/or conducting an immunoassay.

The binding processes are well known in the art and generally consist of cross-linking covalently binding or physically adsorbing a protein molecule, antibody or the like to the solid support. By using well-known techniques, such as contact or non-contact printing, masking or photolithography, the location of each spot can be defined.

Once suitable binding molecules (discussed above) have been identified and isolated, the skilled person can manufacture an array using methods well known in the art of molecular biology.

In one embodiment, the screening may comprise using an assay comprising a second binding agent capable of binding to the one or more biomarkers, the second binding agent having a detectable moiety.

In one embodiment, the second binding agent is an antibody or a fragment thereof (for example, as described above in relation to the first binding agent).

Typically, the assay is an ELISA (Enzyme Linked Immunosorbent Assay) which usually involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemiluminescent systems based on enzymes such as luciferase can also be used.

Conjugation with the vitamin biotin is also employed used since this can readily be detected by its reaction with enzyme-linked avidin or streptavidin to which it binds with great specificity and affinity.

It will be appreciated by persons skilled in the art that there is a degree of fluidity in the biomarker composition of the signatures of the invention. Thus, different combinations of the biomarkers may be equally useful in the diagnosis, prognosis and/or characterisation of colorectal cancer. In this way, each biomarker (either alone or in combination with one or more other biomarkers) makes a contribution to the signature.

### Compounds and methods for treating CRC

The identification of the biomarkers as defined in Table 1 and/or Table 6 allows not only the detection of advanced colonic adenomas and colonic adenocarcinomas (colorectal cancer), but enables also methods of treating colorectal cancers and also provides for compounds for use in methods of treating colorectal cancers.

While early diagnosis of colorectal cancer often allows for curative surgical removal of the tumour, later diagnosis may result in a (chemo)therapeutic treatment instead. Therapeutic agents used to treat colorectal cancer include monoclonal antibodies, small molecule inhibitors and chemotherapeutic agents.

Typical therapeutic monoclonal antibodies include but are not limited to bevacizumab, cetuximab or panitumumab. Typical small molecule inhibitors include but are not limited to erlotinib, sorafenib or alisertib. Typical chemotherapeutic agents include but are not limited to 5-FU, capecitabine, irinotecan oxaliplatin, or leucovorin or any combination thereof. Combination therapies of, for example, a therapeutic monoclonal antibody and a small molecule inhibitor may be used. Thus, any combination of two or more of a monoclonal antibody, a small molecule inhibitor and a chemotherapeutic agent is envisaged.

### Kit for performing the method

The kit for performing the method according to the invention may be selected from any suitable assay and data processing apparatus and equipments.

The suitable selection will be well within the ability of those skilled in the art, and further description is not necessary here.

### "Comprising"

The term "comprising" and related terms herein is to be interpreted as embracing "consisting essentially of" and "consisting of", these two expressions being interchangeable with "comprising" in all definitions and discussion in this patent in order to specify alternative extents of exclusion of unspecified elements additional to the recited elements.

The term "comprising" and related expressions means "including" and therefore leaves open the option of including unspecified elements, whether essential or not. The term "consisting essentially of" and related expressions permits the presence of elements that do not materially affect the basic and novel or functional characteristic(s) of that embodiment of the invention. The term "consisting of" and related expressions means "consisting only of" and therefore excludes any element not recited in that description of the embodiment.

### Brief Description of the Figures

The invention shall now be further described by the following example with reference to the attached figures. The example is provided by way of example only, without any intended limitation of the scope of the invention.
Figure 1A shows the number of human proteins detected in stool samples from 12 CRC patients and from 10 control patients. The Venn diagram shows the number of proteins detected in CRC or control stool samples and their overlap;
Figure 1B shows the subcellular location of the identified human proteins. The pie chart shows the percentage of different subcellular locations of the 830 human proteins that were identified in 22 stool samples. The ratios were similar for CRC patients and control patients;
Figure 2A shows a scatterplot of protein quantifications of hemoglobin alpha as measured by LC-MS/MS compared to hemoglobin quantification by FIT of individual stool samples;
Figure 2B shows a scatterplot of protein quantifications of hemoglobin beta as measured by LC-MS/MS compared to hemoglobin quantification by FIT of individual stool samples;
Figure 2C shows a scatterplot of protein quantifications of hemoglobin alpha and beta as measured by LC-MS/MS of individual stool samples;
Figure 2D shows a scatterplot of protein quantifications of S100A8 and S100A9 as measured by LC-MS/MS of individual stool samples. Filled circles represent stool samples from subjects with CRC (n=12), crosses represent stool samples from subjects without colon neoplasia (n=10); and
Figure 3 shows a scatterplot of protein quantifications of Complement component 4B measured by LC-MS/MS in relation to FIT values of individual stool samples. The dotted line shows the cut-off of 75 ng/ml for FIT. A FIT value less than 75 ng/ml is regarded as a negative test result (Van Veen, NTG, 2009). Filled circles represent stool samples from subjects with CRC (n=12), crosses represent stool samples from subjects without colon neoplasia (n=10).

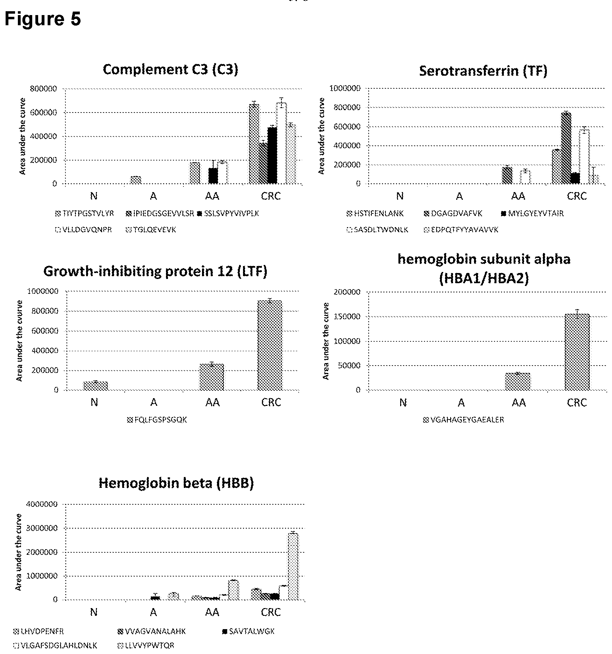

Figures 4 and 5 show bar graphs with the mean of the areas under the curves of all transitions of each peptide in triplicate analyses. One to five different peptides per protein are depicted. Peptides were measured in triplicate in pools from stool samples from controls (N, n=5), non-advanced adenoma patients (A, n=5), advanced adenoma patients (AA, n=5) and CRC patients (CRC, n=5). Error bars represent standard deviations.

### TABLES

The following tables list the biomarkers according to the disclosure, and may continue over several pages.

**Table 1: Biomarkers indicative of colorectal cancer.**

| Accession Number | Gene Symbol | Entrez Gene Name |
|---|---|---|
| IPI00892604 | C4A/C4B | complement component 4B (Chido blood group) |
| IPI00018206 | GOT2 | glutamic-oxaloacetic transaminase 2, mitochondrial (aspartate aminotransferase 2) |
| IPI00027497 | GPI | glucose-6-phosphate isomerase |
| IPI00643920 | TKT | transketolase |
| IPI00337741 | APEH | N-acylaminoacyl-peptide hydrolase |
| IPI00453473 | HIST4H4 (includes others) | histone cluster 1, H4c |
| IPI00007797 | FABP5 | fatty acid binding protein 5 (psoriasis-associated) |
| IPI00012585 | HEXB | hexosaminidase B (beta polypeptide) |
| IPI00296215 | EPCAM | epithelial cell adhesion molecule |
| IPI00604590 | NME1-NME2 | NME1-NME2 readthrough |
| IPI00022314 | SOD2 | superoxide dismutase 2, mitochondrial |
| IPI00027107 | TUFM | Tu translation elongation factor, mitochondrial |
| IPI00010706 | GSS | glutathione synthetase |
| IPI00418169 | ANXA2 | annexin A2 |
| IPI00303476 | ATP5B | ATP synthase, H+ transporting, mitochondrial F1 complex, beta polypeptide |
| IPI00220362 | HSPE1 | heat shock 10kDa protein 1 (chaperonin 10) |
| IPI00220766 | GLO1 | glyoxalase I |
| IPI00003935 | HIST2H2BE (includes others) | histone cluster 2, H2be |
| IPI00032313 | S100A4 | S100 calcium binding protein A4 |
| IPI00013895 | S100A11 | S100 calcium binding protein A11 |
| IPI00106687 | LXN | latexin |
| IPI00034280 | DHRS11 | dehydrogenase/reductase (SDR family) member 11 |
| IPI00008787 | NAGLU | N-acetylglucosaminidase, alpha |
| IPI00018768 | TSN | translin |
| IPI00299155 | PSMA4 | proteasome (prosome, macropain) subunit, alpha type, 4 |
| IPI00029623 | PSMA6 | proteasome (prosome, macropain) subunit, alpha type, 6 |
| IPI00010271 | RAC1 | ras-related C3 botulinum toxin substrate 1 (rho family, small GTP binding protein Rac1) |
| IPI00012007 | AHCY | adenosylhomocysteinase |
| IPI00385751 | FUCA1 | fucosidase, alpha-L- 1, tissue |
| IPI00017526 | S100P | S100 calcium binding protein P |
| IPI00028006 | PSMB2 | proteasome (prosome, macropain) subunit, beta type, 2 |
| IPI00793375 | XPNPEP1 | X-prolyl aminopeptidase (aminopeptidase P) 1, soluble |
| IPI00554788 | KRT18 | keratin 18 |
| IPI00019380 | NCBP1 | nuclear cap binding protein subunit 1, 80kDa |
| IPI00012989 | MAN2B1 | mannosidase, alpha, class 2B, member 1 |
| IPI00027463 | S100A6 | S100 calcium binding protein A6 |
| IPI00022774 | VCP | valosin-containing protein |
| IPI00300086 | QPRT | quinolinate phosphoribosyltransferase |
| IPI00472073 | HLA-B | major histocompatibility complex, class I, B |
| IPI00453476 | PGAM1 | phosphoglycerate mutase 1 (brain) |
| IPI00020999 | ENPP3 | ectonucleotide pyrophosphatase/phosphodiesterase 3 |
| IPI00010304 | SERPINB10 | serpin peptidase inhibitor, clade B (ovalbumin), member 10 |
| IPI00007244 | MPO | myeloperoxidase |
| IPI00877726 | CKMT1A/CKMT1B | creatine kinase, mitochondrial 1B |
| IPI00027409 | PRTN3 | proteinase 3 |
| IPI00027769 | ELANE | elastase, neutrophil expressed |
| IPI00004362 | MORC1 | MORC family CW-type zinc finger 1 |
| IPI00719280 | UBB | ubiquitin B |
| IPI00026962 | PLA2G2A | phospholipase A2, group IIA (platelets, synovial fluid) |
| IPI00027466 | CA4 | carbonic anhydrase IV |
| IPI00071703 | GFM2 | G elongation factor, mitochondrial 2 |
| IPI00219806 | S100A7 | S100 calcium binding protein A7 |
| IPI00827847 | BPI | bactericidal/permeability-increasing protein |
| IPI00176125 | COL6A5 | collagen, type VI, alpha 5 |
| IPI00470355 | LHX8 | LIM homeobox 8 |
| IPI00942117 | CRISP3 | cysteine-rich secretory protein 3 |
| IPI00022246 | AZU1 | azurocidin 1 |
| IPI00045512 | HMCN1 | hemicentin 1 |
| IPI00300376 | TGM3 | transglutaminase 3 (E polypeptide, protein-glutamine-gamma-glutamyltransferase) |
| IPI00640468 | CDC42BPA | CDC42 binding protein kinase alpha (DMPK-like) |
| IPI00028064 | CTSG | cathepsin G |
| IPI00006988 | RETN | resistin |
| IPI00024934 | MUT | methylmalonyl CoA mutase |
| IPI00552983 | ARMCX4 | armadillo repeat containing, X-linked 4 |
| IPI00217987 | ITGAM | integrin, alpha M (complement component 3 receptor 3 subunit) |
| IPI00165045 | CACNA1E | calcium channel, voltage-dependent, R type, alpha 1E subunit |
| IPI00018363 | TIAM2 | T-cell lymphoma invasion and metastasis 2 |
| IPI00217560 | HIRA | HIR histone cell cycle regulation defective homolog A (S. cerevisiae) |
| IPI00294653 | DOPEY2 | dopey family member 2 |
| IPI00004401 | ITGB1BP3 | integrin beta 1 binding protein 3 |
| IPI00300117 | SCN7A | sodium channel, voltage-gated, type VII, alpha |
| IPI00061114 | RAB3C | RAB3C, member RAS oncogene family |
| IPI00168442 | C9orf79 | chromosome 9 open reading frame 79 |
| IPI00385215 | NFATC4 | nuclear factor of activated T-cells, cytoplasmic, calcineurin-dependent 4 |
| IPI00024467 | UGGT2 | UDP-glucose glycoprotein glucosyltransferase 2 |
| IPI00297056 | CRNN | cornulin |
| IPI00914663 | KCP | kielin/chordin-like protein |
| IPI00418592 | CD1E | CD1e molecule |
| IPI00642206 | CCDC18 | coiled-coil domain containing 18 |
| IPI00514090 | LTA4H | leukotriene A4 hydrolase |
| IPI00745872 | ALB | albumin |
| IPI00478003 | A2M | alpha-2-macroglobulin |
| IPI00783987 | C3 | complement component 3 |
| IPI00654755 | HBB | hemoglobin, beta |
| IPI00022463 | TF | transferrin |
| IPI00410714 | HBA1/HBA2 | hemoglobin, alpha 1 |
| IPI00298860 | LTF | lactotransferrin |
| IPI00017601 | CP | ceruloplasmin (ferroxidase) |
| IPI00465436 | CAT | catalase |
| IPI00555812 | GC | group-specific component (vitamin D binding protein) |
| IPI00032179 | SERPINC1 | serpin peptidase inhibitor, clade C (antithrombin), member 1 |
| IPI00219713 | FGG | fibrinogen gamma chain |
| IPI00007047 | S100A8 | S100 calcium binding protein A8 |
| IPI00375676 | FTL | ferritin, light polypeptide |
| IPI00021439 | ACTB | actin, beta |
| IPI00022418 | FN1 | fibronectin 1 |
| IPI00005721 | DEFA1 (includes others) | defensin, alpha 1 |
| IPI00291866 | SERPING1 | serpin peptidase inhibitor, clade G (C1 inhibitor), member 1 |
| IPI00022420 | RBP4 | retinol binding protein 4, plasma |
| IPI00027350 | PRDX2 | peroxiredoxin 2 |
| IPI00021885 | FGA | fibrinogen alpha chain |
| IPI00029863 | SERPINF2 | serpin peptidase inhibitor, clade F (alpha-2 antiplasmin, pigment epithelium derived factor), member 2 |
| IPI00218414 | CA2 | carbonic anhydrase II |
| IPI00020091 | ORM1/ORM2 | orosomucoid 1 |
| IPI00217966 | LDHA | lactate dehydrogenase A |
| IPI00298971 | VTN | vitronectin |
| IPI00215894 | KNG1 | kininogen 1 |
| IPI00023006 | ACTC1 | actin, alpha, cardiac muscle 1 |
| IPI00022417 | LRG1 | leucine-rich alpha-2-glycoprotein 1 |
| IPI00023728 | GGH | gamma-glutamyl hydrolase (conjugase, folylpolygammaglutamyl hydrolase) |
| IPI00465248 | ENO1 | enolase 1, (alpha) |
| IPI00216691 | PFN1 | profilin 1 |
| IPI00292946 | SERPINA7 | serpin peptidase inhibitor, clade A (alpha-1 antiproteinase, antitrypsin), member 7 |
| IPI00022426 | AMBP | alpha-1-microglobulin/bikunin precursor |
| IPI00021405 | LMNA | lamin A/C |
| IPI00006662 | APOD | apolipoprotein D |
| IPI00003515 | TRIP11 | thyroid hormone receptor interactor 11 |
| IPI00021727 | C4BPA | complement component 4 binding protein, alpha |
| IP100010779 | TPM4 | tropomyosin 4 |
| IPI00302592 | FLNA | filamin A, alpha |
| IPI00641737 | HP | haptoglobin |
| IPI00022488 | HPX | hemopexin |
| IPI00473011 | HBD | hemoglobin, delta |
| IPI00298497 | FGB | fibrinogen beta chain |
| IPI00027462 | S100A9 | S100 calcium binding protein A9 |
| IPI00032291 | C5 | complement component 5 |
| IPI00031036 | SLC26A3 | solute carrier family 26, member 3 |
| IPI00022395 | C9 | complement component 9 |
| IPI00022391 | APCS | amyloid P component, serum |
| IPI00022895 | A1BG | alpha-1-B glycoprotein |
| IPI00887739 | LOC100133511 | complement C3-like |
| IPI00218192 | ITIH4 | inter-alpha (globulin) inhibitor H4 (plasma Kallikrein-sensitive glycoprotein) |
| IPI00011252 | C8A | complement component 8, alpha polypeptide |
| IPI00292530 | ITIH1 | inter-alpha (globulin) inhibitor H1 |
| IPI00937735 | ACADVL | acyl-CoA dehydrogenase, very long chain |
| IPI00009268 | ACY1 | cDNA FLJ60317, highly similar to Aminoacylase-1 |
| IPI00166331 | ANKRD35 | Ankyrin repeat domain-containing protein 35 |
| IPI00299635 | BIRC6 | baculoviral IAP repeat-containing 6 |
| IPI00219575 | BLMH | Bleomycin hydrolase |
| IPI00026240 | BST1 | bone marrow stromal cell antigen 1 |
| IPI00929313 | C13orf40 | hypothetical protein LOC643677 |
| IPI00027983 | CDA | Cytidine deaminase |
| IPI00852865 | CHIT1 | chitinase 1 (chitotriosidase) |
| IPI00022810 | CTSC | Dipeptidyl-peptidase 1 |
| IPI00299150 | CTSS | Cathepsin S |
| IPI00006024 | DOCK4 | Isoform 2 of Dedicator of cytokinesis protein 4 |
| IPI00016862 | GSR | Glutathione reductase |
| IPI00941901 | HERC1 | hect (homologous to the E6-AP (UBE3A) carboxyl terminus) domain and RCC1 (CHC1)-like domain (RLD) 1 |
| IPI00005826 | HERC2 | hect domain and RLD 2 |
| IPI00746667 | HLA-DRB5 | HLA class II histocompatibility antigen, DRB1-11 beta chain |
| IPI00027223 | IDH1 | Isocitrate dehydrogenase [NADP] cytoplasmic |
| IPI00305461 | ITIH2 | inter-alpha (globulin) inhibitor H2 |
| IPI00748360 | KIAA1797 | Uncharacterized protein KIAA1797 |
| IPI00019038 | LYZ | Lysozyme C |
| IPI00303335 | NEB | Nebulin |
| IPI00065454 | NEK10 | NIMA (never in mitosis gene a)- related kinase 10 (NEK10); |
| IPI00257882 | PEPD | peptidase D |
| IPI00003590 | QSOX1 | quiescin Q6 sulfhydryl oxidase |
| IPI00939604 | RNASET2 | Ribonuclease T2 |
| IPI00553177 | SERPINA1 | serpin peptidase inhibitor, clade A (alpha-1 antiproteinase, antitrypsin), member 1 |
| IPI00550991 | SERPINA3 | serpin peptidase inhibitor, clade A (alpha-1 antiproteinase, antitrypsin), member 3 |
| IPI00022204 | SERPINB3 | serpin peptidase inhibitor, clade B (ovalbumin), member 3 |
| IPI00307733 | SETD2 | SET domain containing 2 |
| IPI00218013 | SGOL2 | Shugoshin-like 2 |
| IPI00010949 | SIAE | sialic acid acetylesterase |
| IPI00386444 | SYNE1 | spectrin repeat containing, nuclear envelope 1 |
| IPI00744692 | TALDO1 | Transaldolase 1 |
| IPI00375371 | TAS2R42 | Taste receptor type 2 member 42 |
| IPI00465028 | TPI1 | triosephosphate isomerase 1 |
| IPI00291175 | VCL | Vinculin |
| IPI00029647 | ZG16 | Zymogen granule membrane protein 16 |
| IPI00016255 | PLBD1 | hypothetical protein LOC79887 |
| IPI00477505 | ANKRD28 | Isoform 1 of Serine/threonine-protein phosphatase 6 regulatory ankyrin repeat subunit A |
| IPI00032293 | CST3 | Cystatin-C |
| IPI00293867 | DDT | D-dopachrome decarboxylase |
| IPI00059242 | SYAP1 | Synapse-associated protein 1 |
| IPI00219622 | PSMA2 | Proteasome subunit alpha type-2 |
| IPI00221222 | SUB1 | SUB1 homolog (S. cerevisiae) |
| IPI00022791 | MFAP3 | Microfibril-associated glycoprotein 3 |
| IPI00011229 | CTSD | Cathepsin D |
| IPI00025019 | PSMB1 | proteasome (prosome, macropain) subunit, beta type, 1 |
| IPI00479306 | PSMB5 | proteasome (prosome, macropain) subunit, beta type, 5 |
| IPI00234793 | KCTD15 | cDNA FLJ61112, highly similar to BTB/POZ domain-containing protein KCTD15 |
| IPI00010796 | P4HB | prolyl 4-hydroxylase, beta polypeptide |
| IPI00927606 | GPX1 | glutathione peroxidase 1 |
| IPI00783625 | SERPINB5 | serpin peptidase inhibitor, clade B (ovalbumin), member 5 |
| IPI00024701 | COL4A3BP | collagen, type IV, alpha 3 (Goodpasture antigen) binding protein |
| IPI00000811 | PSMB6 | proteasome (prosome, macropain) subunit, beta type, 6 |
| IPI00021298 | KRT20 | Keratin 20 |
| IPI00025084 | CAPNS1 | Calpain small subunit 1 |
| IPI00024919 | PRDX3 | peroxiredoxin 3 |
| IPI00059930 | NACC2 | NACC family member 2, BEN and BTB (POZ) domain containing |
| IPI00003817 | ARHGDIB | Rho GDP-dissociation inhibitor 2 |
| IPI00293276 | MIF | Macrophage migration inhibitory factor |
| IPI00514622 | RANBP6 | Ran-binding protein 6 |
| IPI00333141 | SPNS3 | spinster homolog 3 (Drosophila) |
| IPI00922181 | MCM2 | minichromosome maintenance complex component 2 |
| IPI00031708 | FAH | Fumarylacetoacetase |
| IPI00003865 | HSPA8 | heat shock 70kDa protein 8 |
| IPI00299024 | BASP1 | brain abundant, membrane attached signal protein 1 |
| IPI00181135 | BCAT2 | Branched-chain-amino-acid aminotransferase |
| IPI00219365 | MSN | Moesin |
| IPI00032134 | SERPINB8 | serpin peptidase inhibitor, clade B (ovalbumin), member 8 |
| IPI00853547 | G6PD | glucose-6-phosphate dehydrogenase isoform a |
| IPI00478758 | C10orf119 | Isoform 1 of UPF0557 protein C10orf119 |
| IPI00873020 | PSAP | Prosaposin |
| IPI00000875 | EEF1G | eukaryotic translation elongation factor 1 gamma |
| IPI00014398 | FHL1 | four and a half LIM domains 1 |
| IPI00301395 | CPVL | carboxypeptidase, vitellogenic-like |
| IPI00790262 | TTLL3 | tubulin tyrosine ligase-like family, member 3 |
| IPI00942608 | 26kDaprotein | IPI:IPI00942608.1 \|ENSEMBL:ENSP0000 |
| IPI00023038 | PRB1/PRB2 | proline-rich protein BstNI subfamily 2 |
| IPI00001895 | PCDH8 | Protocadherin-8 |
| IPI00419215 | A2ML1 | Alpha-2-macroglobulin-like protein 1 |
| IPI00644409 | GDA | Guanine deaminase |
| IPI00009650 | LCN1 | Lipocalin-1 |
| IPI00217467 | HIST1H1E | Histone H1.4 |
| IPI00937064 | ZAN | IPI:IPI00937064.1\|REFSEQ:XP_002342720 |
| IPI00396378 | HNRNPA2B1 | heterogeneous nuclear ribonucleoprotein A2/B1 |
| IPI00465261 | ERAP2 | endoplasmic reticulum aminopeptidase 2 |
| IPI00021263 | YWHAZ | 14-3-3 protein zeta/delta |
| IPI00011241 | GPR39 | G-protein coupled receptor 39 |
| IPI00786880 | KIAA1783 | similar to KIAA1783 protein |
| IPI00168479 | APOA1 BP | apolipoprotein A-I binding protein |
| IPI00031084 | PSD2 | pleckstrin and Sec7 domain containing 2 |
| IPI00001593 | PRCP | prolylcarboxypeptidase (angiotensinase C) |
| IPI00218343 | TUBA1C | Tubulin alpha-1C chain |
| IPI00021536 | CALML5 | Calmodulin-like protein 5 |
| IPI00028091 | ACTR3 | ARP3 actin-related protein 3 homolog (yeast) |
| IPI00796366 | MYL6 | myosin, light chain 6, alkali, smooth muscle and non-muscle |
| IPI00301058 | VASP | Vasodilator-stimulated phosphoprotein |
| IPI00470573 | ACTR2 | ARP2 actin-related protein 2 homolog (yeast) |
| IPI00884078 | RF-IP18 | Rheumatoid factor RF-IP18 |
| IPI00169383 | PGK1 | Phosphoglycerate kinase 1 |
| IPI00299619 | SLC35F1 | Solute carrier family 35 member F1 |
| IPI00419916 | ALPL | alkaline phosphatase, liver/bone/kidney |
| IPI00218319 | TPM3 | tropomyosin 3 |
| IPI00005118 | HK3 | Hexokinase-3 |
| IPI00418471 | VIM | Vimentin |
| IPI00218918 | ANXA1 | Annexin A1 |
| IPI00930073 | KRT6C | IPI:IPI00930073.1 \|TREMBL:B2R853 |
| IPI00299145 | KRT6C | Keratin, type II cytoskeletal 6C |
| IPI00007858 | MYH13 | myosin, heavy chain 13, skeletal muscle |
| IPI00297235 | CCPG1 | cell cycle progression 1 |
| IPI00927726 | H-INV | Hypothetical protein |
| IPI00009008 | CACNA1D | calcium channel, voltage-dependent, L type, alpha 1D subunit |
| IPI00304554 | LYPD5 | LY6/PLAUR domain containing 5 |
| IPI00815807 | ADCK2 | aarF domain containing kinase 2 |
| IPI00743335 | MYO1C | Myosin-Ic |
| IPI00007393 | APPBP2 | amyloid beta precursor protein (cytoplasmic tail) binding protein 2 |
| IPI00295976 | ITGA2B | integrin, alpha 2b (platelet glycoprotein IIb of IIb/IIIa complex, antigen CD41) |
| IPI00641706 | TUBB6 | tubulin, beta 6 |
| IPI00060201 | SYTL4 | synaptotagmin-like 4 |
| IPI00908634 | AQP4 | aquaporin 4 |
| IPI00016786 | CDC42 | cell division cycle 42 (GTP binding protein, 25kDa) |
| IPI00033494 | MYL12B | myosin, light chain 12B, regulatory |
| IPI00409756 | LOC100293553 | protein L-Myc-2-like |
| IPI00015148 | RAP1B | RAP1B, member of RAS oncogene family |
| IPI00027502 | GP9 | glycoprotein IX (platelet) |
| IPI00473014 | DSTN | Destrin |
| IPI00022394 | C1QC | complement component 1, q subcomponent, C chain |
| IPI00290337 | EPS8 | epidermal growth factor receptor pathway substrate 8 |
| IPI00018671 | DUSP3 | dual specificity phosphatase 3 |
| IPI00478231 | RHOA | ras homolog gene family, member A |
| IPI00220278 | MYL9 | myosin, light chain 9, regulatory |
| IPI00419585 | PPIA | peptidylprolyl isomerase A (cyclophilin A) |
| IPI00012011 | CFL1 | Cofilin-1 |

**Table 2 - A subset of biomarkers from Table 1 which are significantly differentially expressed in all CRC samples relative to a control sample**

| Accession Number | Gene Symbol | Entrez Gene Name | Fold Change Control vs CRC | P-values Control vs CRC | Identified in nr. CRCs (n=12) | Identified in nr. Controls (n=10) | Detected in plasma | Identified in Cancer Cell Lines | mean spectral counts per positive CRC | mean spectral counts per positive control |
|---|---|---|---|---|---|---|---|---|---|---|
| IPI00892604 | C4A/C4B | complement component 4B (Chido blood group) | ∼ | 2.45E-06 | 10 | 0 | no | yes | 11 | na |
| IPI00018206 | GOT2 | glutamic-oxaloacetic transaminase 2, mitochondrial (aspartate aminotransferase 2) | ∼ | 4.93E-03 | 5 | 0 | no | yes | 9 | na |
| IPI00027497 | GPI | glucose-6-phosphate isomerase | 49.74 | 5.44E-03 | 7 | 1 | no | yes | 7 | 1 |
| IPI00643920 | TKT | transketolase | 24.13 | 1.71E-02 | 6 | 1 | no | yes | 6 | 1 |
| IPI00337741 | APEH | N-acylaminoacyl-peptide hydrolase | 12.45 | 4.72E-04 | 9 | 2 | no | yes | 6 | 2 |
| IPI00453473 | HIST4H4 (includes others) | histone cluster 1, H4c | 2.72 | 4.42E-02 | 9 | 4 | no | yes | 5 | 2 |
| IPI00007797 | FABP5 | fatty acid binding protein 5 (psoriasis-associated) | ∼ | 3.48E-02 | 3 | 0 | no | yes | 5 | na |
| IPI00012585 | HEXB | hexosaminidase B (beta polypeptide) | 14.77 | 1.35E-02 | 6 | 1 | no | yes | 5 | 2 |
| IPI00296215 | EPCAM | epithelial cell adhesion molecule | ∼ | 3.56E-02 | 3 | 0 | no | yes | 4 | na |
| IPI00604590 | NME1-NME2 | NME1-NME2 readthrough | ∼ | 1.34E-02 | 4 | 0 | no | yes | 4 | na |
| IPI00022314 | SOD2 | superoxide dismutase 2, mitochondrial | ∼ | 6.04E-03 | 5 | 0 | no | yes | 4 | na |
| IPI00027107 | TUFM | Tu translation elongation factor, mitochondrial | 2.02 | 7.75E-03 | 10 | 2 | no | yes | 3 | 5 |
| IPI00010706 | GSS | glutathione synthetase | ∼ | 1.08E-02 | 4 | 0 | no | yes | 3 | na |
| IPI00418169 | ANXA2 | annexin A2 | ∼ | 4.47E-03 | 5 | 0 | no | yes | 3 | na |
| IPI00303476 | ATP5B | ATP synthase, H+ transporting, mitochondrial F1 complex, beta polypeptide | 7.02 | 2.62E-02 | 6 | 1 | no | yes | 3 | 1 |
| IPI00220362 | HSPE1 | heat shock 10kDa protein 1 (chaperonin 10) | ∼ | 9.57E-03 | 4 | 0 | no | yes | 3 | na |
| IPI00220766 | GLO1 | glyoxalase I | ∼ | 5.24E-04 | 7 | 0 | no | yes | 2 | na |
| IPI00003935 | HIST2H2BE (includes others) | histone cluster 2, H2be | 9.25 | 3.83E-02 | 5 | 1 | no | yes | 2 | 1 |
| IPI00032313 | S100A4 | S100 calcium binding protein A4 | ∼ | 1.40E-02 | 4 | 0 | no | yes | 2 | na |
| IPI00013895 | S100A11 | S100 calcium binding protein A11 | ∼ | 3.55E-03 | 5 | 0 | no | yes | 2 | na |
| IPI00106687 | LXN | latexin | 4.85 | 1.98E-02 | 7 | 2 | no | yes | 2 | 2 |
| IPI00034280 | DHRS11 | dehydrogenase/ reductase (SDR family) member 11 | 7.23 | 6.98E-03 | 8 | 2 | no | yes | 2 | 1 |
| IPI00008787 | NAGLU | N-acetylglucosaminidase, alpha | 11.00 | 4.44E-03 | 7 | 1 | no | yes | 2 | 1 |
| IPI00018768 | TSN | translin | ∼ | 1.45E-02 | 4 | 0 | no | yes | 2 | na |
| IPI00299155 | PSMA4 | proteasome (prosome, macropain) subunit, alpha type, 4 | ∼ | 1.48E-02 | 4 | 0 | no | yes | 2 | na |
| IPI00029623 | PSMA6 | proteasome (prosome, macropain) subunit, alpha type, 6 | ∼ | 3.02E-02 | 3 | 0 | no | yes | 2 | na |
| IPI00010271 | RAC1 | ras-related C3 botulinum toxin substrate 1 (rho family, small GTP binding protein Rac1) | ∼ | 4.46E-02 | 3 | 0 | no | yes | 2 | na |
| IPI00012007 | AHCY | Adenosyl homocysteinase | ∼ | 1.68E-03 | 6 | 0 | no | yes | 2 | na |
| IPI00385751 | FUCA1 | fucosidase, alpha-L- 1, tissue | 14.06 | 2.67E-02 | 6 | 1 | no | yes | 2 | 1 |
| IPI00017526 | S100P | S100 calcium binding protein P | ∼ | 3.57E-03 | 5 | 0 | no | yes | 2 | na |
| IPI00028006 | PSMB2 | proteasome (prosome, macropain) subunit, beta type, 2 | ∼ | 3.77E-03 | 5 | 0 | no | yes | 2 | na |
| IPI00793375 | XPNPEP1 | X-prolyl aminopeptidase (aminopeptidase P) 1, soluble | ∼ | 3.35E-02 | 3 | 0 | no | yes | 2 | na |
| IPI00554788 | KRT18 | keratin 18 | ∼ | 3.79E-02 | 3 | 0 | no | yes | 2 | na |
| IPI00019380 | NCBP1 | nuclear cap binding protein subunit 1, 80kDa | ∼ | 4.02E-02 | 3 | 0 | no | yes | 2 | na |
| IPI00012989 | MAN2B1 | mannosidase, alpha, class 2B, member 1 | 7.64 | 2.11E-02 | 7 | 2 | no | yes | 1 | 1 |
| IPI00027463 | S100A6 | S100 calcium binding protein A6 | 4.33 | 7.50E-03 | 10 | 3 | no | yes | 1 | 1 |
| IPI00022774 | VCP | valosin-containing protein | ∼ | 3.16E-02 | 3 | 0 | no | yes | 1 | na |
| IPI00300086 | QPRT | quinolinate phosphoribosyltransferase | ∼ | 1.33E-02 | 4 | 0 | no | yes | 1 | na |
| IPI00472073 | HLA-B | major histocompatibility complex, class I, B | ∼ | 5.15E-03 | 5 | 0 | no | yes | 1 | na |
| IPI00453476 | PGAM1 | phosphoglycerate mutase 1 (brain) | ∼ | 2.06E-02 | 4 | 0 | no | yes | 1 | na |
| IPI00020999 | ENPP3 | ectonucleotide pyrophosphatase/phosphodiesterase 3 | 2.77 | 6.30E-04 | 10 | 2 | no | no | 11 | 14 |
| IPI00010304 | SERPINB10 | serpin peptidase inhibitor, clade B (ovalbumin), member 10 | 32.16 | 2.84E-02 | 6 | 1 | no | no | 10 | 4 |
| IPI00007244 | MPO | myeloperoxidase | 22.73 | 5.30E-04 | 10 | 3 | no | no | 10 | 2 |
| IPI00877726 | CKMT1A/CKMT1B | creatine kinase, mitochondrial 1B | 2.08 | 2.15E-03 | 11 | 4 | no | no | 7 | 7 |
| IPI00027409 | PRTN3 | proteinase 3 | 93.49 | 1.90E-05 | 10 | 1 | no | no | 5 | 1 |
| IPI00027769 | ELANE | elastase, neutrophil expressed | 23.48 | 1.47E-02 | 6 | 1 | no | no | 5 | 1 |
| IPI00004362 | MORC1 | MORC family CW-type zinc finger 1 | 6.22 | 3.95E-02 | 5 | 1 | no | no | 4 | 1 |
| IPI00719280 | UBB | ubiquitin B | 3.45 | 1.49E-02 | 11 | 6 | no | no | 4 | 2 |
| IPI00026962 | PLA2G2A | phospholipase A2, group IIA (platelets, synovial fluid) | 6.13 | 4.30E-03 | 7 | 1 | no | no | 3 | 1 |
| IPI00027466 | CA4 | carbonic anhydrase IV | 4.02 | 3.87E-03 | 10 | 2 | no | no | 3 | 4 |
| IPI00071703 | GFM2 | G elongation factor, mitochondrial 2 | 4.03 | 2.72E-02 | 10 | 5 | no | no | 3 | 1 |
| IPI00219806 | S100A7 | S100 calcium binding protein A7 | 3.15 | 4.75E-02 | 7 | 4 | no | no | 3 | 1 |
| IPI00827847 | BPI | bactericidal/permeability -increasing protein | ∼ | 1.61E-02 | 4 | 0 | no | no | 3 | na |
| IPI00176125 | COL6A5 | collagen, type VI, alpha 5 | 2.74 | 2.13E-02 | 8 | 2 | no | no | 3 | 2 |
| IPI00470355 | LHX8 | LIM homeobox 8 | ∼ | 1.30E-02 | 4 | 0 | no | no | 3 | na |
| IPI00942117 | CRISP3 | cysteine-rich secretory protein 3 | ∼ | 1.45E-02 | 4 | 0 | no | no | 2 | na |
| IPI00022246 | AZU1 | azurocidin 1 | ∼ | 2.27E-05 | 8 | 0 | no | no | 2 | na |
| IPI00045512 | HMCN1 | hemicentin 1 | 8.30 | 6.80E-03 | 8 | 2 | no | no | 2 | 1 |
| IPI00300376 | TGM3 | transglutaminase 3 (E polypeptide, protein-glutamine-gamma-glutamyltransferase) | ∼ | 3.67E-02 | 3 | 0 | no | no | 2 | na |
| IPI00640468 | CDC42BPA | CDC42 binding protein kinase alpha (DMPK-like) | 1.88 | 4.92E-02 | 8 | 3 | no | no | 2 | 1 |
| IPI00028064 | CTSG | cathepsin G | ∼ | 1.03E-02 | 4 | 0 | no | no | 2 | na |
| IPI00006988 | RETN | resistin | ∼ | 3.74E-02 | 3 | 0 | no | no | 2 | na |
| IPI00024934 | MUT | methylmalonyl CoA mutase | 11.42 | 7.07E-03 | 8 | 2 | no | no | 2 | 1 |
| IPI00552983 | ARMCX4 | armadillo repeat containing, X-linked 4 | 12.86 | 1.65E-02 | 7 | 1 | no | no | 2 | 2 |
| IPI00217987 | ITGAM | integrin, alpha M (complement component 3 receptor 3 subunit) | ∼ | 1.59E-02 | 4 | 0 | no | no | 2 | na |
| IPI00165045 | CACNA1E | calcium channel, voltage-dependent, R type, alpha 1E subunit | ∼ | 7.77E-04 | 7 | 0 | no | no | 1 | na |
| IPI00018363 | TIAM2 | T-cell lymphoma invasion and metastasis 2 | 18.20 | 4.58E-03 | 7 | 1 | no | no | 1 | 1 |
| IPI00217560 | HIRA | HIR histone cell cycle regulation defective homolog A (S. cerevisiae) | ∼ | 8.36E-03 | 5 | 0 | no | no | 1 | na |
| IPI00294653 | DOPEY2 | dopey family member 2 | 13.89 | 2.46E-02 | 6 | 1 | no | no | 1 | 1 |
| IPI00004401 | ITGB1BP3 | integrin beta 1 binding protein 3 | ∼ | 3.38E-02 | 3 | 0 | no | no | 1 | na |
| IPI00300117 | SCN7A | sodium channel, voltage-gated, type VII, alpha | ∼ | 3.38E-02 | 3 | 0 | no | no | 1 | na |
| IPI00061114 | RAB3C | RAB3C, member RAS oncogene family | ∼ | 3.41E-02 | 3 | 0 | no | no | 1 | na |
| IPI00168442 | C9orf79 | chromosome 9 open reading frame 79 | ∼ | 3.89E-02 | 3 | 0 | no | no | 1 | na |
| IPI00385215 | NFATC4 | nuclear factor of activated T-cells, cytoplasmic, calcineurin-dependent 4 | ∼ | 3.95E-02 | 3 | 0 | no | no | 1 | na |
| IPI00024467 | UGGT2 | UDP-glucose glycoprotein glucosyltransferase 2 | ∼ | 4.02E-02 | 3 | 0 | no | no | 1 | na |
| IPI00297056 | CRNN | cornulin | ∼ | 4.04E-02 | 3 | 0 | no | no | 1 | na |
| IPI00914663 | KCP | kielin/chordin-like protein | ∼ | 9.68E-03 | 4 | 0 | no | no | 1 | na |
| IPI00418592 | CD1E | CD1e molecule | ∼ | 1.46E-02 | 4 | 0 | no | no | 1 | na |
| IPI00642206 | CCDC18 | coiled-coil domain containing 18 | ∼ | 4.58E-03 | 5 | 0 | no | no | 1 | na |
| IPI00514090 | LTA4H | leukotriene A4 hydrolase | ∼ | 4.50E-02 | 3 | 0 | no | no | 1 | na |
| IP100745872 | ALB | albumin | 4.71 | 1.41E-02 | 12 | 10 | yes | yes | 576 | 124 |
| IPI00478003 | A2M | alpha-2-macroglobulin | 34.08 | 9.01E-06 | 12 | 6 | yes | yes | 141 | 11 |
| IPI00783987 | C3 | complement component 3 | 43.72 | 2.21E-04 | 12 | 6 | yes | yes | 119 | 5 |
| IPI00654755 | HBB | hemoglobin, beta | 9.87 | 5.89E-05 | 12 | 7 | yes | yes | 108 | 14 |
| IPI00022463 | TF | transferrin | 1713.68 | 7.84E-06 | 11 | 1 | yes | yes | 96 | 1 |
| IPI00410714 | HBA1/HBA2 | hemoglobin, alpha 1 | 44.44 | 1.77E-04 | 12 | 5 | yes | yes | 68 | 2 |
| IPI00298860 | LTF | lactotransferrin | 59.22 | 3.14E-05 | 10 | 2 | yes | yes | 43 | 6 |
| IPI00017601 | CP | ceruloplasmin (ferroxidase) | 94.27 | 1.28E-04 | 11 | 3 | yes | yes | 41 | 2 |
| IPI00465436 | CAT | catalase | 77.03 | 1.66E-03 | 10 | 3 | yes | yes | 24 | 1 |
| IPI00555812 | GC | group-specific component (vitamin D binding protein) | ∼ | 3.43E-02 | 3 | 0 | yes | yes | 20 | na |
| IPI00032179 | SERPINC1 | serpin peptidase inhibitor, clade C (antithrombin), member 1 | 2.17 | 3.20E-02 | 12 | 10 | yes | yes | 20 | 11 |
| IPI00219713 | FGG | fibrinogen gamma chain | ~ | 1.11E-03 | 6 | 0 | yes | yes | 20 | na |
| IPI00007047 | S100A8 | S100 calcium binding protein A8 | 3.87 | 1.12E-06 | 12 | 9 | yes | yes | 17 | 5 |
| IPI00375676 | FTL | ferritin, light polypeptide | 2.51 | 2.92E-02 | 12 | 10 | yes | yes | 12 | 5 |
| IPI00021439 | ACTB | actin, beta | 2.44 | 1.07E-02 | 10 | 3 | yes | yes | 12 | 11 |
| IPI00022418 | FN1 | fibronectin 1 | 17.62 | 1.31E-02 | 10 | 4 | yes | yes | 11 | 2 |
| IPI00005721 | DEFA1 (includes others) | defensin, alpha 1 | 4.16 | 4.47E-05 | 12 | 9 | yes | yes | 8 | 2 |
| IPI00291866 | SERPING1 | serpin peptidase inhibitor, clade G (C1 inhibitor), member 1 | ~ | 2.88E-04 | 7 | 0 | yes | yes | 8 | na |
| IPI00022420 | RBP4 | retinol binding protein 4, plasma | 4.47 | 1.20E-02 | 8 | 3 | yes | yes | 8 | 6 |
| IPI00027350 | PRDX2 | peroxiredoxin 2 | 36.98 | 4.57E-03 | 7 | 1 | yes | yes | 6 | 1 |
| IPI00021885 | FGA | fibrinogen alpha chain | 21.98 | 3.30E-02 | 6 | 2 | yes | yes | 6 | 1 |
| IPI00029863 | SERPINF2 | serpin peptidase inhibitor, clade F (alpha-2 antiplasmin, pigment epithelium derived factor), member 2 | 8.61 | 8.05E-04 | 9 | 1 | yes | yes | 4 | 8 |
| IPI00218414 | CA2 | carbonic anhydrase II | 2.87 | 1.12E-02 | 7 | 1 | yes | yes | 4 | 10 |
| IPI00020091 | ORM1/ ORM2 | orosomucoid 1 | 4.11 | 2.56E-02 | 9 | 3 | yes | yes | 4 | 3 |
| IPI00217966 | LDHA | lactate dehydrogenase A | ~ | 1.85E-03 | 6 | 0 | yes | yes | 4 | na |
| IPI00298971 | VTN | vitronectin | ~ | 1.15E-03 | 6 | 0 | yes | yes | 4 | na |
| IPI00215894 | KNG1 | kininogen 1 | ~ | 3.33E-04 | 7 | 0 | yes | yes | 4 | na |
| IPI00023006 | ACTC1 | actin, alpha, cardiac muscle 1 | 3.17 | 2.33E-03 | 10 | 3 | yes | yes | 4 | 2 |
| IPI00022417 | LRG1 | leucine-rich alpha-2-glycoprotein 1 | 31.63 | 4.06E-03 | 7 | 1 | yes | yes | 3 | 1 |
| IPI00023728 | GGH | gamma-glutamyl hydrolase (conjugase, folylpolygammaglutamyl hydrolase) | 6.57 | 1.73E-02 | 7 | 2 | yes | yes | 3 | 3 |
| IPI00465248 | ENO1 | enolase 1, (alpha) | 5.38 | 3.17E-02 | 7 | 2 | yes | yes | 3 | 1 |
| IPI00216691 | PFN1 | profilin 1 | ∼ | 4.15E-02 | 3 | 0 | yes | yes | 3 | na |
| IPI00292946 | SERPINA7 | serpin peptidase inhibitor, clade A (alpha-1 antiproteinase, antitrypsin), member 7 | ∼ | 6.51E-04 | 7 | 0 | yes | yes | 3 | na |
| IPI00022426 | AMBP | alpha-1-microglobulin/bikunin precursor | ∼ | 1.75E-03 | 6 | 0 | yes | yes | 3 | na |
| IPI00021405 | LMNA | lamin A/C | ∼ | 1.47E-02 | 4 | 0 | yes | yes | 3 | na |
| IPI00006662 | APOD | apolipoprotein D | 3.38 | 4.74E-03 | 8 | 1 | yes | yes | 2 | 4 |
| IPI00003515 | TRIP11 | thyroid hormone receptor interactor 11 | 7.65 | 3.20E-03 | 9 | 1 | yes | yes | 2 | 2 |
| IPI00021727 | C4BPA | complement component 4 binding protein, alpha | ∼ | 1.23E-02 | 4 | 0 | yes | yes | 2 | na |
| IPI00010779 | TPM4 | tropomyosin 4 | ∼ | 4.12E-02 | 3 | 0 | yes | yes | 2 | na |
| IPI00302592 | FLNA | filamin A, alpha | ∼ | 2.45E-03 | 6 | 0 | yes | yes | 1 | na |
| IPI00641737 | HP | haptoglobin | 18.82 | 2.89E-04 | 11 | 6 | yes | no | 89 | 6 |
| IPI00022488 | HPX | hemopexin | ∼ | 4.42E-05 | 8 | 0 | yes | no | 23 | na |
| IPI00473011 | HBD | hemoglobin, delta | ∼ | 6.31E-05 | 8 | 0 | yes | no | 15 | na |
| IPI00298497 | FGB | fibrinogen beta chain | ∼ | 1.18E-02 | 4 | 0 | yes | no | 11 | na |
| IPI00027462 | S100A9 | S100 calcium binding protein A9 | 4.41 | 1.82E-05 | 12 | 8 | yes | no | 10 | 3 |
| IPI00032291 | C5 | complement component 5 | 33.44 | 9.34E-03 | 8 | 2 | yes | no | 8 | 1 |
| IPI00031036 | SLC26A3 | solute carrier family 26, member 3 | 64.79 | 1.18E-02 | 7 | 1 | yes | no | 6 | 1 |
| IPI00022395 | C9 | complement component 9 | 12.69 | 4.19E-04 | 11 | 5 | yes | no | 6 | 1 |
| IPI00022391 | APCS | amyloid P component, serum | 5.75 | 2.53E-03 | 10 | 3 | yes | no | 6 | 5 |
| IPI00022895 | A1BG | alpha-1-B glycoprotein | ∼ | 1.31E-02 | 4 | 0 | yes | no | 6 | na |
| IPI00887739 | LOC100133511 | complement C3-like | ∼ | 4.38E-03 | 5 | 0 | yes | no | 5 | na |
| IPI00218192 | ITIH4 | inter-alpha (globulin) inhibitor H4 (plasma Kallikrein-sensitive glycoprotein) | ∼ | 3.64E-02 | 3 | 0 | yes | no | 5 | na |
| IPI00011252 | C8A | complement component 8, alpha polypeptide | ∼ | 3.26E-02 | 3 | 0 | yes | no | 3 | na |
| IPI00292530 | ITIH1 | inter-alpha (globulin) inhibitor H1 | ∼ | 1.05E-02 | 4 | 0 | yes | no | 1 | na |

**Table 3 - A subset of biomarkers from Table 1 which are differentially expressed in FIT-negative CRC samples relative to a control sample**

| Accession Number | Gene Symbol | Entrez Gene Name |
|---|---|---|
| IPI00022426 | AMBP | alpha-1-microglobulin/bikunin precursor |
| IPI00022246 | AZU1 | azurocidin 1 |
| IPI00827847 | BPI | bactericidal/permeability-increasing protein |
| IPI00892604 | C4A/C4B | complement component 4B (Chido blood group) |
| IPI00021727 | C4BPA | complement component 4 binding protein, alpha |
| IPI00168442 | C9orf79 | chromosome 9 open reading frame 79 |
| IPI00165045 | CACNA1E | calcium channel, voltage-dependent, R type, alpha 1E subunit |
| IPI00642206 | CCDC18 | coiled-coil domain containing 18 |
| IPI00297056 | CRNN | cornulin |
| IPI00296215 | EPCAM | epithelial cell adhesion molecule |
| IPI00219713 | FGG | fibrinogen gamma chain |
| IPI00302592 | FLNA | filamin A, alpha |
| IPI00220766 | GLO1 | glyoxalase I |
| IPI00018206 | GOT2 | glutamic-oxaloacetic transaminase 2, mitochondrial (aspartate aminotransferase 2) |
| IPI00010706 | GSS | glutathione synthetase |
| IPI00217560 | HIRA | HIR histone cell cycle regulation defective homolog A (S. cerevisiae) |
| IPI00472073 | HLA-B | major histocompatibility complex, class I, B |
| IPI00022488 | HPX | hemopexin |
| IPI00217987 | ITGAM | integrin, alpha M (complement component 3 receptor 3 subunit) |
| IPI00215894 | KNG1 | kininogen 1 |
| IPI00554788 | KRT18 | keratin 18 |
| IPI00217966 | LDHA | lactate dehydrogenase A |
| IPI00470355 | LHX8 | LIM homeobox 8 |
| IPI00887739 | LOC100133511 | complement C3-like |
| IPI00019380 | NCBP1 | nuclear cap binding protein subunit 1, 80kDa |
| IPI00604590 | NME1-NME2 | NME1-NME2 readthrough |
| IPI00299155 | PSMA4 | proteasome (prosome, macropain) subunit, alpha type, 4 |
| IPI00300117 | SCN7A | sodium channel, voltage-gated, type VII, alpha |
| IPI00292946 | SERPINA7 | serpin peptidase inhibitor, clade A (alpha-1 antiproteinase, antitrypsin), member 7 |
| IPI00291866 | SERPING1 | serpin peptidase inhibitor, clade G (C1 inhibitor), member 1 |
| IPI00024467 | UGGT2 | UDP-glucose glycoprotein glucosyltransferase 2 |
| IPI00298971 | VTN | vitronectin |
| IPI00478003 | A2M | alpha-2-macroglobulin |
| IPI00022391 | APCS | amyloid P component, serum |
| IPI00337741 | APEH | N-acylaminoacyl-peptide hydrolase |
| IPI00552983 | ARMCX4 | armadillo repeat containing, X-linked 4 |
| IPI00783987 | C3 | complement component 3 |
| IPI00032291 | C5 | complement component 5 |
| IPI00022395 | C9 | complement component 9 |
| IPI00027466 | CA4 | carbonic anhydrase IV |
| IPI00465436 | CAT | catalase |
| IPI00176125 | COL6A5 | collagen, type VI, alpha 5 |
| IPI00017601 | CP | ceruloplasmin (ferroxidase) |
| IPI00005721 | DEFA1(include sothers) | defensin, alpha 1 |
| IPI00294653 | DOPEY2 | dopey family member 2 |
| IPI00020999 | ENPP3 | ectonucleotide pyrophosphatase/phosphodiesterase 3 |
| IPI00022418 | FN1 | fibronectin 1 |
| IPI00375676 | FTL | ferritin, light polypeptide |
| IPI00385751 | FUCA1 | fucosidase, alpha-L- 1, tissue |
| IPI00071703 | GFM2 | G elongation factor, mitochondrial 2 |
| IPI00023728 | GGH | gamma-glutamyl hydrolase (conjugase, folylpolygammaglutamyl hydrolase) |
| IPI00027497 | GPI | glucose-6-phosphate isomerase |
| IPI00410714 | HBA1/HBA2 | hemoglobin, alpha 1 |
| IPI00654755 | HBB | hemoglobin, beta |
| IPI00012585 | HEXB | hexosaminidase B (beta polypeptide) |
| IPI00045512 | HMCN1 | hemicentin 1 |
| IPI00022417 | LRG1 | leucine-rich alpha-2-glycoprotein 1 |
| IPI00298860 | LTF | lactotransferrin |
| IPI00106687 | LXN | latexin |
| IPI00012989 | MAN2B1 | mannosidase, alpha, class 2B, member 1 |
| IPI00007244 | MPO | myeloperoxidase |
| IPI00024934 | MUT | methylmalonyl CoA mutase |
| IPI00026962 | PLA2G2A | phospholipase A2, group IIA (platelets, synovial fluid) |
| IPI00027350 | PRDX2 | peroxiredoxin 2 |
| IPI00027409 | PRTN3 | proteinase 3 |
| IPI00022420 | RBP4 | retinol binding protein 4, plasma |
| IPI00027463 | S100A6 | S100 calcium binding protein A6 |
| IPI00219806 | S100A7 | S100 calcium binding protein A7 |
| IPI00007047 | S100A8 | S100 calcium binding protein A8 |
| IPI00027462 | S100A9 | S100 calcium binding protein A9 |
| IPI00010304 | SERPINB10 | serpin peptidase inhibitor, clade B (ovalbumin), member 10 |
| IPI00029863 | SERPINF2 | serpin peptidase inhibitor, clade F (alpha-2 antiplasmin, pigment epithelium derived factor), member 2 |
| IPI00031036 | SLC26A3 | solute carrier family 26, member 3 |
| IPI00022463 | TF | transferrin |
| IPI00018363 | TIAM2 | T-cell lymphoma invasion and metastasis 2 |
| IPI00643920 | TKT | transketolase |
| IPI00003515 | TRIP11 | thyroid hormone receptor interactor 11 |
| IPI00027107 | TUFM | Tu translation elongation factor, mitochondrial |
| IPI00181135 | BCAT2 | branched chain amino-acid transaminase 2, mitochondrial |
| IPI00024701 | COL4A3BP | collagen, type IV, alpha 3 (Goodpasture antigen) binding protein |
| IPI00032134 | SERPINB8 | serpin peptidase inhibitor, clade B (ovalbumin), member 8 |
| IPI00333141 | SPNS3 | spinster homolog 3 (Drosophila) |
| IPI00937735 | ACADVL | acyl-CoA dehydrogenase, very long chain |
| IPI00009268 | ACY1 | Unmapped by Ingenuity |
| IPI00166331 | ANKRD35 | ankyrin repeat domain 35 |
| IPI00299635 | BIRC6 | baculoviral IAP repeat-containing 6 |
| IPI00219575 | BLMH | bleomycin hydrolase |
| IPI00026240 | BST1 | bone marrow stromal cell antigen 1 |
| IPI00929313 | C13orf40 | chromosome 13 open reading frame 40 |
| IPI00027983 | CDA | cytidine deaminase |
| IPI00852865 | CHIT1 | chitinase 1 (chitotriosidase) |
| IPI00022810 | CTSC | cathepsin C |
| IPI00299150 | CTSS | cathepsin S |
| IPI00006024 | DOCK4 | dedicator of cytokinesis 4 |
| IPI00016862 | GSR | glutathione reductase |
| IPI00941901 | HERC1 | hect (homologous to the E6-AP (UBE3A) carboxyl terminus) domain and RCC1 (CHC1)-like domain (RLD) 1 |
| IPI00005826 | HERC2 | hect domain and RLD 2 |
| IPI00746667 | HLA-DRB5 | major histocompatibility complex, class II, DR beta 5 |
| IPI00027223 | IDH1 | isocitrate dehydrogenase 1 (NADP+), soluble |
| IPI00305461 | ITIH2 | inter-alpha (globulin) inhibitor H2 |
| IPI00748360 | KIAA1797 | KIAA1797 |
| IPI00019038 | LYZ | lysozyme |
| IPI00303335 | NEB | nebulin |
| IPI00065454 | NEK10 | NIMA (never in mitosis gene a)- related kinase 10 |
| IPI00257882 | PEPD | peptidase D |
| IPI00003590 | QSOX1 | quiescin Q6 sulfhydryl oxidase 1 |
| IPI00939604 | RNASET2 | ribonuclease T2 |
| IPI00553177 | SERPINA1 | serpin peptidase inhibitor, clade A (alpha-1 antiproteinase, antitrypsin), member 1 |
| IPI00550991 | SERPINA3 | serpin peptidase inhibitor, clade A (alpha-1 antiproteinase, antitrypsin), member 3 |
| IPI00022204 | SERPINB3 | serpin peptidase inhibitor, clade B (ovalbumin), member 3 |
| IPI00307733 | SETD2 | SET domain containing 2 |
| IPI00218013 | SGOL2 | shugoshin-like 2 |
| IPI00010949 | SIAE | sialic acid acetylesterase |
| IPI00386444 | SYNE1 | spectrin repeat containing, nuclear envelope 1 |
| IPI00744692 | TALDO1 | transaldolase 1 |
| IPI00375371 | TAS2R42 | taste receptor, type 2, member 42 |
| IPI00465028 | TPI1 | triosephosphate isomerase 1 |
| IPI00291175 | VCL | vinculin |
| IPI00029647 | ZG16 | zymogen granule protein 16 |

**Table 4 - A subset of biomarkers from Table 1 which were found to be expressed only in CRC samples and not in control samples**

| Accession Number | Gene Symbol | Entrez Gene Name |
|---|---|---|
| IPI00892604 | C4A/C4B | complement component 4B (Chido blood group) |
| IPI00018206 | GOT2 | glutamic-oxaloacetic transaminase 2, mitochondrial (aspartate aminotransferase 2) |
| IPI00007797 | FABP5 | fatty acid binding protein 5 (psoriasis-associated) |
| IPI00296215 | EPCAM | epithelial cell adhesion molecule |
| IPI00604590 | NME1-NME2 | NME1-NME2 readthrough |
| IPI00022314 | SOD2 | superoxide dismutase 2, mitochondrial |
| IPI00010706 | GSS | glutathione synthetase |
| IPI00418169 | ANXA2 | annexin A2 |
| IPI00220362 | HSPE1 | heat shock 10kDa protein 1 (chaperonin 10) |
| IPI00220766 | GLO1 | glyoxalase I |
| IPI00032313 | S100A4 | S100 calcium binding protein A4 |
| IPI00013895 | S100A11 | S100 calcium binding protein A11 |
| IPI00018768 | TSN | translin |
| IPI00299155 | PSMA4 | proteasome (prosome, macropain) subunit, alpha type, 4 |
| IPI00029623 | PSMA6 | proteasome (prosome, macropain) subunit, alpha type, 6 |
| IPI00010271 | RAC1 | ras-related C3 botulinum toxin substrate 1 (rho family, small GTP binding protein Rac1) |
| IPI00012007 | AHCY | adenosylhomocysteinase |
| IPI00017526 | S100P | S100 calcium binding protein P |
| IPI00028006 | PSMB2 | proteasome (prosome, macropain) subunit, beta type, 2 |
| IPI00793375 | XPNPEP1 | X-prolyl aminopeptidase (aminopeptidase P) 1, soluble |
| IPI00554788 | KRT18 | keratin 18 |
| IPI00019380 | NCBP1 | nuclear cap binding protein subunit 1, 80kDa |
| IPI00022774 | VCP | valosin-containing protein |
| IPI00300086 | QPRT | quinolinate phosphoribosyltransferase |
| IPI00472073 | HLA-B | major histocompatibility complex, class I, B |
| IPI00453476 | PGAM1 | phosphoglycerate mutase 1 (brain) |
| IPI00827847 | BPI | bactericidal/permeability-increasing protein |
| IPI00470355 | LHX8 | LIM homeobox 8 |
| IPI00942117 | CRISP3 | cysteine-rich secretory protein 3 |
| IPI00022246 | AZU1 | azurocidin 1 |
| IPI00300376 | TGM3 | transglutaminase 3 (E polypeptide, protein-glutamine-gamma-glutamyltransferase) |
| IPI00028064 | CTSG | cathepsin G |
| IPI00006988 | RETN | resistin |
| IPI00217987 | ITGAM | integrin, alpha M (complement component 3 receptor 3 subunit) |
| IPI00165045 | CACNA1E | calcium channel, voltage-dependent, R type, alpha 1E subunit |
| IPI00217560 | HIRA | HIR histone cell cycle regulation defective homolog A (S. cerevisiae) |
| IPI00004401 | ITGB1 BP3 | integrin beta 1 binding protein 3 |
| IPI00300117 | SCN7A | sodium channel, voltage-gated, type VII, alpha |
| IPI00061114 | RAB3C | RAB3C, member RAS oncogene family |
| IPI00168442 | C9orf79 | chromosome 9 open reading frame 79 |
| IPI00385215 | NFATC4 | nuclear factor of activated T-cells, cytoplasmic, calcineurin-dependent 4 |
| IPI00024467 | UGGT2 | UDP-glucose glycoprotein glucosyltransferase 2 |
| IPI00297056 | CRNN | cornulin |
| IPI00914663 | KCP | kielin/chordin-like protein |
| IPI00418592 | CD1E | CD1e molecule |
| IPI00642206 | CCDC18 | coiled-coil domain containing 18 |
| IPI00514090 | LTA4H | leukotriene A4 hydrolase |
| IPI00555812 | GC | group-specific component (vitamin D binding protein) |
| IPI00219713 | FGG | fibrinogen gamma chain |
| IPI00291866 | SERPING1 | serpin peptidase inhibitor, clade G (C1 inhibitor), member 1 |
| IPI00217966 | LDHA | lactate dehydrogenase A |
| IPI00298971 | VTN | vitronectin |
| IPI00215894 | KNG1 | kininogen 1 |
| IPI00216691 | PFN1 | profilin 1 |
| IPI00292946 | SERPINA7 | serpin peptidase inhibitor, clade A (alpha-1 antiproteinase, antitrypsin), member 7 |
| IPI00022426 | AMBP | alpha-1-microglobulin/bikunin precursor |
| IPI00021405 | LMNA | lamin A/C |
| IPI00021727 | C4BPA | complement component 4 binding protein, alpha |
| IP100010779 | TPM4 | tropomyosin 4 |
| IPI00302592 | FLNA | filamin A, alpha |
| IPI00022488 | HPX | hemopexin |
| IPI00473011 | HBD | hemoglobin, delta |
| IPI00298497 | FGB | fibrinogen beta chain |
| IPI00022895 | A1BG | alpha-1-B glycoprotein |
| IPI00887739 | LOC100133511 | complement C3-like |
| IPI00218192 | ITIH4 | inter-alpha (globulin) inhibitor H4 (plasma Kallikrein-sensitive glycoprotein) |
| IPI00011252 | C8A | complement component 8, alpha polypeptide |
| IPI00292530 | ITIH1 | inter-alpha (globulin) inhibitor H1 |
| IPI00016255 | PLBD1 | hypothetical protein LOC79887 |
| IPI00477505 | ANKRD28 | Isoform 1 of Serine/threonine-protein phosphatase 6 regulatory ankyrin repeat subunit A |
| IPI00032293 | CST3 | Cystatin-C |
| IPI00293867 | DDT | D-dopachrome decarboxylase |
| IPI00059242 | SYAP1 | Synapse-associated protein 1 |
| IPI00219622 | PSMA2 | Proteasome subunit alpha type-2 |
| IPI00221222 | SUB1 | SUB1 homolog (S. cerevisiae) |
| IPI00022791 | MFAP3 | Microfibril-associated glycoprotein 3 |
| IPI00011229 | CTSD | Cathepsin D |
| IPI00025019 | PSMB1 | proteasome (prosome, macropain) subunit, beta type, 1 |
| IPI00479306 | PSMB5 | proteasome (prosome, macropain) subunit, beta type, 5 |
| IPI00234793 | KCTD15 | cDNA FLJ61112, highly similar to BTB/POZ domain-containing protein KCTD15 |
| IPI00010796 | P4HB | prolyl 4-hydroxylase, beta polypeptide |
| IPI00927606 | GPX1 | glutathione peroxidase 1 |
| IPI00783625 | SERPINB5 | serpin peptidase inhibitor, clade B (ovalbumin), member 5 |
| IPI00024701 | COL4A3BP | collagen, type IV, alpha 3 (Goodpasture antigen) binding protein |
| IPI00000811 | PSMB6 | proteasome (prosome, macropain) subunit, beta type, 6 |
| IPI00021298 | KRT20 | Keratin 20 |
| IPI00025084 | CAPNS1 | Calpain small subunit 1 |
| IPI00024919 | PRDX3 | peroxiredoxin 3 |
| IPI00059930 | NACC2 | NACC family member 2, BEN and BTB (POZ) domain containing |
| IPI00003817 | ARHGDIB | Rho GDP-dissociation inhibitor 2 |
| IPI00293276 | MIF | Macrophage migration inhibitory factor |
| IPI00514622 | RANBP6 | Ran-binding protein 6 |
| IPI00333141 | SPNS3 | Isoform 1 of Protein spinster homolog 3 |
| IPI00922181 | MCM2 | minichromosome maintenance complex component 2 |
| IPI00031708 | FAH | Fumarylacetoacetase |
| IPI00003865 | HSPA8 | heat shock 70kDa protein 8 |
| IPI00299024 | BASP1 | brain abundant, membrane attached signal protein 1 |
| IPI00181135 | BCAT2 | Branched-chain-amino-acid aminotransferase |
| IPI00219365 | MSN | Moesin |
| IPI00032134 | SERPINB8 | Serpin B8 |
| IPI00853547 | G6PD | glucose-6-phosphate dehydrogenase |
| IPI00478758 | C10orf119 | Isoform 1 of UPF0557 protein C10orf119 |
| IPI00873020 | PSAP | Prosaposin |
| IPI00000875 | EEF1G | eukaryotic translation elongation factor 1 gamma |
| IPI00014398 | FHL1 | four and a half LIM domains 1 |
| IPI00301395 | CPVL | carboxypeptidase, vitellogenic-like |
| IPI00790262 | TTLL3 | tubulin tyrosine ligase-like family, member 3 |
| IPI00942608 | 26kDaprotein | IPI:IPI00942608.1\|ENSEMBL:ENSP0000 |
| IPI00023038 | PRB1/PRB2 | proline-rich protein BstNI subfamily 2 |
| IPI00001895 | PCDH8 | Protocadherin-8 |
| IPI00419215 | A2ML1 | Alpha-2-macroglobulin-like protein 1 |
| IPI00644409 | GDA | Guanine deaminase |
| IPI00009650 | LCN1 | Lipocalin-1 |
| IPI00217467 | HIST1H1E | Histone H1.4 |
| IPI00937064 | ZAN | IPI:IPl00937064.1\|REFSEQ:XP_002342720 |
| IPI00396378 | HNRNPA2B1 | heterogeneous nuclear ribonucleoprotein A2/B1 |
| IPI00465261 | ERAP2 | endoplasmic reticulum aminopeptidase 2 |
| IPI00021263 | YWHAZ | 14-3-3 protein zeta/delta |
| IPI00011241 | GPR39 | G-protein coupled receptor 39 |
| IPI00786880 | KIAA1783 | similar to KIAA1783 protein |
| IPI00168479 | APOA1 BP | apolipoprotein A-I binding protein |
| IPI00031084 | PSD2 | pleckstrin and Sec7 domain containing 2 |
| IPI00001593 | PRCP | prolylcarboxypeptidase (angiotensinase C) |
| IPI00218343 | TUBA1C | Tubulin alpha-1C chain |
| IPI00021536 | CALML5 | Calmodulin-like protein 5 |
| IPI00028091 | ACTR3 | ARP3 actin-related protein 3 homolog (yeast) |
| IPI00796366 | MYL6 | myosin, light chain 6, alkali, smooth muscle and non-muscle |
| IPI00301058 | VASP | Vasodilator-stimulated phosphoprotein |
| IPI00470573 | ACTR2 | ARP2 actin-related protein 2 homolog (yeast) |
| IPI00884078 | RF-IP18 | Rheumatoid factor RF-IP18 |
| IPI00169383 | PGK1 | Phosphoglycerate kinase 1 |
| IPI00299619 | SLC35F1 | Solute carrier family 35 member F1 |
| IPI00419916 | ALPL | alkaline phosphatase, liver/bone/kidney |
| IPI00218319 | TPM3 | tropomyosin 3 |
| IPI00005118 | HK3 | Hexokinase-3 |
| IPI00418471 | VIM | Vimentin |
| IPI00218918 | ANXA1 | Annexin A1 |
| IPI00930073 | KRT6C | IPI:IPI00930073.1\|TREMBL:B2R853 |
| IPI00299145 | KRT6C | Keratin, type II cytoskeletal 6C |
| IPI00007858 | MYH13 | myosin, heavy chain 13, skeletal muscle |
| IPI00297235 | CCPG1 | cell cycle progression 1 |
| IPI00927726 | H-INV | Hypothetical protein |
| IPI00009008 | CACNA1D | calcium channel, voltage-dependent, L type, alpha 1D subunit |
| IPI00304554 | LYPD5 | LY6/PLAUR domain containing 5 |
| IPI00815807 | ADCK2 | aarF domain containing kinase 2 |
| IPI00743335 | MY01C | Myosin-Ic |
| IPI00007393 | APPBP2 | amyloid beta precursor protein (cytoplasmic tail) binding protein 2 |
| IPI00295976 | ITGA2B | integrin, alpha 2b (platelet glycoprotein IIb of IIb/IIIa complex, antigen CD41) |
| IPI00641706 | TUBB6 | tubulin, beta 6 |
| IPI00060201 | SYTL4 | synaptotagmin-like 4 |
| IPI00908634 | AQP4 | aquaporin 4 |
| IPI00016786 | CDC42 | cell division cycle 42 (GTP binding protein, 25kDa) |
| IPI00033494 | MYL12B | myosin, light chain 12B, regulatory |
| IPI00409756 | LOC100293553 | protein L-Myc-2-like |
| IPI00015148 | RAP1B | RAP1B, member of RAS oncogene family |
| IPI00027502 | GP9 | glycoprotein IX (platelet) |
| IPI00473014 | DSTN | Destrin |
| IPI00022394 | C1QC | complement component 1, q subcomponent, C chain |
| IPI00290337 | EPS8 | epidermal growth factor receptor pathway substrate 8 |
| IPI00018671 | DUSP3 | dual specificity phosphatase 3 |
| IPI00478231 | RHOA | ras homolog gene family, member A |
| IPI00220278 | MYL9 | myosin, light chain 9, regulatory |
| IPI00419585 | PPIA | peptidylprolyl isomerase A (cyclophilin A) |
| IPI00012011 | CFL1 | Cofilin-1 |

**Table 5: A subset of biomarkers from table 1 which were identified in an independent (verification) set of stool samples**

| Accession Number | Gene Symbol | Entrez Gene Name |
|---|---|---|
| IPI00022895 | A1BG | alpha-1-B glycoprotein |
| IPI00478003 | A2M | alpha-2-macroglobulin |
| IPI00021439 | ACTB | actin, beta |
| IPI00023006 | ACTC1 | actin, alpha, cardiac muscle 1 |
| IPI00745872 | ALB | albumin |
| IPI00022391 | APCS | amyloid P component, serum |
| IPI00337741 | APEH | N-acylaminoacyl-peptide hydrolase |
| IPI00006662 | APOD | apolipoprotein D |
| IPI00022246 | AZU1 | azurocidin 1 |
| IPI00783987 | C3 | complement component 3 |
| IPI00892604 | C4A/C4B | complement component 4B (Chido blood group) |
| IPI00032291 | C5 | complement component 5 |
| IPI00218414 | CA2 | carbonic anhydrase II |
| IPI00027466 | CA4 | carbonic anhydrase IV |
| IPI00465436 | CAT | catalase |
| IPI00017601 | CP | ceruloplasmin (ferroxidase) |
| IPI00028064 | CTSG | cathepsin G |
| IPI00005721 | DEFA1 (includesothers) | defensin, alpha 1 |
| IPI00027769 | ELANE | elastase, neutrophil expressed |
| IPI00465248 | ENO1 | enolase 1, (alpha) |
| IPI00302592 | FLNA | filamin A, alpha |
| IPI00022418 | FN1 | fibronectin 1 |
| IPI00375676 | FTL | ferritin, light polypeptide |
| IPI00385751 | FUCA1 | fucosidase, alpha-L- 1, tissue |
| IPI00023728 | GGH | gamma-glutamyl hydrolase (conjugase, folylpolygammaglutamyl hydrolase) |
| IPI00018206 | GOT2 | glutamic-oxaloacetic transaminase 2, mitochondrial (aspartate aminotransferase 2) |
| IPI00027497 | GPI | glucose-6-phosphate isomerase |
| IPI00010706 | GSS | glutathione synthetase |
| IPI00410714 | HBA1/HBA2 | hemoglobin, alpha 1 |
| IPI00654755 | HBB | hemoglobin, beta |
| IPI00473011 | HBD | hemoglobin, delta |
| IPI00012585 | HEXB | hexosaminidase B (beta polypeptide) |
| IPI00453473 | HIST4H4(includesothers) | histone cluster 1, H4c |
| IPI00472073 | HLA-B | major histocompatibility complex, class I, B |
| IPI00641737 | HP | haptoglobin |
| IPI00022488 | HPX | hemopexin |
| IPI00220362 | HSPE1 | heat shock 10kDa protein 1 (chaperonin 10) |
| IPI00217966 | LDHA | lactate dehydrogenase A |
| IPI00887739 | LOC100133511 | complement C3-like |
| IPI00022417 | LRG1 | leucine-rich alpha-2-glycoprotein 1 |
| IPI00298860 | LTF | lactotransferrin |
| IPI00106687 | LXN | latexin |
| IPI00012989 | MAN2B1 | mannosidase, alpha, class 2B, member 1 |
| IPI00007244 | MPO | myeloperoxidase |
| IPI00008787 | NAGLU | N-acetylglucosaminidase, alpha |
| IPI00020091 | ORM1/ORM2 | orosomucoid 1 |
| IPI00216691 | PFN1 | profilin 1 |
| IPI00026962 | PLA2G2A | phospholipase A2, group IIA (platelets, synovial fluid) |
| IPI00027409 | PRTN3 | proteinase 3 |
| IPI00299155 | PSMA4 | proteasome (prosome, macropain) subunit, alpha type, 4 |
| IPI00029623 | PSMA6 | proteasome (prosome, macropain) subunit, alpha type, 6 |
| IPI00028006 | PSMB2 | proteasome (prosome, macropain) subunit, beta type, 2 |
| IPI00022420 | RBP4 | retinol binding protein 4, plasma |
| IPI00006988 | RETN | resistin |
| IPI00007047 | S100A8 | S100 calcium binding protein A8 |
| IPI00027462 | S100A9 | S100 calcium binding protein A9 |
| IPI00010304 | SERPINB10 | serpin peptidase inhibitor, clade B (ovalbumin), member 10 |
| IPI00032179 | SERPINC1 | serpin peptidase inhibitor, clade C (antithrombin), member 1 |
| IPI00029863 | SERPINF2 | serpin peptidase inhibitor, clade F (alpha-2 antiplasmin, pigment epithelium derived factor), member 2 |
| IPI00291866 | SERPING1 | serpin peptidase inhibitor, clade G (C1 inhibitor), member 1 |
| IPI00022314 | SOD2 | superoxide dismutase 2, mitochondrial |
| IPI00022463 | TF | transferrin |
| IPI00643920 | TKT | transketolase |
| IPI00719280 | UBB | ubiquitin B |
| IPI00884078 | | Unmapped by Ingenuity |
| IPI00942608 | 26kDaprotein | Unmapped by Ingenuity |
| IPI00419215 | A2ML1 | alpha-2-macroglobulin-like 1 |
| IPI00937735 | ACADVL | acyl-CoA dehydrogenase, very long chain |
| IPI00009268 | ACY1 | Unmapped by Ingenuity |
| IPI00419916 | ALPL | alkaline phosphatase, liver/bone/kidney |
| IPI00219575 | BLMH | bleomycin hydrolase |
| IPI00026240 | BST1 | bone marrow stromal cell antigen 1 |
| IPI00009008 | CACNA1D | calcium channel, voltage-dependent, L type, alpha 1D subunit |
| IPI00877726 | CKMT1A/CKMT1 B | creatine kinase, mitochondrial 1B |
| IPI00299150 | CTSS | cathepsin S |
| IPI00031708 | FAH | fumarylacetoacetate hydrolase (fumarylacetoacetase) |
| IPI00644409 | GDA | guanine deaminase |
| IPI00016862 | GSR | glutathione reductase |
| IPI00396378 | HNRNPA2B1 | heterogeneous nuclear ribonucleoprotein A2/B1 |
| IPI00003865 | HSPA8 | heat shock 70kDa protein 8 |
| IPI00299145 | KRT6C | keratin 6C |
| IPI00930073 | KRT6C | keratin 6C |
| IPI00009650 | LCN1 | lipocalin 1 (tear prealbumin) |
| IPI00019038 | LYZ | lysozyme |
| IPI00922181 | MCM2 | minichromosome maintenance complex component 2 |
| IPI00257882 | PEPD | peptidase D |
| IPI00016255 | PLBD1 | phospholipase B domain containing 1 |
| IPI00219622 | PSMA2 | proteasome (prosome, macropain) subunit, alpha type, 2 |
| IPI00025019 | PSMB1 | proteasome (prosome, macropain) subunit, beta type, 1 |
| IPI00000811 | PSMB6 | proteasome (prosome, macropain) subunit, beta type, 6 |
| IPI00003590 | QSOX1 | quiescin Q6 sulfhydryl oxidase 1 |
| IPI00939604 | RNASET2 | ribonuclease T2 |
| IPI00553177 | SERPINA1 | serpin peptidase inhibitor, clade A (alpha-1 antiproteinase, antitrypsin), member 1 |
| IPI00550991 | SERPINA3 | serpin peptidase inhibitor, clade A (alpha-1 antiproteinase, antitrypsin), member 3 |
| IPI00022204 | SERPINB3 | serpin peptidase inhibitor, clade B (ovalbumin), member 3 |
| IPI00010949 | SIAE | sialic acid acetylesterase |
| IPI00465028 | TPI1 | triosephosphate isomerase 1 |
| IPI00029647 | ZG16 | zymogen granule protein 16 homolog (rat) |

**Table 6: Proteins that are significantly more abundant in colon tumor secretomes compared to normal colon secretomes**

| Accession Number | Gene Symbol | Entrez Gene Name |
|---|---|---|
| IPI00903112 | LTF | lactotransferrin |
| IPI00329573 | COL12A1 | collagen, type XII, alpha 1 |
| IPI00374563 | AGRN | agrin |
| IPI00005024 | MYBBP1A | MYB binding protein (P160) 1a |
| IPI00925034 | TRRAP | transformation/transcription domain-associated protein |
| IPI00221226 | ANXA6 | annexin A6 |
| IPI00028275 | CKAP5 | cytoskeleton associated protein 5 |
| IPI00018350 | MCM5 | minichromosome maintenance complex component 5 |
| IPI00156374 | IPO4 | importin 4 |
| IPI00894235 | NBEAL2 | neurobeachin-like 2 |
| IPI00795318 | MCM4 | minichromosome maintenance complex component 4 |
| IPI00002405 | OAS3 | 2'-5'-oligoadenylate synthetase 3, 100kDa |
| IPI00013214 | MCM3 | minichromosome maintenance complex component 3 |
| IPI00018968 | NAE1 | NEDD8 activating enzyme E1 subunit 1 |
| IPI00438229 | TRIM28 | tripartite motif containing 28 |
| IPI00221354 | FUS | fused in sarcoma |
| IPI00031820 | FARSA | phenylalanyl-tRNA synthetase, alpha subunit |
| IPI00013163 | MNDA | myeloid cell nuclear differentiation antigen |
| IPI00026970 | SUPT16H | suppressor of Ty 16 homolog (S. cerevisiae) |
| IPI00017617 | DDX5 | DEAD (Asp-Glu-Ala-Asp) box polypeptide 5 |
| IPI00031008 | TNC | tenascin C |
| IPI00007175 | NIP7 | nuclear import 7 homolog (S. cerevisiae) |
| IPI00000846 | CHD4 | chromodomain helicase DNA binding protein 4 |
| IPI00465044 | RCC2 | regulator of chromosome condensation 2 |
| IPI00031519 | DNMT1 | DNA (cytosine-5-)-methyltransferase 1 |
| IPI00028357 | XPO4 | exportin 4 |
| IP100010720 | CCT5 | chaperonin containing TCP1, subunit 5 (epsilon) |
| IPI00012340 | SRSF9 | serine/arginine-rich splicing factor 9 |
| IPI00012645 | SPTBN2 | spectrin, beta, non-erythrocytic 2 |
| IPI00032292 | TIMP1 | TIMP metallopeptidase inhibitor 1 |
| IPI00026944 | NID1 | nidogen 1 |
| IPI00013871 | RRM1 | ribonucleotide reductase M1 |
| IPI00386533 | EIF4G1 | eukaryotic translation initiation factor 4 gamma, 1 |
| IPI00149849 | COG4 | component of oligomeric golgi complex 4 |
| IPI00002894 | POLD1 | polymerase (DNA directed), delta 1, catalytic subunit 125kDa |
| IPI00221106 | SF3B2 | splicing factor 3b, subunit 2, 145kDa |
| IPI00015905 | EXOSC2 | exosome component 2 |
| IPI00031517 | MCM6 | minichromosome maintenance complex component 6 |
| IPI00216694 | PLS3 | plastin 3 |
| IPI00218407 | ALDOB | aldolase B, fructose-bisphosphate |
| IPI00556369 | SMG1 | SMG1 homolog, phosphatidylinositol 3-kinase-related kinase (C. elegans) |
| IPI00909083 | GSPT1 | G1 to S phase transition 1 |
| IPI00479786 | KHSRP | KH-type splicing regulatory protein |
| IPI00015953 | DDX21 | DEAD (Asp-Glu-Ala-Asp) box polypeptide 21 |
| IPI00334907 | PITPNB | phosphatidylinositol transfer protein, beta |
| IPI00297572 | AQR | aquarius homolog (mouse) |
| IPI00011274 | HNRPDL | heterogeneous nuclear ribonucleoprotein D-like |
| IPI00024095 | ANXA3 | annexin A3 |
| IPI00293331 | POP1 | processing of precursor 1, ribonuclease P/MRP subunit (S. cerevisiae) |
| IPI00007927 | SMC2 | structural maintenance of chromosomes 2 |
| IPI00011592 | DYNC1LI2 | dynein, cytoplasmic 1, light intermediate chain 2 |
| IPI00003927 | PPID | peptidylprolyl isomerase D |
| IPI00002926 | VPS37B | vacuolar protein sorting 37 homolog B (S. cerevisiae) |
| IPI00012497 | ADRBK1 | adrenergic, beta, receptor kinase 1 |
| IPI00746351 | DIS3 | DIS3 mitotic control homolog (S. cerevisiae) |
| IPI00031960 | POLR1A | polymerase (RNA) I polypeptide A, 194kDa |
| IPI00290566 | TCP1 | t-complex 1 |
| IPI00026952 | PKP3 | plakophilin 3 |
| IPI00856120 | LARP1B | La ribonucleoprotein domain family, member 1B |
| IPI00449049 | PARP1 | poly (ADP-ribose) polymerase 1 |
| IPI00012035 | CD46 | CD46 molecule, complement regulatory protein |
| IPI00419979 | PAK2 | p21 protein (Cdc42/Rac)-activated kinase 2 |
| IPI00303207 | ABCE1 | ATP-binding cassette, sub-family E (OABP), member 1 |
| IPI00872664 | USP14 | ubiquitin specific peptidase 14 (tRNA-guanine transglycosylase) |
| IPI00290770 | CCT3 | chaperonin containing TCP1, subunit 3 (gamma) |
| IPI00294879 | RANGAP1 | Ran GTPase activating protein 1 |
| IPI00013862 | DTYMK | deoxythymidylate kinase (thymidylate kinase) |
| IPI00329692 | NMT1 | N-myristoyltransferase 1 |
| IPI00037283 | DNM1L | dynamin 1-like |
| IPI00008922 | IFITM2 | interferon induced transmembrane protein 2 (1-8D) |
| IPI00304754 | FERMT1 | fermitin family member 1 |
| IPI00396203 | TBCD | tubulin folding cofactor D |
| IPI00009071 | LOC100505793/SRSF10 | serine/arginine-rich splicing factor 10 |
| IPI00022827 | SLK | STE20-like kinase |
| IPI00918002 | MUC5AC/MUC5B | mucin 5AC, oligomeric mucus/gel-forming |
| IPI00008240 | MARS | methionyl-tRNA synthetase |
| IPI00217013 | SMEK1 | SMEK homolog 1, suppressor of mek1 (Dictyostelium) |
| IPI00219097 | HMGB2 | high mobility group box 2 |
| IPI00304596 | NONO | non-POU domain containing, octamer-binding |
| IPI00018219 | TGFBI | transforming growth factor, beta-induced, 68kDa |
| IPI00023824 | FBLN2 | fibulin 2 |
| IPI00022228 | HDLBP | high density lipoprotein binding protein |
| IPI00306322 | COL4A2 | collagen, type IV, alpha 2 |
| IPI00018452 | CPNE1 | copine I |
| IPI00018627 | NAA50 | N(alpha)-acetyltransferase 50, NatE catalytic subunit |
| IPI00007163 | LSM7 | LSM7 homolog, U6 small nuclear RNA associated (S. cerevisiae) |
| IPI00005154 | SSRP1 | structure specific recognition protein 1 |
| IPI00007401 | IPO8 | importin 8 |
| IPI00429538 | YPEL5 | yippee-like 5 (Drosophila) |
| IPI00030116 | PGM3 | phosphoglucomutase 3 |
| IPI00162563 | RNF40 | ring finger protein 40 |
| IPI00219420 | SMC3 | structural maintenance of chromosomes 3 |
| IPI00012501 | REG4 | regenerating islet-derived family, member 4 |
| IPI00029764 | SF3A3 | splicing factor 3a, subunit 3, 60kDa |
| IPI00296099 | THBS1 | thrombospondin 1 |
| IPI00027626 | CCT6A | chaperonin containing TCP1, subunit 6A (zeta 1) |
| IPI00007928 | PRPF8 | PRP8 pre-mRNA processing factor 8 homolog (S. cerevisiae) |
| IPI00023344 | SYMPK | symplekin |
| IPI00375441 | FUBP1 | far upstream element (FUSE) binding protein 1 |
| IPI00005613 | U2AF1 | U2 small nuclear RNA auxiliary factor 1 |
| IPI00002335 | HTT | huntingtin |
| IPI00299254 | EIF5B | eukaryotic translation initiation factor 5B |
| IPI00179953 | NASP | nuclear autoantigenic sperm protein (histone-binding) |
| IPI00514561 | HNRNPK | heterogeneous nuclear ribonucleoprotein K |
| IPI00031812 | YBX1 | Y box binding protein 1 |
| IPI00414320 | ANXA11 | annexin A11 |
| IPI00178431 | RECQL | RecQ protein-like (DNA helicase Q1-like) |
| IPI00029601 | CTTN | cortactin |
| IPI00013683 | TUBB3 | tubulin, beta 3 |
| IPI00646105 | PYCRL | pyrroline-5-carboxylate reductase-like |
| IPI00298057 | PPL | periplakin |
| IPI00173346 | PGM2L1 | phosphoglucomutase 2-like 1 |
| IPI00373869 | C17orf49 | chromosome 17 open reading frame 49 |
| IPI00106491 | MRTO4 | mRNA turnover 4 homolog (S. cerevisiae) |
| IPI00290184 | METTL1 | methyltransferase like 1 |
| IPI00006025 | SART3 | squamous cell carcinoma antigen recognized by T cells 3 |
| IPI00016179 | S100A13 | S100 calcium binding protein A13 |
| IPI00100292 | NPEPL1 | aminopeptidase-like 1 |
| IPI00026689 | CDK1 | cyclin-dependent kinase 1 |
| IPI00217405 | UBR1 | ubiquitin protein ligase E3 component n-recognin 1 |
| IPI00296353 | ARHGAP18 | Rho GTPase activating protein 18 |
| IPI00293434 | SRP14 | signal recognition particle 14kDa (homologous Alu RNA binding protein) |
| IPI00292532 | CAMP | cathelicidin antimicrobial peptide |
| IPI00010740 | SFPQ | splicing factor proline/glutamine-rich |
| IPI00026262 | RASA1 | RAS p21 protein activator (GTPase activating protein) 1 |
| IPI00409601 | RALGAPB | Ral GTPase activating protein, beta subunit (non-catalytic) |
| IPI00013976 | LAMB1 | laminin, beta 1 |
| IPI00554590 | RAB3GAP2 | RAB3 GTPase activating protein subunit 2 (non-catalytic) |
| IPI00302925 | CCT8 | chaperonin containing TCP1, subunit 8 (theta) |
| IPI00103247 | HNRPLL | heterogeneous nuclear ribonucleoprotein L-like |
| IPI00414127 | RANBP1 | RAN binding protein 1 |
| IPI00001458 | KNTC1 | kinetochore associated 1 |
| IPI00221394 | DKC1 | dyskeratosis congenita 1, dyskerin |
| IPI00016613 | CSNK2A1 | casein kinase 2, alpha 1 polypeptide |
| IPI00013455 | CLIP1 | CAP-GLY domain containing linker protein 1 |
| IPI00297779 | CCT2 | chaperonin containing TCP1, subunit 2 (beta) |
| IPI00029048 | TTLL12 | tubulin tyrosine ligase-like family, member 12 |
| IPI00298306 | ATM | ataxia telangiectasia mutated |
| IPI00017451 | SF3A1 | splicing factor 3a, subunit 1, 120kDa |
| IPI00012493 | RPS20 | ribosomal protein S20 |
| IPI00783378 | UBE20 | ubiquitin-conjugating enzyme E2O |
| IPI00742682 | TPR | translocated promoter region (to activated MET oncogene) |
| IPI00002203 | BCCIP | BRCA2 and CDKN1A interacting protein |
| IPI00291783 | GEMIN5 | gem (nuclear organelle) associated protein 5 |
| IPI00019196 | RPP30 | ribonuclease P/MRP 30kDa subunit |
| IPI00056386 | LOH12CR1 | loss of heterozygosity, 12, chromosomal region 1 |
| IPI00019971 | STXBP2 | syntaxin binding protein 2 |
| IPI00020965 | UBE2H | ubiquitin-conjugating enzyme E2H |
| IPI00465045 | DIP2B | DIP2 disco-interacting protein 2 homolog B (Drosophila) |
| IPI00607591 | RAP1GDS1 | RAP1, GTP-GDP dissociation stimulator 1 |
| IPI00171903 | HNRNPM | heterogeneous nuclear ribonucleoprotein M |
| IPI00291802 | LMO7 | LIM domain 7 |
| IPI00004273 | RBM25 | RNA binding motif protein 25 |
| IPI00221234 | ALDH7A1 | aldehyde dehydrogenase 7 family, member A1 |
| IPI00026219 | CPSF1 | cleavage and polyadenylation specific factor 1, 160kDa |
| IPI00015262 | CNN2 | calponin 2 |
| IPI00018465 | CCT7 | chaperonin containing TCP1, subunit 7 (eta) |
| IPI00014238 | KARS | lysyl-tRNA synthetase |
| IPI00000684 | UAP1 | UDP-N-acteylglucosamine pyrophosphorylase 1 |
| IPI00295485 | HSPA4L | heat shock 70kDa protein 4-like |
| IPI00926528 | 138 kDa protein | 138 kDa protein |
| IPI00549189 | THOP1 | thimet oligopeptidase 1 |
| IPI00008552 | GLRX3 | glutaredoxin 3 |
| IPI00011200 | PHGDH | phosphoglycerate dehydrogenase |
| IPI00014197 | CDV3 | CDV3 homolog (mouse) |
| IPI00411559 | SMC4 | structural maintenance of chromosomes 4 |
| IPI00024320 | RBM3 | RNA binding motif (RNP1, RRM) protein 3 |
| IPI00020956 | HDGF | hepatoma-derived growth factor |
| IPI00479217 | HNRNPU | heterogeneous nuclear ribonucleoprotein U (scaffold attachment factor A) |
| IPI00604756 | NRBP1 | nuclear receptor binding protein 1 |
| IPI00418797 | POLR1B | polymerase (RNA) I polypeptide B, 128kDa |
| IPI00019812 | PPP5C | protein phosphatase 5, catalytic subunit |
| IPI00216008 | G6PD | glucose-6-phosphate dehydrogenase |
| IPI00291560 | ARG1 | arginase, liver |
| IPI00008475 | HMGCS1 | 3-hydroxy-3-methylglutaryl-CoA synthase 1 (soluble) |
| IPI00023234 | UBA2 | ubiquitin-like modifier activating enzyme 2 |
| IPI00298991 | KIAA1033 | KIAA1033 |
| IPI00793199 | ANXA4 | annexin A4 |
| IPI00889541 | DDX17 | DEAD (Asp-Glu-Ala-Asp) box polypeptide 17 |
| IPI00165393 | ANP32E | acidic (leucine-rich) nuclear phosphoprotein 32 family, member E |
| IPI00896504 | GNE | glucosamine (UDP-N-acetyl)-2-epimerase/N-acetylmannosamine kinase |
| IPI00157790 | KIAA0368 | KIAA0368 |
| IPI00182728 | VPS4B | vacuolar protein sorting 4 homolog B (S. cerevisiae) |
| IPI00020127 | RPA1 | replication protein A1, 70kDa |
| IPI00219678 | EIF2S1 | eukaryotic translation initiation factor 2, subunit 1 alpha, 35kDa |
| IPI00290461 | EIF3J | eukaryotic translation initiation factor 3, subunit J |
| IPI00784161 | SUPT6H | suppressor of Ty 6 homolog (S. cerevisiae) |
| IPI00218993 | HSPH1 | heat shock 105kDa/110kDa protein 1 |
| IPI00640703 | XPO5 | exportin 5 |
| IPI00333215 | TCEA1 | transcription elongation factor A (SII), 1 |
| IPI00009032 | SSB | Sjogren syndrome antigen B (autoantigen La) |
| IPI00745313 | AEBP1 | AE binding protein 1 |
| IPI00303258 | LMCD1 | LIM and cysteine-rich domains 1 |
| IPI00219330 | ILF3 | interleukin enhancer binding factor 3, 90kDa |
| IPI00019269 | WDR61 | WD repeat domain 61 |
| IPI00386189 | NAA15 | N(alpha)-acetyltransferase 15, NatA auxiliary subunit |
| IPI00000015 | SRSF4 | serine/arginine-rich splicing factor 4 |
| IPI00251559 | RNF20 | ring finger protein 20 |
| IPI00006952 | LACTB2 | lactamase, beta 2 |
| IPI00041325 | NHP2 | NHP2 ribonucleoprotein homolog (yeast) |
| IPI00607714 | C17orf28 | chromosome 17 open reading frame 28 |
| IPI00645702 | CTPS2 | CTP synthase II |
| IPI00163187 | FSCN1 | fascin homolog 1, actin-bundling protein (Strongylocentrotus purpuratus) |
| IPI00289807 | TRNT1 | tRNA nucleotidyl transferase, CCA-adding, 1 |
| IPI00375462 | SREK1 | splicing regulatory glutamine/lysine-rich protein 1 |
| IPI00025158 | STAG1 | stromal antigen 1 |
| IPI00024971 | OSBP | oxysterol binding protein |
| IPI00375015 | DUT | deoxyuridine triphosphatase |
| IPI00396174 | CCDC25 | coiled-coil domain containing 25 |
| IPI00020021 | DEK | DEK oncogene |
| IPI00006378 | CCDC72 | coiled-coil domain containing 72 |
| IPI00291093 | POLR2E | polymerase (RNA) II (DNA directed) polypeptide E, 25kDa |
| IPI00019178 | PSPH | phosphoserine phosphatase |
| IPI00291939 | SMC1A | structural maintenance of chromosomes 1A |
| IPI00006725 | DDX23 | DEAD (Asp-Glu-Ala-Asp) box polypeptide 23 |
| IPI00009010 | TRMT112 | tRNA methyltransferase 11-2 homolog (S. cerevisiae) |
| IPI00018813 | COPS2 | COP9 constitutive photomorphogenic homolog subunit 2 (Arabidopsis) |
| IPI00023640 | PDCD5 | programmed cell death 5 |
| IPI00031681 | CDK2 | cyclin-dependent kinase 2 |
| IPI00011603 | PSMD3 | proteasome (prosome, macropain) 26S subunit, non-ATPase, 3 |
| IPI00221325 | RANBP2 | RAN binding protein 2 |
| IPI00410693 | SERBP1 | SERPINE1 mRNA binding protein 1 |
| IPI00005780 | OGT | O-linked N-acetylglucosamine (GlcNAc) transferase (UDP-N-acetylglucosamine:polypeptide-N-acetylglucosaminyl transferase) |
| IPI00299524 | NCAPD2 | non-SMC condensin I complex, subunit D2 |
| IPI00150057 | SMARCC2 | SWI/SNF related, matrix associated, actin dependent regulator of chromatin, subfamily c, member 2 |
| IPI00032853 | NOP10 | NOP10 ribonucleoprotein homolog (yeast) |
| IPI00002255 | LRBA | LPS-responsive vesicle trafficking, beach and anchor containing |
| IPI00554742 | API5 | apoptosis inhibitor 5 |
| IPI00385267 | SRPR | signal recognition particle receptor (docking protein) |
| IPI00022462 | TFRC | transferrin receptor (p90, CD71) |
| IPI00022305 | BZW2 | basic leucine zipper and W2 domains 2 |
| IPI00025427 | RNASE3 | ribonuclease, RNase A family, 3 |
| IPI00010182 | DBI | diazepam binding inhibitor (GABA receptor modulator, acyl-CoA binding protein) |
| IPI00219005 | FKBP4 | FK506 binding protein 4, 59kDa |
| IPI00184871 | C6orf130 | chromosome 6 open reading frame 130 |
| IPI00103483 | COBRA1 | cofactor of BRCA1 |
| IPI00026215 | FEN1 | flap structure-specific endonuclease 1 |
| IPI00296635 | GBE1 | glucan (1,4-alpha-), branching enzyme 1 |
| IPI00029267 | SNRPB2 | small nuclear ribonucleoprotein polypeptide B |
| IPI00100197 | NSFL1C | NSFL1 (p97) cofactor (p47) |
| IPI00010415 | ACOT7 | acyl-CoA thioesterase 7 |
| IPI00306369 | NSUN2 | NOP2/Sun domain family, member 2 |
| IPI00302927 | CCT4 | chaperonin containing TCP1, subunit 4 (delta) |
| IPI00023845 | KLK6 | kallikrein-related peptidase 6 |
| IPI00003919 | QPCT | glutaminyl-peptide cyclotransferase |
| IPI00465128 | BAG6 | BCL2-associated athanogene 6 |
| IPI00016910 | EIF3C/EIF3CL | eukaryotic translation initiation factor 3, subunit C |
| IPI00007812 | ATP6V1B2 | ATPase, H+ transporting, lysosomal 56/58kDa, V1 subunit B2 |
| IPI00027846 | MMP8 | matrix metallopeptidase 8 (neutrophil collagenase) |
| IPI00023919 | PSMC5 | proteasome (prosome, macropain) 26S subunit, ATPase, 5 |
| IPI00217253 | GCHFR | GTP cyclohydrolase I feedback regulator |
| IPI00384028 | PAPOLA | poly(A) polymerase alpha |
| IPI00219344 | HPCAL1 | hippocalcin-like 1 |
| IPI00012442 | G3BP1 | GTPase activating protein (SH3 domain) binding protein 1 |
| IPI00024163 | POLR3A | polymerase (RNA) III (DNA directed) polypeptide A, 155kDa |
| IPI00647217 | SKIV2L2 | superkiller viralicidic activity 2-like 2 (S. cerevisiae) |
| IPI00031627 | POLR2A | polymerase (RNA) II (DNA directed) polypeptide A, 220kDa |
| IPI00873137 | COL1A2 | collagen, type I, alpha 2 |
| IPI00025039 | FBL | fibrillarin |
| IPI00923606 | EPRS | glutamyl-prolyl-tRNA synthetase |
| IPI00301936 | ELAVL1 | ELAV (embryonic lethal, abnormal vision, Drosophila)-like 1 (Hu antigen R) |
| IPI00183500 | NCBP2 | nuclear cap binding protein subunit 2, 20kDa |
| IPI00001159 | GCN1L1 | GCN1 general control of amino-acid synthesis 1-like 1 (yeast) |
| IPI00024719 | HAT1 | histone acetyltransferase 1 |
| IPI00470883 | STAG2 | stromal antigen 2 |
| IPI00216057 | SORD | sorbitol dehydrogenase |
| IPI00032830 | REXO2 | REX2, RNA exonuclease 2 homolog (S. cerevisiae) |
| IPI00003881 | HNRNPF | heterogeneous nuclear ribonucleoprotein F |
| IPI00216230 | TMPO | thymopoietin |
| IPI00902614 | USP24 | ubiquitin specific peptidase 24 |
| IPI00182757 | KIAA1967 | KIAA1967 |
| IPI00296165 | C1R | complement component 1, r subcomponent |
| IPI00002460 | ANXA7 | annexin A7 |
| IPI00009104 | RUVBL2 | RuvB-like 2 (E. coli) |
| IPI00651738 | ADI1 | acireductone dioxygenase 1 |
| IPI00009328 | EIF4A3 | eukaryotic translation initiation factor 4A3 |
| IPI00456887 | HNRNPUL2 | heterogeneous nuclear ribonucleoprotein U-like 2 |
| IPI00646605 | UBR4 | ubiquitin protein ligase E3 component n-recognin 4 |
| IPI00386718 | SMARCA2 | SWI/SNF related, matrix associated, actin dependent regulator of chromatin, subfamily a, member 2 |
| IPI00008234 | CYB5R2 | cytochrome b5 reductase 2 |
| IPI00300371 | SF3B3 | splicing factor 3b, subunit 3, 130kDa |
| IPI00156282 | GPS1 | G protein pathway suppressor 1 |
| IPI00012369 | MAD2L1 | MAD2 mitotic arrest deficient-like 1 (yeast) |
| IPI00016736 | PLCG1 | phospholipase C, gamma 1 |
| IPI00014263 | EIF4H | eukaryotic translation initiation factor 4H |
| IPI00031556 | U2AF2 | U2 small nuclear RNA auxiliary factor 2 |
| IPI00304589 | TNKS1 BP1 | tankyrase 1 binding protein 1, 182kDa |
| IPI00294578 | TGM2 | transglutaminase 2 (C polypeptide, protein-glutamine-gamma-glutamyltransferase) |
| IPI00027834 | HNRNPL | heterogeneous nuclear ribonucleoprotein L |
| IPI00329213 | INPP5D | inositol polyphosphate-5-phosphatase, 145kDa |
| IPI00296528 | ANXA10 | annexin A10 |
| IPI00013180 | BUD31 | BUD31 homolog (S. cerevisiae) |
| IPI00216048 | PITPNA | phosphatidylinositol transfer protein, alpha |
| IPI00103994 | LARS | leucyl-tRNA synthetase |
| IPI00027681 | NNMT | nicotinamide N-methyltransferase |
| IPI00185374 | PSMD12 | proteasome (prosome, macropain) 26S subunit, non-ATPase, 12 |
| IPI00004838 | CRK | v-crk sarcoma virus CT10 oncogene homolog (avian) |
| IPI00847535 | PRG2 | proteoglycan 2, bone marrow (natural killer cell activator, eosinophil granule major basic protein) |
| IPI00009802 | VCAN | versican |
| IPI00306290 | XPOT | exportin, tRNA (nuclear export receptor for tRNAs) |
| IPI00025347 | EMG1 | EMG1 nucleolar protein homolog (S. cerevisiae) |
| IPI00410091 | C11 orf73 | chromosome 11 open reading frame 73 |
| IPI00024364 | TNP01 | transportin 1 |
| IPI00292150 | LTBP2 | latent transforming growth factor beta binding protein 2 |
| IPI00470891 | CSDE1 | cold shock domain containing E1, RNA-binding |
| IPI00168554 | SRXN1 | sulfiredoxin 1 |
| IPI00103525 | PSPC1 | paraspeckle component 1 |
| IPI00419880 | RPS3A | ribosomal protein S3A |
| IPI00375631 | ISG15 | ISG15 ubiquitin-like modifier |
| IPI00027808 | POLR2B | polymerase (RNA) II (DNA directed) polypeptide B, 140kDa |
| IPI00054042 | GTF21 | general transcription factor IIi |
| IPI00026167 | NHP2L1 | NHP2 non-histone chromosome protein 2-like 1 (S. cerevisiae) |
| IPI00003565 | PSMD10 | proteasome (prosome, macropain) 26S subunit, non-ATPase, 10 |
| IPI00030781 | STAT1 | signal transducer and activator of transcription 1, 91kDa |
| IPI00293026 | EFTUD1 | elongation factor Tu GTP binding domain containing 1 |
| IPI00646226 | MED23 | mediator complex subunit 23 |
| IPI00745433 | EIF2C2 | eukaryotic translation initiation factor 2C, 2 |
| IPI00027838 | RBM4B | RNA binding motif protein 4B |
| IPI00024425 | KIAA0664 | KIAA0664 |
| IPI00024871 | CBFB | core-binding factor, beta subunit |
| IPI00008524 | PABPC1 | poly(A) binding protein, cytoplasmic 1 |
| IPI00018873 | NAMPT | nicotinamide phosphoribosyltransferase |
| IPI00216088 | CRABP2 | cellular retinoic acid binding protein 2 |
| IPI00032342 | TRIP12 | thyroid hormone receptor interactor 12 |
| IPI00154975 | DNAJC9 | DnaJ (Hsp40) homolog, subfamily C, member 9 |
| IPI00186008 | STARD10 | StAR-related lipid transfer (START) domain containing 10 |
| IPI00828098 | RNF213 | ring finger protein 213 |
| IPI00011898 | EIF2B5 | eukaryotic translation initiation factor 2B, subunit 5 epsilon, 82kDa |
| IPI00301434 | BOLA2/BOLA2B | bolA homolog 2 (E. coli) |
| IPI00181391 | MGEA5 | meningioma expressed antigen 5 (hyaluronidase) |
| IPI00022664 | RABGGTA | Rab geranylgeranyltransferase, alpha subunit |
| IPI00384689 | PDXDC1 | pyridoxal-dependent decarboxylase domain containing 1 |
| IPI00552920 | EXOSC8 | exosome component 8 |
| IPI00001734 | PSAT1 | phosphoserine aminotransferase 1 |
| IPI00010105 | EIF6 | eukaryotic translation initiation factor 6 |
| IPI00745105 | C16orf13 | chromosome 16 open reading frame 13 |
| IPI00306436 | STAT3 | signal transducer and activator of transcription 3 (acute-phase response factor) |
| IPI00220815 | EFEMP1 | EGF containing fibulin-like extracellular matrix protein 1 |
| IPI00008301 | DEFA6 | defensin, alpha 6, Paneth cell-specific |
| IPI00015018 | PPA1 | pyrophosphatase (inorganic) 1 |
| IPI00298176 | GPX2 | glutathione peroxidase 2 (gastrointestinal) |
| IPI00456635 | UNC13D | unc-13 homolog D (C. elegans) |
| IPI00218604 | PTPN6 | protein tyrosine phosphatase, non-receptor type 6 |
| IPI00760846 | MYO18A | myosin XVIIIA |
| IPI00296534 | FBLN1 | fibulin 1 |
| IPI00025329 | RPL19 | ribosomal protein L19 |
| IPI00030876 | DIAPH1 | diaphanous homolog 1 (Drosophila) |
| IPI00154451 | MMS19 | MMS19 nucleotide excision repair homolog (S. cerevisiae) |
| IPI00646917 | NUDT21 | nudix (nucleoside diphosphate linked moiety X)-type motif 21 |
| IPI00010404 | SF3B5 | splicing factor 3b, subunit 5, 10kDa |
| IPI00014938 | SARNP | SAP domain containing ribonucleoprotein |
| IPI00002186 | ARFGEF2 | ADP-ribosylation factor guanine nucleotide-exchange factor 2 (brefeldin A-inhibited) |
| IPI00028564 | GBP1 | guanylate binding protein 1, interferon-inducible |
| IPI00179298 | HUWE1 | HECT, UBA and WWE domain containing 1 |
| IPI00027142 | POP7 | processing of precursor 7, ribonuclease P/MRP subunit (S. cerevisiae) |
| IPI00006504 | EIF2B3 | eukaryotic translation initiation factor 2B, subunit 3 gamma, 58kDa |
| IPI00013184 | NAA10 | N(alpha)-acetyltransferase 10, NatA catalytic subunit |
| IPI00015947 | DNAJB1 | DnaJ (Hsp40) homolog, subfamily B, member 1 |
| IPI00019463 | EIF2AK2 | eukaryotic translation initiation factor 2-alpha kinase 2 |
| IPI00003519 | EFTUD2 | elongation factor Tu GTP binding domain containing 2 |
| IPI00004524 | GCA | grancalcin, EF-hand calcium binding protein |
| IPI00333776 | NRCAM | neuronal cell adhesion molecule |
| IPI00552073 | DHX16 | DEAH (Asp-Glu-Ala-His) box polypeptide 16 |
| IPI00013949 | SGTA | small glutamine-rich tetratricopeptide repeat (TPR)-containing, alpha |
| IPI00304742 | STK10 | serine/threonine kinase 10 |
| IPI00305833 | SMU1 | smu-1 suppressor of mec-8 and unc-52 homolog (C. elegans) |
| IPI00150269 | PRPF4 | PRP4 pre-mRNA processing factor 4 homolog (yeast) |
| IPI00028888 | HNRNPD | heterogeneous nuclear ribonucleoprotein D (AU-rich element RNA binding protein 1, 37kDa) |
| IPI00290142 | CTPS | CTP synthase |
| IPI00015195 | CSTF3 | cleavage stimulation factor, 3' pre-RNA, subunit 3, 77kDa |
| IPI00011528 | CSTF1 | cleavage stimulation factor, 3' pre-RNA, subunit 1, 50kDa |
| IPI00013070 | HNRNPUL1 | heterogeneous nuclear ribonucleoprotein U-like 1 |
| IPI00008433 | RPS5 | ribosomal protein S5 |
| IPI00298347 | PTPN11 | protein tyrosine phosphatase, non-receptor type 11 |
| IPI00514234 | LAD1 | ladinin 1 |
| IPI00000057 | COG2 | component of oligomeric golgi complex 2 |
| IPI00014311 | CUL2 | cullin 2 |
| IPI00221093 | RPS17/RPS17L | ribosomal protein S17 |
| IPI00022694 | PSMD4 | proteasome (prosome, macropain) 26S subunit, non-ATPase, 4 |
| IPI00218918 | ANXA1 | annexin A1 |
| IPI00418169 | ANXA2 | annexin A2 |
| IPI00289499 | ATIC | 5-aminoimidazole-4-carboxamide ribonucleotide formyltransferase/IMP cyclohydrolase |
| IPI00022246 | AZU1 | azurocidin 1 |
| IPI00299635 | BIRC6 | baculoviral IAP repeat containing 6 |
| IPI00002147 | CHI3L1 | chitinase 3-like 1 (cartilage glycoprotein-39) |
| IPI00011062 | CPS1 | carbamoyl-phosphate synthase 1, mitochondrial |
| IPI00028064 | CTSG | cathepsin G |
| IPI00005721 | DEFA1 (includes others) | defensin, alpha 1 |
| IPI00099110 | DMBT1 | deleted in malignant brain tumors 1 |
| IPI00027769 | ELANE | elastase, neutrophil expressed |
| IPI00217987 | ITGAM | integrin, alpha M (complement component 3 receptor 3 subunit) |
| IPI00299547 | LCN2 | lipocalin 2 |
| IPI00023673 | LGALS3BP | lectin, galactoside-binding, soluble, 3 binding protein |
| IPI00922181 | MCM2 | minichromosome maintenance complex component 2 |
| IPI00027509 | MMP9 | matrix metallopeptidase 9 (gelatinase B, 92kDa gelatinase, 92kDa type IV collagenase) |
| IPI00007244 | MPO | myeloperoxidase |
| IPI00103397 | MUC5AC/MUC5B | mucin 5AC, oligomeric mucus/gel-forming |
| IPI00019380 | NCBP1 | nuclear cap binding protein subunit 1, 80kDa |
| IPI00299512 | NF1 | neurofibromin 1 |
| IPI00022255 | OLFM4 | olfactomedin 4 |
| IPI00303063 | PDS5A | PDS5, regulator of cohesion maintenance, homolog A (S. cerevisiae) |
| IPI00021085 | PGLYRP1 | peptidoglycan recognition protein 1 |
| IPI00027409 | PRTN3 | proteinase 3 |
| IPI00003590 | QSOX1 | quiescin Q6 sulfhydryl oxidase 1 |
| IPI00009027 | REG1A | regenerating islet-derived 1 alpha |
| IPI00027462 | S100A9 | S100 calcium binding protein A9 |
| IPI00010304 | SERPINB10 | serpin peptidase inhibitor, clade B (ovalbumin), member 10 |
| IPI00783625 | SERPINB5 | serpin peptidase inhibitor, clade B (ovalbumin), member 5 |
| IPI00072534 | UNC45A | unc-45 homolog A (C. elegans) |

**Table 7: A subset of biomarkers from Table 1 which were also significantly more secreted by colon tumor samples than by normal colon (Table 6).**

| Accession | Gene Symbol | Entrez Gene Name |
|---|---|---|
| IPI00418169 | ANXA2 | annexin A2 |
| IPI00022246 | AZU1 | azurocidin 1 |
| IPI00028064 | CTSG | cathepsin G |
| IPI00005721 | DEFA1 (includes others) | defensin, alpha 1 |
| IPI00027769 | ELANE | elastase, neutrophil expressed |
| IPI00217987 | ITGAM | integrin, alpha M (complement component 3 receptor 3 subunit) |
| IPI00007244 | MPO | myeloperoxidase |
| IPI00019380 | NCBP1 | nuclear cap binding protein subunit 1, 80kDa |
| IPI00027409 | PRTN3 | proteinase 3 |
| IPI00027462 | S100A9 | S100 calcium binding protein A9 |
| IPI00010304 | SERPINB10 | serpin peptidase inhibitor, clade B (ovalbumin), member 10 |
| IPI00218918 | ANXA1 | annexin A1 |
| IPI00299635 | BIRC6 | baculoviral IAP repeat containing 6 |
| IPI00922181 | MCM2 | minichromosome maintenance complex component 2 |
| IPI00003590 | QSOX1 | quiescin Q6 sulfhydryl oxidase 1 |
| IPI00783625 | SERPINB5 | serpin peptidase inhibitor, clade B (ovalbumin), member 5 |

The invention is further described by the following, illustrative examples. These are not intended to limit the scope of the invention.

### Example 1

### Materials & Methods

### Stool samples and protein extraction

Written informed consent was obtained from all subjects who provided stool samples and this study was approved by the Medical Ethical Committee of the VU University Medical Center, The Netherlands.

Partial stool samples were collected from referral subjects who underwent colonoscopy between November 2003 and June 2006 at the VU University Medical Center in Amsterdam, The Netherlands. Partial stool samples were collected from before colonoscopy or following diagnosis at colonoscopy and prior to surgical resection of their tumors (see Table 8 for patient characteristics).

### Independent (verification) set of stool samples

Homogenized whole stool samples were collected from subjects referred for and underwent colonoscopy between July 2009 and April 2011 at the VU University Medical Center in Amsterdam, The Netherlands. Whole stool samples were collected before colonoscopy (see table 1 for patient characteristics). The study participants immediately added stabilization buffer to the stool samples after defecation, the samples were processed in the lab with a final stool:bufferw/v ratio of 1:7 within 72 hours, and stored at -80°C until use. Protein extracts were prepared as described above using 2 ml homogenized stool sample as starting material. Equal amounts of protein extracts were mixed and further treated as a single sample. Four pools from different categories were composed i.e. controls (n=5), and individuals with non-advanced adenomas (n=5), advanced adenomas (n=5) and CRC (n=5) (see Table 8 for patient characteristics).

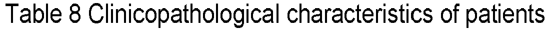

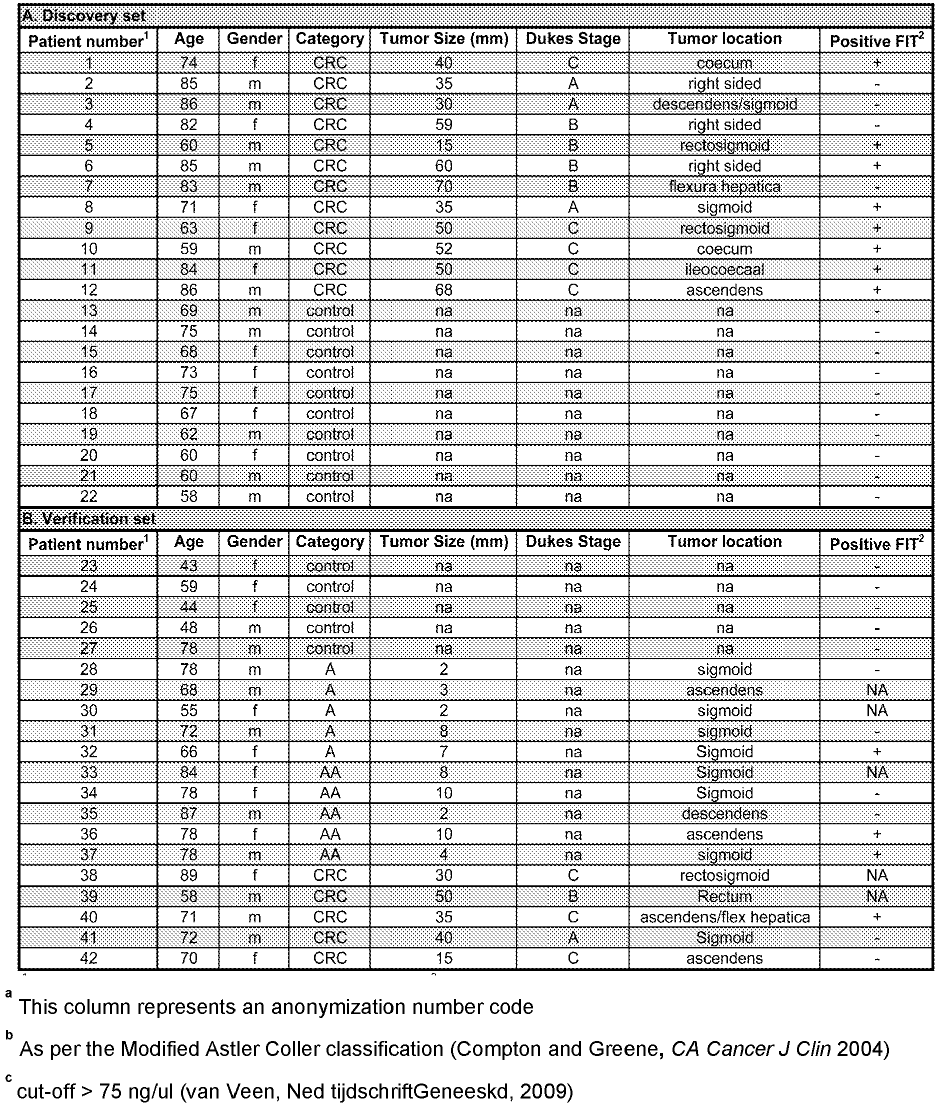

At collection, stool samples were immediately stored at 4°C and transferred to -20°C within 36 hours. Stool samples were thawed and after performing FIT (fecal immunochemical test; FIT), ∼1 g stool was sampled from each stool sample for protein extraction. For this study stool samples from 10 subjects without colon neoplasia and 4 stool samples from CRC patients with a negative FIT score were selected next to 8 stool samples from CRC patients with a positive FIT score.

Stool proteins were extracted as described before (Ang CS, Nice EC. Targeted in-gel MRM: a hypothesis driven approach for colorectal cancer biomarker discovery in human feces. J Proteome Res 2010; 9: 4346-55) with few adaptations. In short, samples were homogenized in a two-fold excess volume of PBS by vortexing and centrifuged at 4°C for 15 minutes at 16.000 G. The supernatants were centrifuged once more at 4°C for 10 minutes at full speed. Following the last spin cycle the supernatants were cleaned from remaining particles by filtering through a 0.22 µM PVDF filter (Millipore, Billerica, MA, USA). Finally, the samples were concentrated to approximately 200 µl using a 3 kDa cut-off filter (Amicon Ultra, Millipore Corporation, Billerica, MA, USA).

### 1D-SDS gel electrophoresis and sample processing for proteomics analysis

Equal protein amounts (∼30 µg) were loaded and separated on precast 4-12% gradient gels (Invitrogen, Carlsbad, USA), fixed in 50% ethanol containing 3% phosphoric acid washed and stained overnight with Coomassie R-250. After staining the gels were washed in MilliQ water and stored at 4°C until processing for in-gel digestion. Each lane was cut in 10 equal individual bands and each band was further processed into tryptic peptides as described before (Albrethsen J, et al. Mol Cell Proteomics 2010; 9: 988-1005; Piersma SR, et al. J Proteome Res 2010; 9: 1913-22).

For samples from an independent stool collection (verification experiment) equal amounts of the samples (20µl) were loaded on a 12.5% SDS-PAGE gel and run into the gel until the proteins entered the running gel. Then the gel was fixed and stained as described above. The samples were cut out of the gel as a single band and further processed to tryptic peptides as described before (Albrethsen J, et al. Mol Cell Proteomics 2010; 9: 988-1005; Piersma SR, et al. J Proteome Res 2010; 9: 1913-22). The peptides were extracted and the volume of the desalted peptide fractions was reduced to 50 µl in a vacuum centrifuge.

### nanoLC-MS/MS proteomics analysis

Peptides were separated by an Ultimate 3000 nanoLC system (Dionex LC-Packings, Amsterdam, The Netherlands) equipped with a 20 cm x 75 µm ID fused silica column custom packed with 3 µm 120 Å ReproSil Pur C18 aqua (Dr Maisch GMBH, Ammerbuch-Entringen, Germany) as described previously (Piersma SR, et al. J Proteome Res 2010; 9: 1913-22). Peptides were trapped on a 5 mm x 300 µm ID Pepmap C18 cartridge (Dionex LC-Packings, Amsterdam, The Netherlands) and separated at 300 nl/min in a 60 min gradient. Intact peptide mass spectra and fragmentation spectra (top 5) were acquired on a LTQ-FT hybrid mass spectrometer (Thermo Fisher, Bremen, Germany). Dynamic exclusion was applied with a repeat count of 1 and an exclusion time of 30 seconds. Peptides from the four pooled samples (verification experiment) were separated in triplicate on a 75 µm x 50 cm custom packed Reprosil C18 aqua column (1.9 µm, 120 Å) in a 240 min. gradient (5-32% Acetonitrile + 0.5% Acetic acid at 300 nl/min) using a U3000 RSLC high pressure nanoLC (Dionex). Eluting peptides were measured on-line by a Q Exactive mass spectrometer (ThermoFisher Scientific) operating in data-dependent acquisition mode. Peptides were ionized using a stainless-steel emitter at a potential of +2 kV (ThermoScientific). Intact peptide ions were detected at a resolution of 35000 and fragment ions at a resolution of 17500; the MS mass range was 350-1500 Da. AGC Target settings for MS were 3E6 charges and for MS/MS 2E5 charges. Peptides were selected for HCD fragmentation at an underfill ratio of 1% and a quadrupole isolation window of 1.5 Da, peptides were fragmented at normalized collision energy of 30 eV.

### SRM analysis

LC-SRM analysis was performed on the four pools from the verification set. Chromatographic separation of peptides was performed by an Ultimate 3000 RSCL Nano LC system (Dionex) equipped with custom packed nano-LC columns consisting of 20 cm x 75 µm ID fused silica custom packed with 3 µm 100 Å ReproSil Pur C18 aqua (Dr Maisch GMBH, Ammerbuch-Entringen, Germany) as described before¹⁶. Samples were analyzed in triplicate on a QTRAP 5500 instrument (AB SCIEX, Foster City, CA) operated in positive SRM mode and equipped with a nano-electrospray source with applied voltage of 2.2 kV and a capillary heater temperature of 225 °C. The scheduled SRM mode comprised the following parameters: SRM detection window of 900 sec, target scan time of 3.0 s, curtain gas of 15, ion source gas 1 of 25, declustering potential of 100, entrance potential of 10. Q1 resolution was set to unit and Q3 resolution to unit. Pause between mass ranges was set to 1 ms. Collision cell exit potentials was set to 36 for all transitions.

### SRM assay development

An SRM assay for the target proteins was developed using the MRMPilot™ software version 2.1 (AB SCIEX, Concord, ON, Canada). For each included protein, 5 peptides were selected and purchased from JPT Peptide Technologies (Berlin, Germany) as non-purified 'SpikeTides'. For each protein one µl of a mixture of the 5 selected peptides was injected at a concentration of approximately 50 fmol per peptide. For each of the peptides, the MRMPilot™ software was set to generate up to 20 theoretically possible SRM transitions, each consisting of the calculated m/z of the precursor ion (at any charge state predicted by the software) in combination with the predicted fragment ions for each predicted precursor. The highest responding peptides/transitions at a theoretically calculated optimum collision energy were determined, as well as the identity of the peptide via SRM triggered MS/MS. Each verification analysis was set up to detect 100 of all theoretically predicted transitions and their theoretically predicted optimum collision energy corresponding to the 5 peptides assessed for each candidate protein. The combined information from each SRM-Information Dependent Acquisition experiment was used to perform Mascot searches against the human Swiss-prot protein database and MultiQuant™ software version 2.1 (AB SCIEX). MultiQuant™ software was also used to generate method files for peptide verification and collision energy optimization and to integrate the results of all optimization cycles. MRMPilot was used to schedule three transitions at the experimentally found optimum and the experimentally found retention time for each peptide. The final assay contained 114 scheduled transitions, 3 for each peptide (1-5 peptides for each of the candidate proteins) and 6 for 2 external control peptides. SRM analyses on each pool were carried out in triplicate.

### SRM Data Analysis

The area under the curve (AUC) and retention time of each transition was determined using Multiquant software (AB SCIEX). Obtained transitions were then verified by two characteristics; firstly a retention time check was carried out to reveal false positives or false negatives in the assignment of transitions. If the retention time of three transitions belonging to 1 peptide was >3 seconds, the three transitions were manually inspected to assess correctness of assignment of the three transitions by the Multiquant software. If this occurred in more than one of the technical replicates then the peptide for that sample was disregarded. Secondly, the consistency of the ratio between two areas under the curve of two transitions (Transition1/Transition2, Transition2/Transition3 and Transition1/Transition3) for each peptide in the CRC pool was verified. A relative standard deviation (RSD) percentage was calculated of these ratios in the triplicate analysis. If the one transition caused the RSD percentage for two ratio's to be >20%, the transitions were manually inspected and if only one transition appeared to be incorrect, the two transitions resulting in <20% RSD over all analyses were selected for further analysis. Again if all three transition ratios had a RSD percentage above 20 then the peptide for that sample was disregarded.

### Database searching and Statistical analysis

MS/MS spectra were searched against the human IPI database 3.62 (83.947 entries) using Sequest (version 27, rev 12). Scaffold version 3.00 (Proteomesoftware, Portland, OR) was used to organize the data and to validate peptide identifications using the PeptideProphet (probability >95%) and ProteinProphet (probability of >99% with 2 peptides or more in one sample) (Keller A, et al. Anal Chem 2002; 74: 5383-92; Nesvizhskii Al, et al. Anal Chem 2003; 75: 4646-58). The software programs SecretomeP and SignalP were used for prediction of non-classical secretion and presence of a signal peptide (Bendtsen JD, et al. Protein Eng Des Sel 2004; 17: 349-56; Bendtsen JD, et al. J Mol Biol 2004; 340: 783-95). Q Exactive MS/MS spectra were searched against the human IPI database 3.68 using MaxQuant 1.2.2.5. Maximum allowed mass deviation was set to 20 ppm for MS and MS/MS. Cysteine carboxamide methylation was set as fixed modification and methionine oxidation and protein N-terminal acetylation were set as variable modifications. Identifications were filtered to 1% FDR at both the peptide and protein level. For each protein the number of assigned MS/MS spectra was summed across the 10 fractions and exported to Excel. Normalized counts were calculated by dividing the counts per protein by the sum of all counts per sample and multiciplication by the average sum across all samples. Differential analysis of proteins present in stool samples from CRC patients versus stool samples from healthy controls was performed using the beta-binomial test. The beta-binomial test takes into account the within-sample variation and the between-sample variation in a single statistical model (Pham TV, et al. Bioinformatics 2010; 26: 363-9).

### Fecal Immunochemical Test analysis

FIT samples (OC-sensor®, Eiken Chemical Co., Tokyo, Japan) were processed with the OC sensor MICRO desktop analyser (Eiken Chemical) and analyzed according to the manufacturer's instructions. A cut-off of 75 ng/ml was used to determine a positive test result (van Veen W, Mali WP. [Colorectal cancer screening: advice from the Health Council of the Netherlands]. Ned Tijdschr Geneeskd 2009; 153:A1441).

### Results

### Human protein identification in stool samples

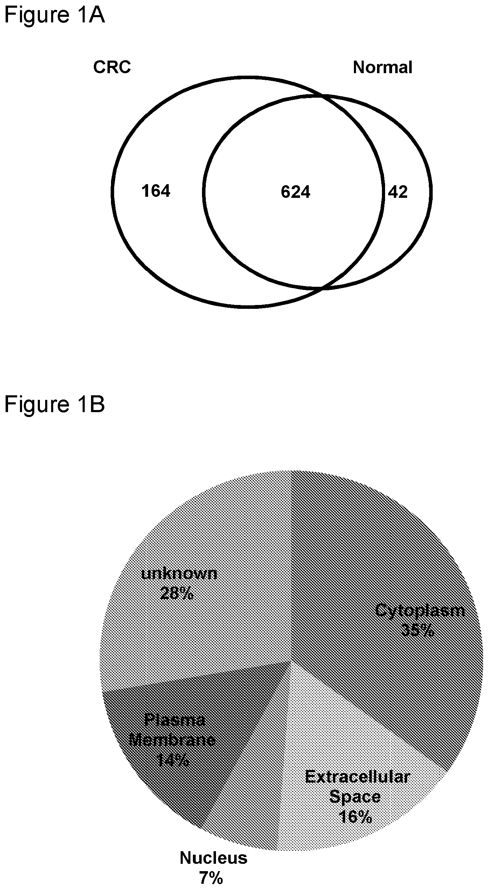

In total 830 human proteins were identified in at least one of the 22 stool samples. Of these, 624 proteins were identified both in CRC and control stool samples, 164 proteins were detected only in CRC stool samples, and 42 proteins were detected only in control stool samples (see Figure 1A). On average the number of identified proteins was consistent across all samples and did not differ significantly between CRC and control samples (326 and 296 proteins, respectively).

The primary annotation of subcellular localization of the proteins was mainly the cytoplasm (35%) and the extracellular space (16%) and did not differ between CRC and control samples (see Figure 1B). When the protein sequences were examined for secretion signals with the software tools signalP indicating signal peptides and secretomeP indicating non-classical secretion, 38% of proteins were predicted to have a signal peptide and the majority of proteins 64% were predicted to be a secreted protein. These data indicate that a high percentage of human proteins in stool samples consists of secreted proteins, e.g. in comparison to only 13% of proteins with a signal peptide in a cell line lysate (Piersma SR, et al. J Proteome Res 2010; 9: 1913-22).

### Verification of LC-MS/MS protein quantification

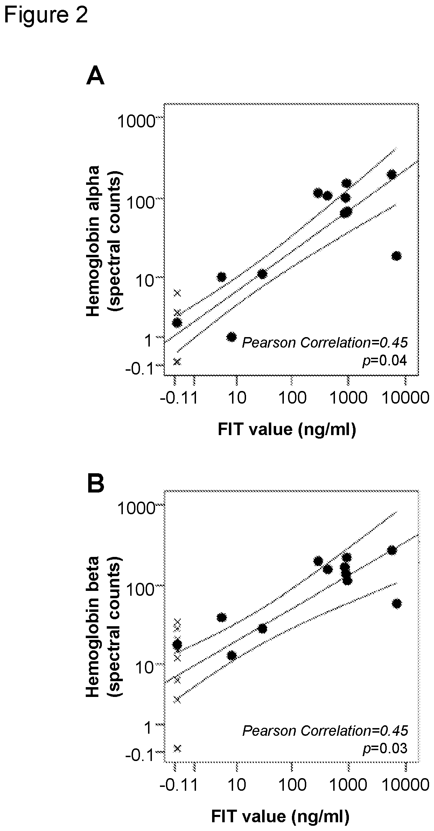

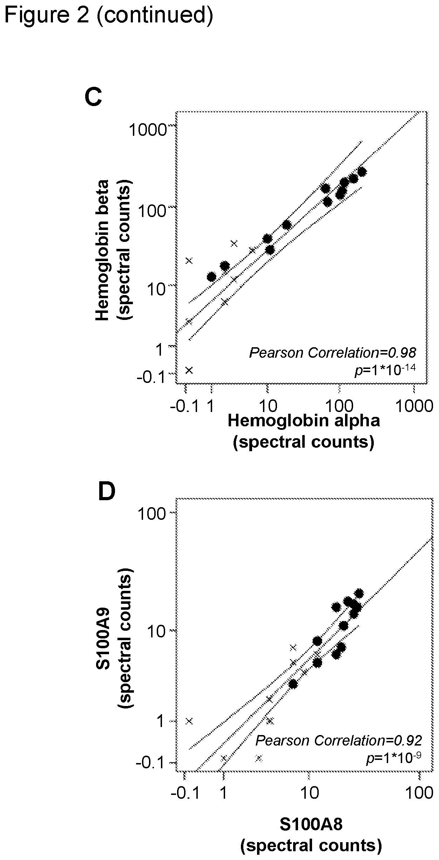

The fecal immunochemical test (FIT) is used in many countries as a non-invasive test for the early detection of CRC, and is based on the detection of hemoglobin. To verify the results obtained by LC-MS/MS, hemoglobin spectral counts were compared with FIT values (ng/ml) determined in the same stool samples. The adult hemoglobin protein is a heterodimer consisting of two α and two β chains (Schechter AN. Blood 2008; 112: 3927-38). As can be seen in Figures 2A and 2B, LC-MS/MS data on both the α and β chain of hemoglobin significantly correlated to FIT values (both *p* = 0.04). In addition, a very strong correlation was observed between the spectral counts of the α and β chain (see Figure 2C; Pearson correlation of 0.98, *p* = 1.1^{∗}10⁻¹⁴).

Another protein which has been frequently detected in stool and associated to CRC is Calprotectin (Bosch LJ, et al. Molecular tests for colorectal cancer screening. Clin Colorectal Cancer 2011; 10:8-23). Calprotectin is a heterodimer as well, consisting of S100A8 and S100A9 subunits (Yui S, et al. Biol Pharm Bull 2003; 26: 753-60). A strong correlation was observed between spectral counts of S100A8 and S100A9 (see Figure 2D; Pearson correlation of 0.92, *p* =1.2^{∗}10⁻⁹), further validating the LC-MS/MS data obtained from stool.

These results on hemoglobin and calprotectin show that LC-MS/MS on stool samples provides us with robust quantification of proteins, and is a valid approach for protein marker discovery.

### Origin of human stool proteins

The mechanisms of how tumor-derived biomarkers end up in stool can be broadly divided in leaked markers, secreted markers and exfoliated markers (Osborn NK, Ahlquist DA. Gastroenterology 2005; 128: 192-206). Hemoglobin is a typical example of a leaked marker from disturbed blood vessels in a neoplastic lesion. Secreted and exfoliated markers can be derived from the epithelial cells lining the colorectal lumen, but can also originate from cells in the surrounding stroma, such as immune cells. An overlap analysis with a previously obtained dataset of plasma proteins (unpublished data) together with the public available Human Proteome Organisation (HUPO), Human Plasma Proteome Project (HPPP) database (www.hupo.org/research/hppp; Omenn GS, et al. Proteomics 2005; 5: 3226-45; States DJ, et al. Nat Biotechnol 2006; 24: 333-8) (high confidence list) revealed that 21.6 % of the 830 identified proteins possibly originate from blood. To estimate how many of the 830 identified human proteins originate from epithelial cells, an overlap analysis was performed with proteins detected in CRC cell lines (unpublished data). Almost half of these proteins were also identified in the CRC cell lines suggesting an epithelial origin.

### Stool proteins that discriminate CRC patients from control subjects

Unsupervised hierarchical cluster analysis of human proteins identified in all CRC and control stool samples revealed two clusters. Cluster one grouped nine CRC stool samples together, and cluster two grouped all ten control stool samples and three CRC stool samples together (data not shown). Stool samples from CRC patients thus show a specific protein expression pattern as compared to stool from control subjects. This protein expression pattern can therefore be applied to discriminate most of the CRC stool samples from control stool samples without further selection of specific proteins. The complete stool protein dataset was analyzed with beta binomial statistics to identify potential cancer-associated proteins (Pham TV, et al. Bioinformatics 2010; 26: 363-9). From the 830 human stool proteins, 221 proteins were differentially detected, of which the levels of 134 proteins were significantly higher in CRC compared to control stool samples (p< 0.05; Table 2), while 87 proteins were significantly lower in controls compared to CRC stool samples.

Cluster analysis based on the 221 differentially detected proteins in CRC and control stool samples revealed two clusters as well (data not shown). Again, the nine CRC stool samples clustered together in one cluster, and the same three CRC stool samples mentioned above clustered together with the control stool samples in the second cluster.

Within the second cluster, the three CRC stool samples grouped together with one control sample. The protein expression pattern of these three CRC stool samples contained aspects of both the control stool samples and the other nine CRC stool samples, indicating that a selection of these proteins would be able to discriminate these CRC samples from controls.

In addition, three out of four FIT negative CRC patients clustered together with FIT positive CRC patients. Therefore this group of proteins contains potential candidate biomarkers that complement FIT.

### Verification by semi-high throughput nanoLC-MS/MS

In order to verify these initial results, four pools were created from an independent set of 20 stool samples. Next to CRC cases and negative controls, also samples from patients with adenomas were included. In fact, since most adenomas do not progress to cancer, for the purpose of CRC screening, only the detection of adenomas with high risk for progression besides CRC matters (Levin B et al., Gastroenterology 2008;134:1570-1595) . Therefore, pools of stool samples from patients with non-advanced adenoma as well as advanced adenomas (generally regarded as of higher risk to progression) were included. In these verification samples, 414 human proteins were identified. Out of the 830 human proteins identified in the discovery set, 331 (40%) were also detected in the verification set (see table 5). From the 134 human proteins significantly enriched in CRC stool samples, 63 (47%) were also detected in the verification set. The proteins detected in both the discovery and the verification set represented proteins with a significantly higher abundance (average spectral count of 15) compared to proteins that were not found back in the verification set (average spectral count of 4) (*p*=3.5^{∗}10⁻²⁷).

### Proof of concept of candidate protein biomarkers for validation by targeted MS

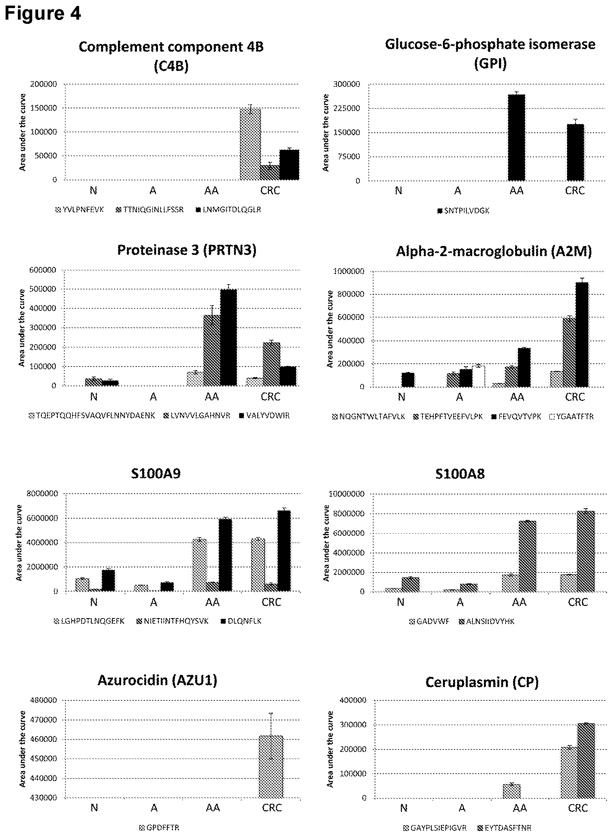

From the 134 proteins significantly enriched in CRC stool samples, a subset of proteins with a potential epithelial origin (i.e. detected in CRC cell lines), that were significantly enriched in FIT-negative CRC patients compared to controls, and were recovered in the verification set in the advanced adenoma and/or CRC pools (n=29) were selected for further validation. First results for 13 of these proteins confirmed the results obtained by LC-MS/MS in the verification set. These included 6 proteins detected in CRC and/or advanced adenoma pools while not detected in controls or adenomas (e.g. C4B and Glucose-6-phosphate isomerase (GPI) (figure 4); and 7 proteins detected in low levels in the controls and adenomas while being more abundantly present in advanced adenomas and CRC, e.g. PRTN3 and A2M (figure 4).

### Proteins that complement FIT for detecting CRC

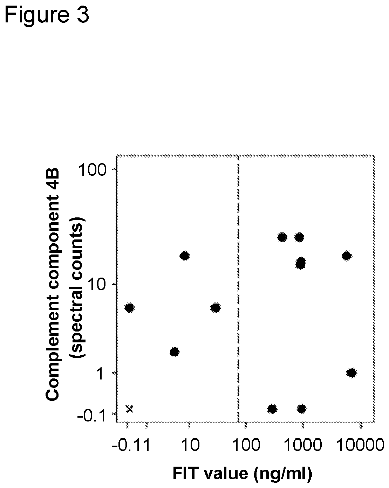

Novel biomarkers for early detection of CRC should perform significantly better than FIT, or should complement FIT, in order to increase diagnostic accuracy. As expected from their correlation with FIT, hemoglobin α and β were both significantly more abundantly present in CRC compared to control stool samples. The data revealed several proteins of which levels were significantly higher in CRC stool samples compared to control stool samples with a higher discriminative power than hemoglobin. It is of interest to see if these proteins detect the same CRC samples as hemoglobin does, or if they detect other CRC samples. Figure 3 shows an example of one of these proteins (C4A/C4B) that detects all FIT-negative CRC patients, without detecting control subject, thereby reaching a 100% sensitivity and specificity in this setting. However, not only proteins with a higher discriminative power than hemoglobin have potential to complement FIT.

For this reason, proteins that showed significantly higher levels in the FIT negative CRC stool samples compared to all controls were investigated. Indeed, out of the 134 proteins with significantly higher levels in CRC versus control stool samples, around 90% also showed significantly higher levels in the FIT negative CRC stool samples. This indicates that these candidates have high potential to be of added value to the current detection of hemoglobin.

The present study has delivered in-depth proteomic analysis on human stool samples, providing a list of 134 human proteins that were significantly enriched in stool samples from CRC patients, of which several showed highly significant discriminative power. Thus, discriminative markers for improving current FIT tests have been identified.

### Example 2

### Generation of dataset of biomarkers from CRC tissue and patient-matched normal colon tissue, for detection in biofluids.

### Material and Methods

### Patients

A total of four patients that underwent surgical resection at the VU University medical center (Amsterdam, the Netherlands) were included in this study. Collection, storage, and use of tissue and patient data were performed in accordance with the Code for Proper Secondary Use of Human Tissue in the Netherlands (Societies DFOBS. http://www.federa.org/). A pathologist inspected all samples to obtain information on tumor size, tumor and nodal stage, differentiation grade, mucinous differentiation. For an overview of the clinicopathological characteristics, see table 1.

### Tissue handling and tissue secretome collection

The tissue secretome collection was performed as described before (Celis JE. et al. Mol. Cell Proteomics 2004;3:327-4). In short, following surgical resection, the specimen was immediately transferred to the pathology department, were a pathologist excised a representative part of the tumor and adjacent normal colon mucosa (near an unaffected resection margin). These pieces of tissue were cut into cubes of approximately 1 mm³ and rinsed in PBS to remove blood and stool particles. Subsequently the tissue particles were incubated in 100µl PBS for 1 hour at 37°C. Following this incubation the samples were briefly centrifuged (2000 rpm at 4°C for 2 minutes) and the supernatant was transferred to a new eppendorf tube. The supernatants were centrifuged at maximum speed (13.200 rpm at 4°C for 20 minutes) to remove all remaining cells and debris. The soluble fractions further, referred to as the 'tissue secretomes', were stored at -80°C until further use. The tissues were processed by standard formalin fixation and paraffin embedded for histological evaluation (supplementary figure 4). Microsatellite instability (MSI) status was determined using the MSI Analysis System version 1.2 (Promega Corporation, Madison, USA) as described before (de wit M. et al. Gut 2012;61:855-64).

### Gel electrophoresis and sample preparation for proteomics analysis

Protein concentrations were determined using the BCA protein assay (Pierce, Thermo Fisher Scientific, Rockford, USA). Twenty µg of proteins were separated by gel electrophoresis using a pre-cast 1D 4-12% gradient SDS-PAGE gel (Invitrogen, Carlsbad, USA). For gel images see supplementary figure 2a-c. The gels were fixed in 50% ethanol containing 3% phosphoric acid and stained with Coomassie R-250. Gels were washed in MilliQ water and stored at 4°C until processing for in-gel digestion. Each lane was cut in 10 gel bands and further processed into tryptic peptides as described before (Piersma SR, et al. J. Proteome Res. 2010;9:1913-22). The volume of the desalted peptide fractions was reduced to 50 µl in a vacuum centrifuge.

### nanoLC-MS/MS proteomics analysis

Peptides were separated by an Ultimate 3000 nanoLC system (Dionex LC-Packings, Amsterdam, The Netherlands) equipped with a 20 cm × 75 µm ID fused silica column custom packed with 3 µm 120 Å ReproSil Pur C18 aqua (Dr Maisch GMBH, Ammerbuch-Entringen, Germany). After injection, peptides were trapped at 6 µl/min on a 1 cm × 100 µm ID trap column packed with 5 µm 120 Å ReproSil C18aqua at 2% buffer B (buffer A: 0.05% formic acid in MQ; buffer B: 80% acetonitrile + 0.05% formic acid in MQ) and separated at 300 nl/min in a 10-40% buffer B gradient in 60 min. Eluting peptides were ionized at 1.7 kV in a Nanomate Triversa Chip-based nanospray source using a Triversa LC coupler (Advion, Ithaca, NJ). Intact peptide mass spectra and fragmentation spectra were acquired on a LTQ-FT hybrid mass spectrometer (Thermo Fisher, Bremen, Germany). Intact masses were measured at resolution 50.000 in the ICR cell using a target value of 1 × 10⁶ charges. In parallel, following an FT pre-scan, the top 5 peptide signals (charge-states 2+ and higher) were submitted to MS/MS in the linear ion trap (3 amu isolation width, 30 ms activation, 35% normalized activation energy, Q value of 0.25 and a threshold of 5000 counts). Dynamic exclusion was applied with a repeat count of 1 and an exclusion time of 30 seconds.

### Database searching

To identify proteins from the acquired data MS/MS spectra were searched against the human IPI database v3.59 (80128 entries) using Sequest (version 27, rev 12), which is part of the BioWorks 3.3 data analysis package (Thermo Fisher, San Jose, CA). Following database searching the DTA and OUT files were imported into Scaffold Scaffold_2_06_01 (Proteome software, Portland, OR). Scaffold was used to organize the gel-band data and to validate peptide identifications using the Peptide Prophet algorithm (Nesvizhskii Al, et al., Anal. Chem. 2003;75:4646-58). Only identifications with a probability >95% were retained. Subsequently, the Protein Prophet algorithm (Keller A, et al., Anal. Chem. 2002;74:5383-92) was applied and protein identifications with a probability of >99% with 2 peptides or more in at least one of the samples were retained. Proteins that contained similar peptides and could not be differentiated based on MS/MS analysis alone were grouped.

### Data mining and statistical analysis

For each protein identified, the number of assigned spectra was exported to Excel. Differential analysis of samples was performed using a paired Beta-Binominal test as described previously (Pham TV et al., Expert Rev. Mol. Diagn. 2012;12:343-59). (Szklarczyk D et al., Nucleic Acids Res. 2011;39:D561-8). This modification resulted in a paired test thereby taking into account the origin of these samples e.g., comparing protein signatures between two tissues derived from the same patient. Additional general protein information was retrieved using Ingenuity Pathway Analysis (Ingenuity® Systems, www.ingenuity.com). Known and predicted protein-protein interactions were investigated using STRING version 9.0 (www.string-db.org) (Szklarczyk D, et al., Nucleic Acids Res. 2011;39:D561-8). For cluster and gene ontology analyses we used the Cytoscape platform for network analysis (www.cytoscape.org) (Smoot ME et al., Bioinformatics 2011;27:431-2), using the plug-ins ClusterONE version 0.93 for clustering and BINGO version 2.44 for the analysis of biological processes within our obtained networks based on GO annotations Maere S et al., Bioinformatics 2005;21:3448-9 and Nepusz T et al., Nat Meth 2012;9:471-2). Additionally, transmembrane domains and signal peptide sequences were investigated using secretomeP server 2.0 (Bendtsen JD et al., Protein Eng. Des. Sel. 2004;17:349-56).

### Results

### Identification of secreted proteins in CRC and normal tissue secretomes

Tissue secretomes were collected from four CRCs and matched adjacent normal colon tissue as described before by Celis at al. (Celis JE et al., Mol. Cell Proteomics 2004;3:327-44) and processed for mass spectrometry. The tumor of patient 4 was found to be MSI and the tumors of patients 1-3 were microsatellite stable (patient details in table 8. A total of 2703 non-redundant proteins were identified in the tumor secretomes of the four CRCs and their matched normal tissue secretomes. On average 1986 proteins were identified per sample, ranging from 1264 to 2292 proteins.

Of the total of 2703 proteins, 2366 were identified in the tumor secretomes as well as in the normal tissue secretomes, 283 only in the tumor secretomes and 54 only in the normal tissue secretomes. The number of identified proteins was significantly higher in the CRC secretomes than in the normal tissue secretomes (2198 and 1775 on average, respectively, *p=0.03),* while the number of unique identifications in the samples did not differ significantly (supplementary figure 2b and c, *P=0.2*)*.* In total, 961 out of 2420 (40%) of proteins were identified in all four normal tissue secretomes and 1627 out of 2649 (61%) were detected in all four cancer secretomes.

### Proteins enriched in the cancer secretomes

To obtain an overview of the dataset, an unsupervised hierarchical clustering was performed using the normalized spectral count data from all 2703 identified proteins. Two major clusters were identified; one containing the normal tissue sample of patient 3 and the other containing all other samples, this could be explained by the lower amount of proteins identified in this sample compared to the others (N = 1264). In the second cluster again two sub clusters were formed; one containing the other three normal tissue samples and the other all cancer samples. Within the cancer cluster the samples of patients 1-3 formed a separate cluster from the sample of patient 4, which can be explained by the different molecular background of this sample i.e. this latter tumor was MSI whereas the tumors of patients 1-3 were microsatellite stable (table 8). Overall the unsupervised cluster analysis indicated that the protein expression pattern was more similar among the four tumor samples than between paired tumor and normal tissues from the same patient. Potential biomarkers should be enriched in the tumor secretomes compared to the normal tissue secretomes. To identify such proteins the complete protein dataset was further analyzed with a statistical test for paired samples, i.e. taking into account the relationship between normal and cancer tissue samples from individual patients, to identify CRC associated proteins (Pham TV et al., Expert Rev. Mol. Diagn. 2012;12:343-59). From the 2703 proteins, 522 proteins were significantly differentially present (P<0.05), 409 of which were more abundant in tumor secretomes compared to normal tissue secretomes, thus representing the proteins of likely highest value for discrimination between CRC patients and normal controls (see table 6).

### Overlap analysis of proteins enriched in the cancer secretomes and stool proteins

Protein biomarkers that can be detected in blood or stool are of interest since they could be applied in a standard clinical setting alongside the routinely used markers such as CEA and CA19-9 or haemoglobin. Measuring molecules directly in the biological sample that will be used for screening, i.e. stool, can yield biomarkers that are stable and can reliably be detected in stool samples. An overlap analysis with a previously obtained dataset of stool proteins (unpublished data) revealed that 383 of the 2703 identified proteins are possibly detectable in stool. Out of the 409 significantly more abundant proteins in CRC secretomes compared to normal secretomes, 16 proteins of which were also detected in stool (see table 7).

### Industrial Applicability

The present disclosure thus provides a set of biomarkers for screening for colorectal cancer, or susceptibility to colorectal cancer. The biomarkers enable early diagnosis such that early stage surgical removal of a tumor before metastasis is possible.

The foregoing broadly describes the present invention without limitation to particular embodiments. Variations and modifications as will be readily apparent to those skilled in the art. The scope of the invention is defined by the following claims.

## Claims

1. A method for screening for the presence of advanced colorectal adenoma, the method comprising:
screening a stool sample obtained from an individual for two or more biomarkers comprising myeloperoxidase (MPO) and one or more biomarkers selected from the group consisting of: azurocidin 1 (AZU1), cathepsin G (CTSG), defensin, alpha 1 (DEFA1), elastase, neutrophil expressed (ELANE), integrin, alpha M (complement component 3 receptor 3 subunit) (ITGAM), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), annexin A2 (ANXA2), serpin peptidase inhibitor, clade B (ovalbumin), member 5 (SERPINB5), wherein the presence of or increased expression of MPO and the one or more biomarkers, relative to a control sample, is indicative that the individual is at risk of suffering from or is suffering from advanced colorectal adenoma.

2. The method of claim 1, comprising screening for two or more of the further biomarkers.

3. The method of any one of the preceding claims, comprising screening for four or more of the further biomarkers.

4. The method of any one of the preceding claims, comprising screening for all sixteen biomarkers.

5. The method of any one of the preceding claims, wherein the stool sample is screened for the two or more biomarkers using targeted mass spectrometry.

6. The method of any one of claims 1 to 4, wherein the stool sample is screened for the two or more biomarkers using binding agents capable of binding to the two or more biomarkers.

7. The method of claim 6, wherein the binding agents are antibodies or fragments thereof.

8. The method of claim 7, wherein the antibodies or fragments thereof are selected from the group consisting of scFv, Fab, and a binding domain of an immunoglobulin molecule.

9. The method of any one of claims 1-4, wherein the screening is performed using an array, optionally wherein the array is a bead-based array or a surface-based array.

10. The method of any one of the preceding claims, wherein the control sample comprises a biological sample from an individual known to be free from advanced colorectal adenoma.

11. The method of any one of the preceding claims, wherein the biological sample is also analysed by the fecal immunochemical test.

12. Use of two or more biomarkers comprising myeloperoxidase (MPO), and one or more biomarkers selected from the group consisting of: azurocidin 1 (AZU1), cathepsin G (CTSG), defensin, alpha 1 (DEFA1), elastase, neutrophil expressed (ELANE), integrin, alpha M (complement component 3 receptor 3 subunit) (ITGAM), nuclear cap binding protein subunit 1, 80kDa (NCBP1), proteinase 3 (PRTN3), S100 calcium binding protein A9 (S100A9), serpin peptidase inhibitor, clade B (ovalbumin), member 10 (SERPINB10), annexin A1 (ANXA1), baculoviral IAP repeat containing 6 (BIRC6), minichromosome maintenance complex component 2 (MCM2), quiescin Q6 sulfhydryl oxidase 1 (QSOX1), annexin A2 (ANXA2), serpin peptidase inhibitor, clade B (ovalbumin), member 5 (SERPINB5) as a biomarker in stool samples for diagnosing or predicting the presence of advanced colorectal adenoma in an individual.

13. Use of an array for determining whether an individual is at risk of suffering from or is suffering from advanced colorectal adenoma according to the method of any one of claims 1-12, the array comprising one or more binding agents as defined in any one of claims 6 to 8.

14. Use of myeloperoxidase (MPO) as a biomarker in stool samples for screening for the presence of advanced colorectal adenoma.

## Patentansprüche

1. Verfahren zum Screenen auf das Vorhandensein eines fortgeschrittenen kolorektalen Adenoms, das Verfahren umfassend:
Screenen einer Stuhlprobe, erhalten von einem Individuum, auf zwei oder mehrere Biomarker, umfassend Myeloperoxidase (MPO) und einen oder mehrere Biomarker, ausgewählt aus der Gruppe bestehend aus: Azurocidin 1 (AZU1), Cathepsin G (CTSG), Defensin, Alpha 1 (DEFA1), Elastase, neutrophil exprimiert (ELANE), Integrin, Alpha M (Komplementkomponente 3-Rezeptor 3-Untereinheit) (ITGAM), nukleäre Kappen-Bindeprotein-Untereinheit 1, 80 kDa (NCBP1), Proteinase 3 (PRTN3), S100 Calcium-Bindeprotein A9 (S100A9), Serpin-Peptidase-Inhibitor, Klade B (Ovalbumin), Glied 10 (SERPINB10), Annexin A1 (ANXA1), Baculovirus IAP Wiederholung-enthaltend 6 (BIRC6), Minichromosom- Erhaltungs-Komplex Komponente 2 (MCM2), Quiescin-Q6-Sulfhydryl-oxidase 1 (QSOX1), Annexin A2 (ANXA2), Serpin-Peptidase-Inhibitor, Klade B (Ovalbumin), Glied 5 (SERPINB5), wobei das Vorhandensein von oder erhöhte Expression von MPO und dem einen oder den mehreren Biomarkern, relativ zu einer Kontrollprobe, indikativ ist, dass das Individuum in Gefahr ist an fortgeschrittenem kolorektalem Adenom zu leiden oder daran leidet.

2. Verfahren nach Anspruch 1, umfassend Screenen auf zwei oder mehrere der weiteren Biomarker.

3. Verfahren nach einem der vorhergehenden Ansprüche, umfassend Screenen auf vier oder mehrere der weiteren Biomarker.

4. Verfahren nach einem der vorhergehenden Ansprüche, umfassend Screenen auf alle sechzehn Biomarker.

5. Verfahren nach einem der vorhergehenden Ansprüche, wobei die Stuhlprobe auf die zwei oder mehreren Biomarker unter Verwendung gezielter Massenspektrometrie gescreent wird.

6. Verfahren nach einem der Ansprüche 1 bis 4, wobei die Stuhlprobe auf die zwei oder mehreren Biomarker unter Verwendung von Bindemitteln, fähig an die zwei oder mehreren Biomarker zu binden, gescreent wird.

7. Verfahren nach Anspruch 6, wobei die Bindemittel Antikörper oder Fragmente davon sind.

8. Verfahren nach Anspruch 7, wobei die Antikörper oder Fragmente davon ausgewählt sind aus der Gruppe, bestehend aus scFv, Fab, und einer Bindedomäne eines Immunglobulinmoleküls.

9. Verfahren nach einem der Ansprüche 1-4, wobei das Screenen unter Verwendung eines Arrays durchgeführt wird, wobei optional das Array ein Kugel-basiertes Array oder ein Oberflächen-basiertes Array ist.

10. Verfahren nach einem der vorhergehenden Ansprüche, wobei die Kontrollprobe eine biologische Probe von einem Individuum umfasst, von dem bekannt ist, dass es frei von fortgeschrittenem kolorektalem Adenom ist.

11. Verfahren nach einem der vorhergehenden Ansprüche, wobei die biologische Probe auch durch den fäkal immunochemischen Test analysiert wird.

12. Verwendung von zwei oder mehreren Biomarkern, umfassend Myeloperoxidase (MPO), und einen oder mehrere Biomarker, ausgewählt aus der Gruppe bestehend aus: Azurocidin 1 (AZU1), Cathepsin G (CTSG), Defensin, Alpha 1 (DEFA1), Elastase, neutrophil exprimiert (ELANE), Integrin, Alpha M (Komplementkomponente 3-Rezeptor 3-Untereinheit) (ITGAM), nukleäre Kappen-Bindeprotein-Untereinheit 1, 80 kDa (NCBP1), Proteinase 3 (PRTN3), S100 Calcium-Bindeprotein A9 (S100A9), Serpin-Peptidase-Inhibitor, Klade B (Ovalbumin), Glied 10 (SERPINB10), Annexin A1 (ANXA1), Baculovirus IAP Wiederholung-enthaltend 6 (BIRC6), Minichromosom- Erhaltungs-Komplex Komponente 2 (MCM2), Quiescin-Q6-Sulfhydryl-oxidase 1 (QSOX1), Annexin A2 (ANXA2), Serpin-Peptidase-Inhibitor, Klade B (Ovalbumin), Glied 5 (SERPINB5) als ein Biomarker in Stuhlproben zum Diagnostizieren oder Vorhersagen des Vorhandenseins von fortgeschrittenem kolorektalem Adenom in einem Individuum.

13. Verwendung eines Arrays zum Bestimmen, ob ein Individuum in Gefahr ist an fortgeschrittenem kolorektalem Adenom, gemäß dem Verfahren von einem der Ansprüche 1-12, oder daran leidet, das Array umfassend ein oder mehrere Bindemittel wie in einem der Ansprüche 6 bis 8 definiert.

14. Verwendung von Myeloperoxidase (MPO) als einen Biomarker in Stuhlproben zum Screenen auf das Vorhandensein von fortgeschrittenem kolorektalem Adenom.

## Revendications

1. Procédé de dépistage de la présence d'adénome colorectal avancé, comprenant :
le dépistage dans un échantillon de selles provenant d'un individu de deux ou plusieurs biomarqueurs comprenant la myéloperoxydase (MPO) et un ou plusieurs biomarqueurs choisis dans le groupe comprenant:
les azurocidine 1 (AZU1), cathepsine G (CTSG), défensine, alpha 1 (DEFA1), élastase, neutrophile exprimé (ELANE), intégrine, alpha M (sous-unité du récepteur du composant 3 du complément 3) (ITGAM), sous-unité 1 de la protéine de liaison à la coiffe nucléaire, 80 kDa (NCBP1), protéinase 3 (PRTN3), protéine de liaison du calcium S100 A9 (S100A9), inhibiteur de la serpine peptidase, clade B (ovalbumine) membre 10 (SERPINB10), annexine A1 (ANXA1), répétition baculovirale IAP contenant 6 (BIRC6), composant de complexe de maintenance minichromosome 2 (MCM2), quiescine Q6 sulfydryl oxydase 1 (QSOX1), annexine A2 (ANXA2), inhibiteur de serpine peptidase, clade B (ovalbumine) membre 5 (SERPINB5),
dans lequel la présence ou l'expression accrue de MPO le ou les biomarqueurs, par rapport à un échantillon de contrôle, indique que la personne est à risque de souffrir ou souffre d'adénome colorectal avancé.

2. Procédé selon la revendication 1, comprenant le dépistage de deux ou plus des biomarqueurs supplémentaires.

3. Procédé selon l'une quelconque des revendications précédentes, comprenant le dépistage de quatre ou plus des biomarqueurs supplémentaires.

4. Procédé selon l'une quelconque des revendications précédentes, comprenant le dépistage de tous les seize biomarqueurs.

5. Procédé selon l'une quelconque des revendications précédentes, dans lequel lesdits deux biomarqueurs ou plus sont dépistés en utilisant une spectrométrie de masse ciblée dans l'échantillon de selles.

6. Procédé selon l'une quelconque des revendications 1 à 4, dans lequel lesdits deux biomarqueurs ou plus sont dépistés dans l'échantillon de selles en utilisant des agents de liaison capables de se lier auxdits deux biomarqueurs ou plus.

7. Procédé selon la revendication 6, dans lequel les agents de liaison sont des anticorps ou des fragments de ceux-ci.

8. Procédé selon la revendication 7, dans lequel les anticorps ou leurs fragments sont choisis dans le groupe constitué par scFv, Fab et un domaine de liaison d'une molécule d'immunoglobuline.

9. Procédé selon l'une quelconque des revendications 1 à 4, dans lequel le dépistage est effectué en utilisant un réseau, facultativement dans lequel le réseau est un réseau à base de billes ou un réseau à base de surface.

10. Procédé selon l'une quelconque des revendications précédentes, dans lequel l'échantillon de contrôle comprend un échantillon biologique d'un individu dont on sait qu'il est dépourvu d'adénome colorectal avancé.

11. Procédé selon l'une quelconque des revendications précédentes, dans lequel l'échantillon biologique est également analysé par le test immunochimique fécal.

12. Utilisation de deux biomarqueurs ou plus comprenant la myéloperoxydase (MPO) et un ou plusieurs biomarqueurs choisis dans le groupe constitué de : azurocidine 1 (AZU1), cathepsine G (CTSG), défensine, alpha 1 (DEFA1), élastase, neutrophile exprimé (ELANE), intégrine, alpha M (sous-unité du récepteur du composant 3 du complément 3) (ITGAM), sous-unité 1 de la protéine de liaison à la coiffe nucléaire , 80 kDa (NCBP1), protéinase 3 (PRTN3), protéine de liaison du calcium S100 A9 (S100A9), inhibiteur de la serpine peptidase, clade B (ovalbumine) membre 10 (SERPINB10), annexine A1 (ANXA1), répétition baculovirale IAP contenant 6 (BIRC6), composant 2 du complexe de maintenance du minichromosome (MCM2), quiescine Q6 sulfydryl oxydase 1 (QSOX1), annexine A2 (ANXA2), inhibiteur de la serpine peptidase, clade B (ovalbumine) membre 5 (SERPINB5) en tant que biomarqueur dans l'échantillon de selles pour diagnostiquer ou prédire la présence d'un adénome colorectal avancé chez un individu.

13. Utilisation d'un réseau pour déterminer si un individu est à risque de souffrir ou souffre d'adénome colorectal avancé selon le procédé de l'une quelconque des revendications 1 à 12, le réseau comprenant un ou plusieurs agents de liaison tels que définis dans l'une quelconque des revendications 6 à 8.

14. Utilisation de la myéloperoxydase (MPO) en tant que biomarqueur dans les échantillons de selles pour le dépistage de la présence d'adénome colorectal avancé.
